# EUROPEAN PATENT SPECIFICATION

(11) **EP 4 317 423 B1**
(45) Date of publication and mention of the grant of the patent: **06.05.2026**
(21) Application number: 22781610.5
(22) Date of filing: 30.03.2022
(51) Int. Cl.: C12N 5/09, C12M 1/04

(54) **METHOD FOR PRODUCING IMMUNE CELL COMPOSITION**
VERFAHREN ZUR HERSTELLUNG EINER IMMUNZELLENZUSAMMENSETZUNG
PROCÉDÉ DE PRODUCTION D'UNE COMPOSITION DE CELLULES IMMUNITAIRES

(30) Priority: 30.03.2021 KR 20210040926
(43) Date of publication of application: 07.02.2024
(73) Proprietor: Neogentc Corp., Seoul 05505 (KR); The Asan Foundation, Seoul 05505 (KR); University Of Ulsan Foundation For Industry Cooperation, Ulsan 44610 (KR)
(72) Inventor: LEE, Hee Jin, Seoul 06003 (KR); LEE, Kyu Jin, Seoul 05516 (KR); PARK, Hye Seon, Seoul 04575 (KR); GONG, Gyung Yub, Seongnam-si, Gyeonggi-do 13449 (KR); LIM, Chae Lyul, Hanam-si, Gyeonggi-do 12904 (KR); KIM, Young Ae, Seoul 05532 (KR); CHOI, Ji Il, Seoul 08702 (KR)
(74) Representative: dompatent
(86) International application number: PCT/KR2022/004509
(87) International publication number: WO 2022/211491

(56) References cited:
- WO-A1-2019/136459
- WO-A1-2019/217753
- WO-A1-2020/096927
- WO-A1-2020/232029
- CN-A- 110 938 594
- KR-A- 20200 138 329
- US-A1- 2017 152 478
- JIANJIAN JIN, MARIANNA SABATINO, ROBERT SOMERVILLE, JOHN R. WILSON, MARK E. DUDLEY, DAVID F. STRONCEK, AND STEVEN A. ROSENBERG: "Simplified Method of the Growth of Human Tumor Infiltrating Lymphocytes in Gas-permeable Flasks to Numbers Needed for Patient Treatment", NIH PUBLIC ACCESS AUTHOR MANUSCRIPT, 1 April 2012 (2012-04-01), pages 283 - 292, XP002778973, Retrieved from the Internet <URL:https://www.ncbi.nlm.nih.gov/pmc/articles/PMC3315105/pdf/nihms361851.pdf> [retrieved on 20180312], DOI: 10.1097/CJI.0b013e31824e801f

## Description

### [Technical Field]

The present application relates to a method for producing an immune cell composition. More particularly, the present application relates to a method for producing an immune cell composition comprising tumor-infiltrating lymphocytes (TILs).

### [Background Art]

Cancer immunotherapy is one of the methods of treating cancer and this method activates the immune system of the human body to treat cancer. As a type of immunotherapeutic used in cancer immunotherapy, there is immune cell therapy, which is a cell therapy method in which immune cells in the body are collected and reinforced or modified using a genetic engineering technique, and then put back into the body.

One of such cell therapy methods is a method using tumor-infiltrating lymphocytes (TILs), and here, the tumor infiltrating lymphocytes (TILs) refer to lymphocytes gathered around cancer cells. The therapy using TILs includes a process of collecting naturally-occurring T cells infiltrating a patient's tumor and then activating and expanding the T cells. A large number of activated T cells that have been activated and expanded in a large number through the above process are injected back into the patient, resulting in destruction of the tumor.

In other words, the process of culturing and expanding TILs is essential for cancer therapy using tumor infiltrating lymphocytes. However, in this process, the isolation TILs from tumor tissue, culture and expansion are complicated, and there is a difficulty in expanding TILs with a higher yield. Therefore, a more efficient process for TIL culture and expansion is required, and the present application relates to a method for producing an immune cell composition with a high yield.

### [Disclosure]

### [Technical Problem]

The present application is directed to providing a method for efficiently producing an immune cell composition with a high yield, compared to a conventional production method with a low yield.

In one embodiment, the present application provides a method for efficiently culturing and expanding immune cells.

In one specific embodiment, the present application provides a method for efficiently culturing and expanding tumor-infiltrating lymphocytes (TILs).

### [Technical Solution]

One aspect of the present application provides a method for producing an immune cell composition.

Another aspect of the present application provides an immune cell composition including TILs.

Still another aspect of the present application provides a use of an immune cell composition including TILs.

### [Advantageous Effects]

A method for producing an immune cell composition according to the present application has higher efficiency than that of a convention method for producing an immune cell composition. Specifically, compared to the conventional method for producing an immune cell composition, a low concentration of IL-2 is used in the method of the present application, and immune cells may be obtained in high yield.

### [Description of Drawings]

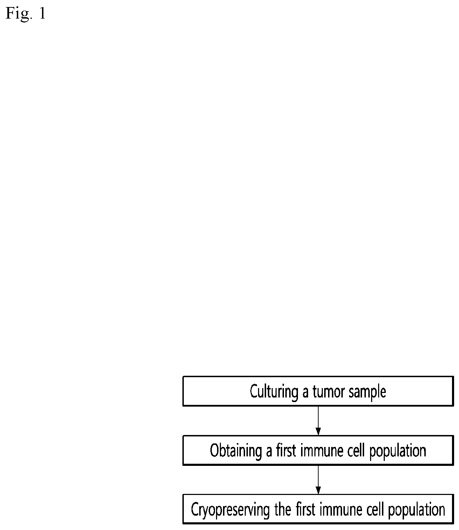

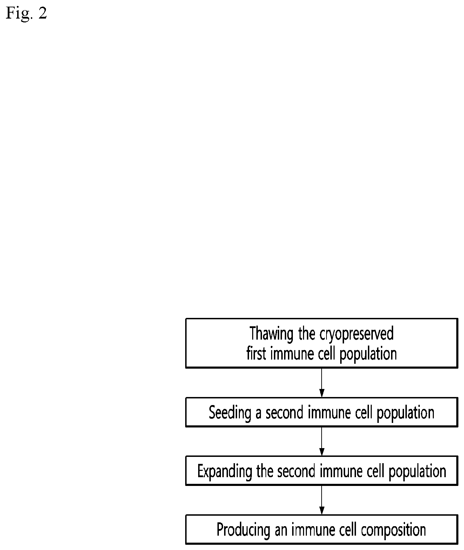

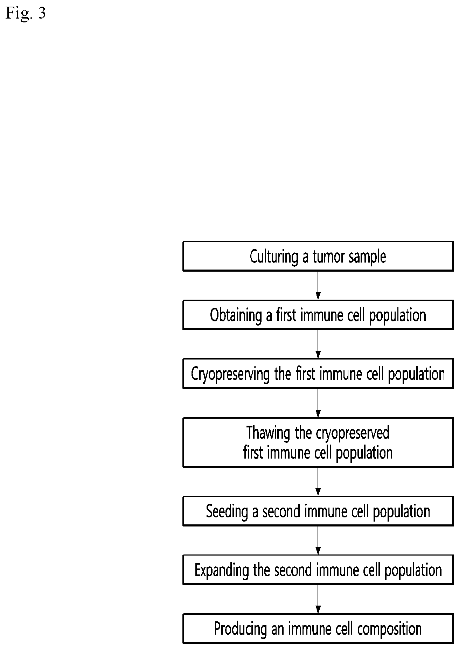

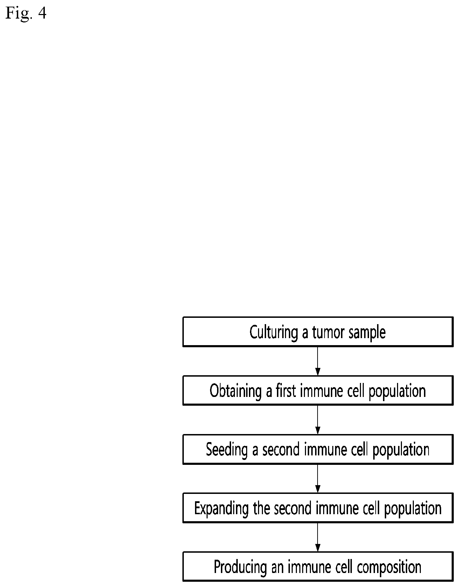

FIGS. 1 to 4 illustrate Examples of a process of producing an immune cell composition.
FIG. 1 illustrates a process of producing a semi-product (an intermediate product) (a first immune cell population produced and acquired by isolating an immune cell population from a sample and culturing).
FIG. 2 illustrates a process of producing a final product (an immune cell composition produced by acquiring a third immune cell population expanded from a second immune cell population) from a cryopreserved semi-product.
FIG. 3 illustrates a process of producing a finished product from a tumor sample, wherein the process is an example that includes a cryopreservation step.
FIG. 4 illustrates a process of producing a finished product from a tumor sample, wherein the process is an example that does not include a cryopreservation step.
FIG. 5 shows the experimental results obtained by measuring a tumor-infiltrating lymphocyte (TIL) expansion yield.
FIGS. 6 and 7 show the experimental results for optimizing an IL-2 concentration in the production of TILs.

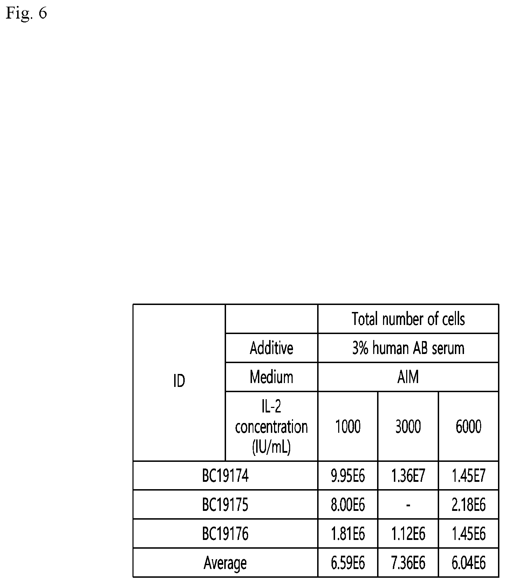

FIG. 6 shows the experimental results for optimizing an IL-2 concentration in TIL culture.

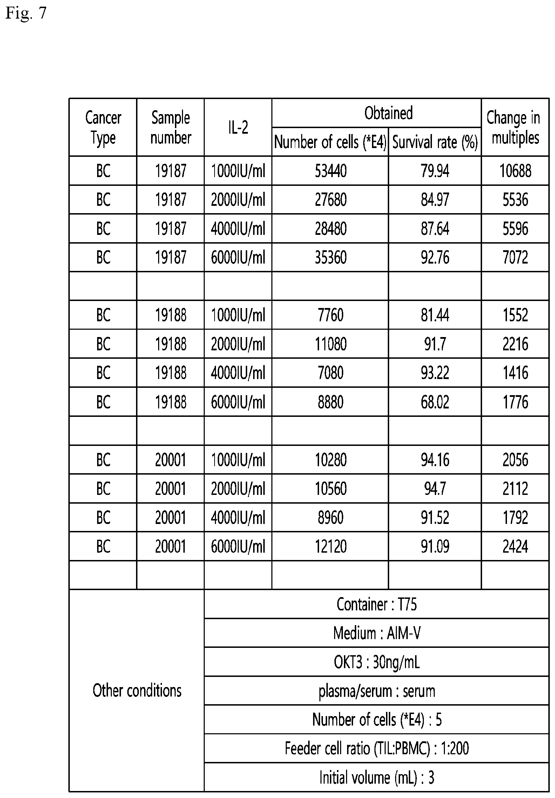

FIG. 7 shows the experimental results for optimizing an IL-2 concentration in TIL expansion.

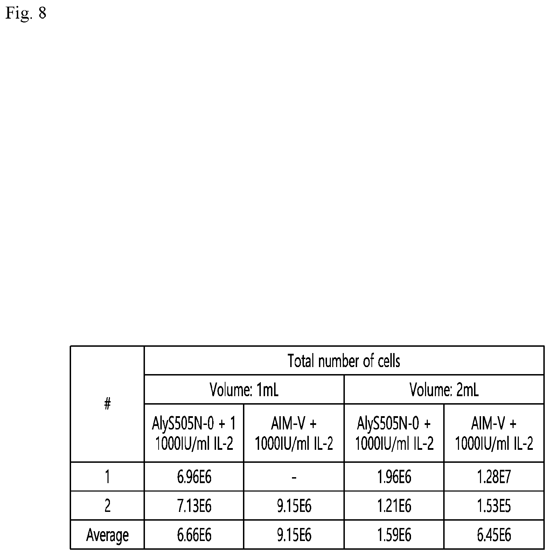

FIG. 8 shows the experimental results for the cell proliferation rate of TILs according to the volume of a composition used in culture.

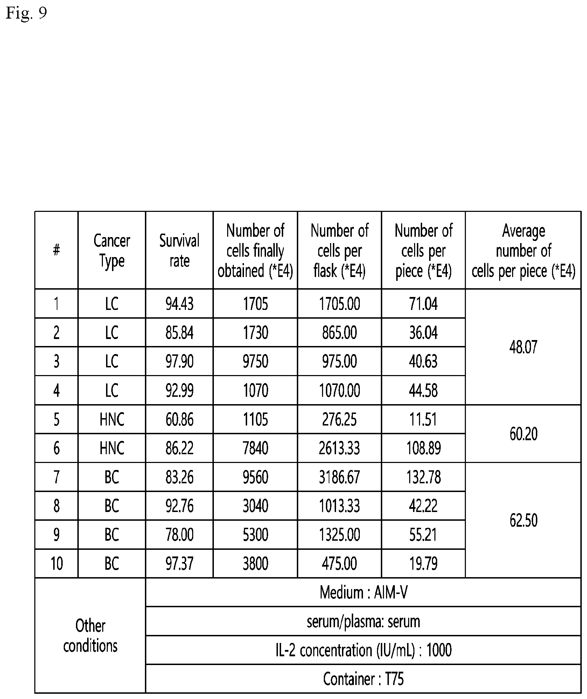

FIG. 9 shows the experimental results obtained by measuring the number of culture cells per tumor tissue in TIL culture.

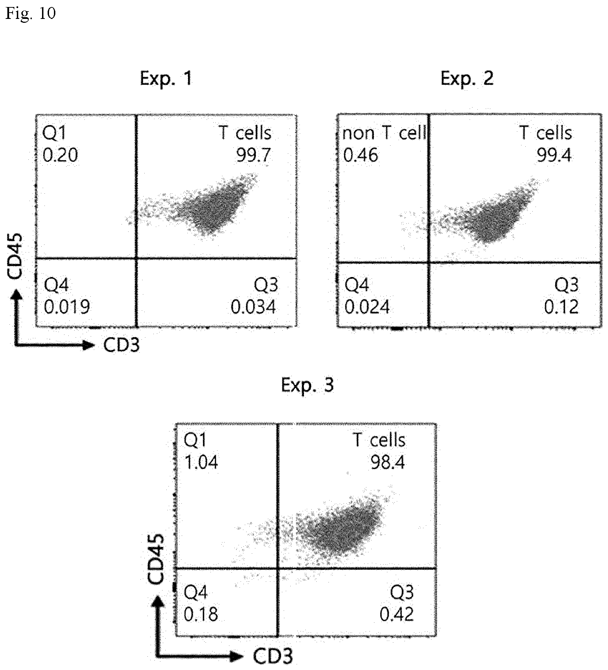

FIG. 10 shows the results of confirming the percentage of T cells in cultured and expanded TILs.

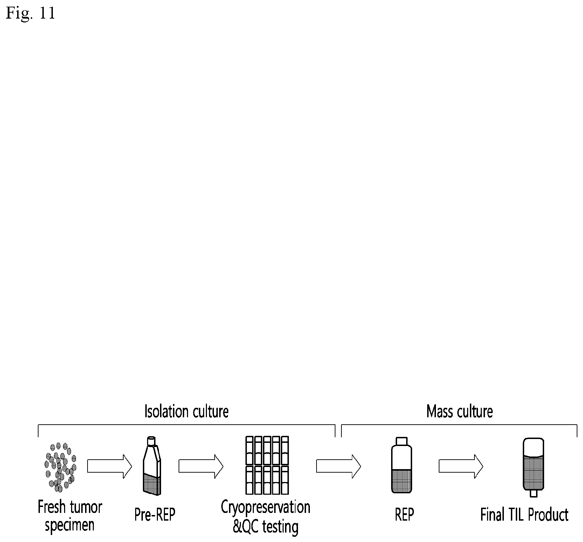

FIG. 11 shows a representative example of a process for isolation/culture of TILs from a tumor sample and mass culture (expansion) of TILs.

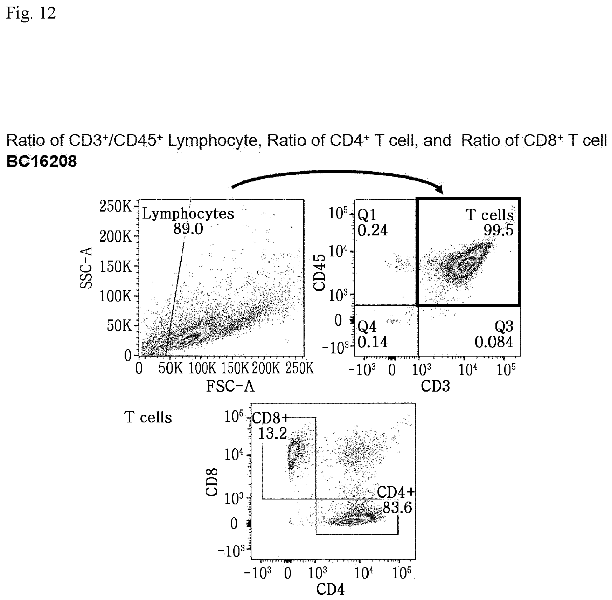

FIG. 12 shows the results of confirming the percentages of CD8+ T cells and CD4+ T cells in TILs (sample No. BC16208) expanded from a cryopreserved and thawed semi-product.

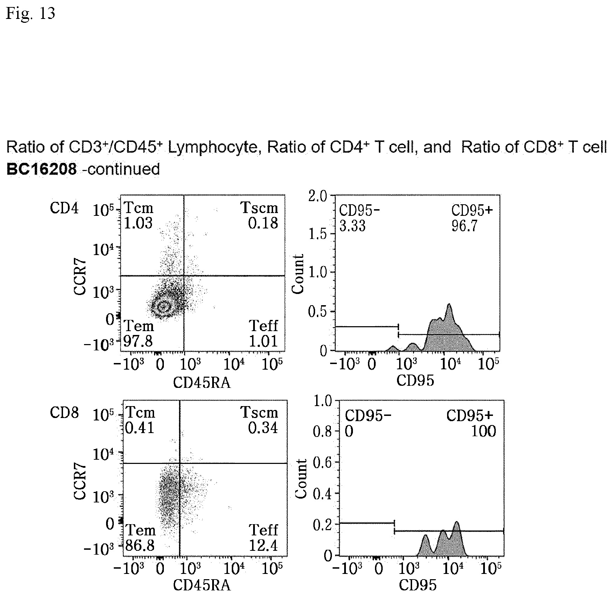

FIG. 13 shows the results of confirming the type of T cells in the CD4+ T cells and CD8+ T cells identified in FIG. 12.

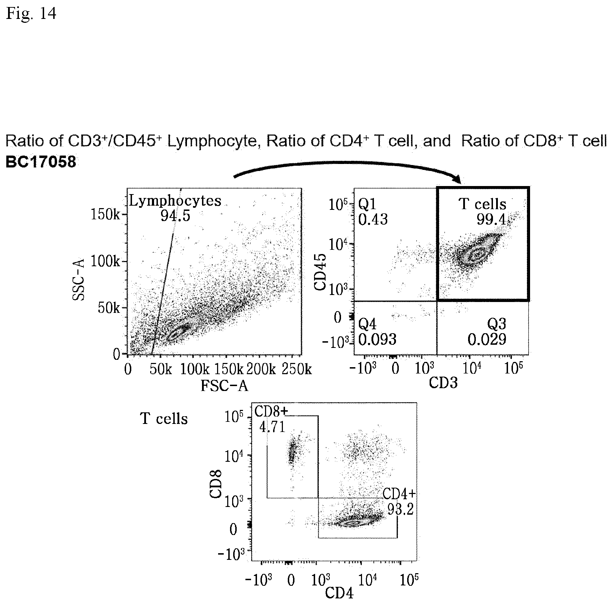

FIG. 14 shows the results of confirming CD8+ T cell and CD4+ T cell percentages in TILs (sample No. BC17058) expanded from a cryopreserved and thawed semi-product.

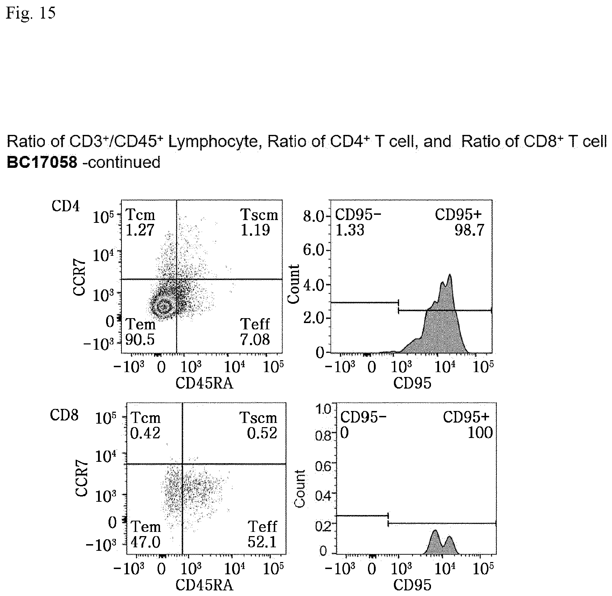

FIG. 15 shows the results of confirming the type of T cells in the CD4+ T cells and CD8+ T cells identified in FIG. 14.

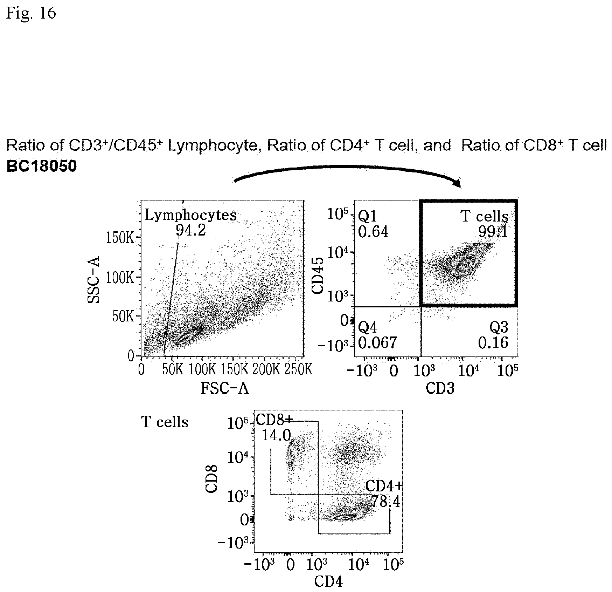

FIG. 16 shows the results of confirming the percentages of CD8+ T cells and CD4+ T cells in TILs (sample No. BC18050) expanded from a cryopreserved and thawed semi-product.

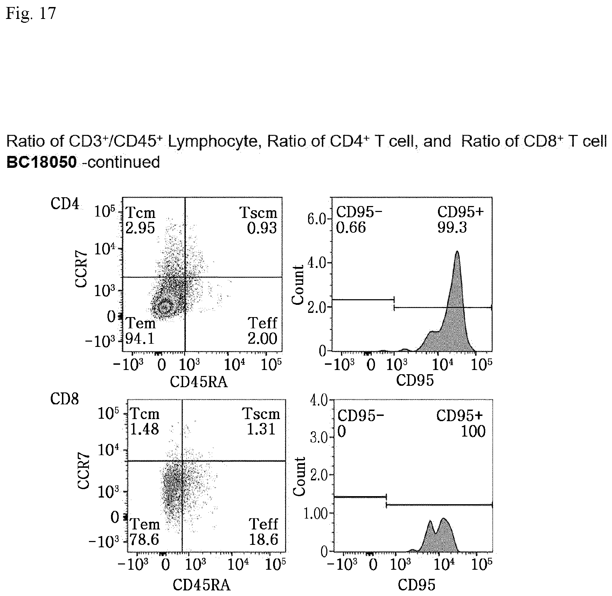

FIG. 17 shows the results of confirming the type of T cells in the CD4+ T cells and CD8+ T cells identified in FIG. 16.

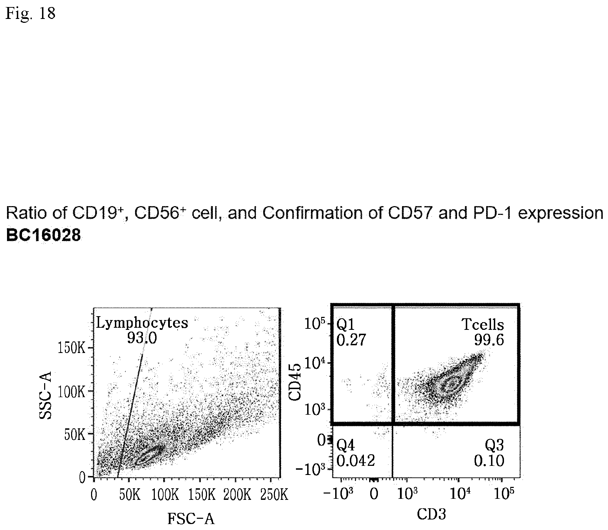

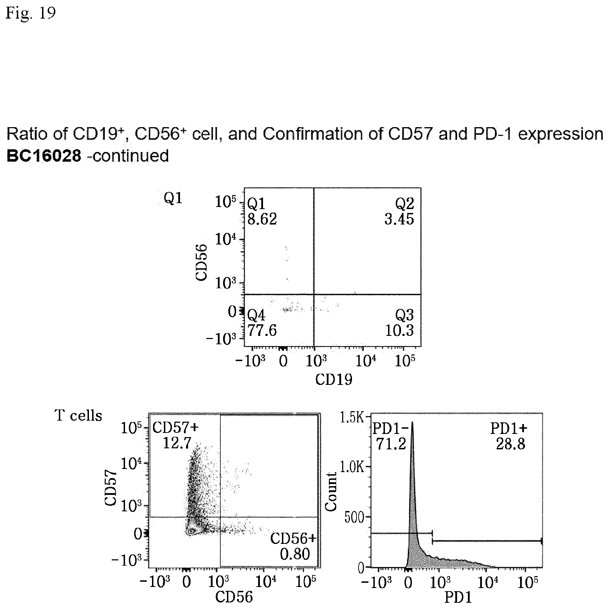

FIGS. 18 and 19 show the results of confirming the percentages of immune cells existing in TILs (sample No. BC16028) expanded from a cryopreserved and thawed semi-product.

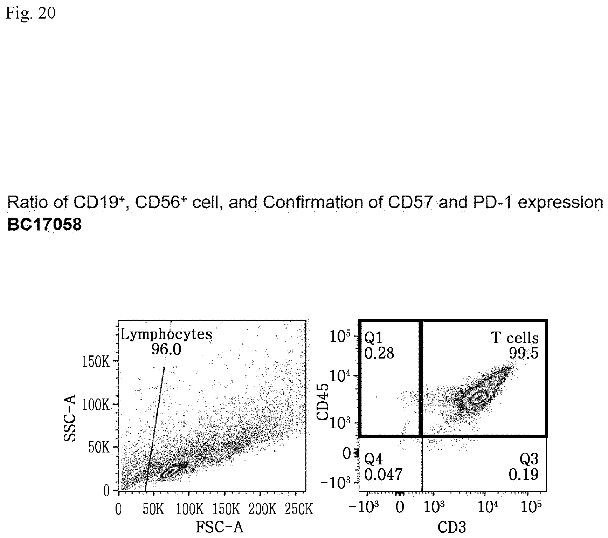

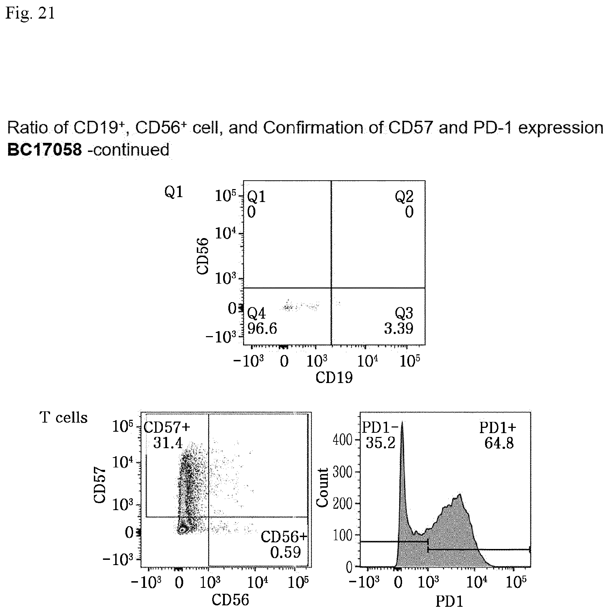

FIGS. 20 and 21 show the results of confirming the percentages of immune cells existing in TILs (sample No. BC17058) expanded from a cryopreserved and thawed semi-product.

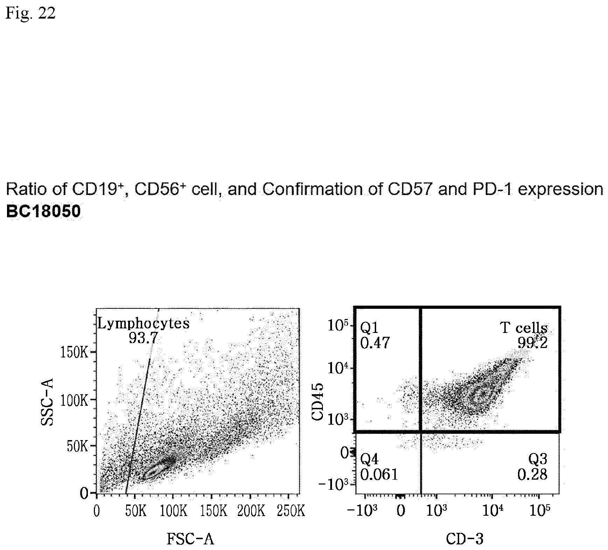

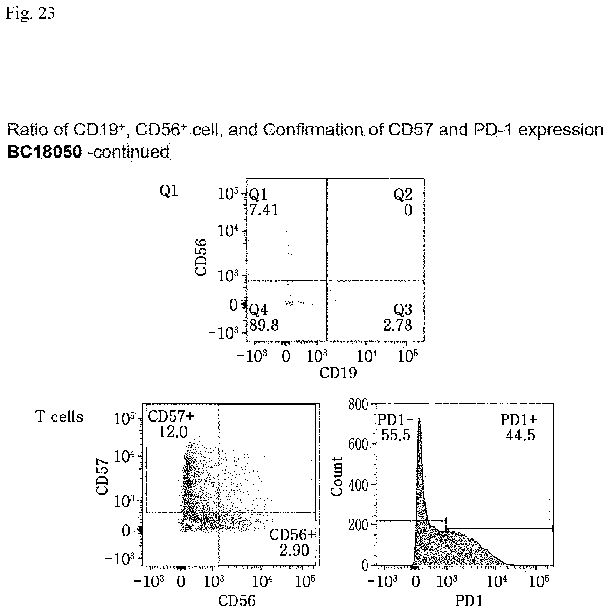

FIGS. 22 and 23 show the results of confirming the percentages of immune cells existing in TILs (sample No. BC18050) expanded from a cryopreserved and thawed semi-product.

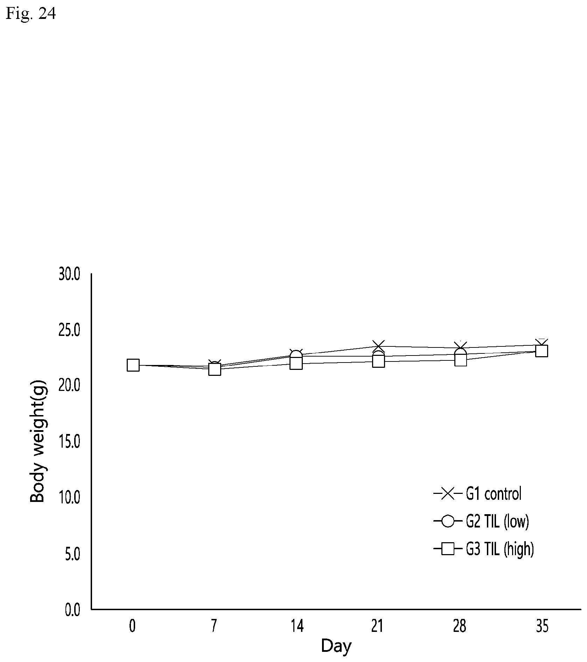

FIG. 24 shows the results of confirming the change in body weight of a mouse in each group after TIL administration.

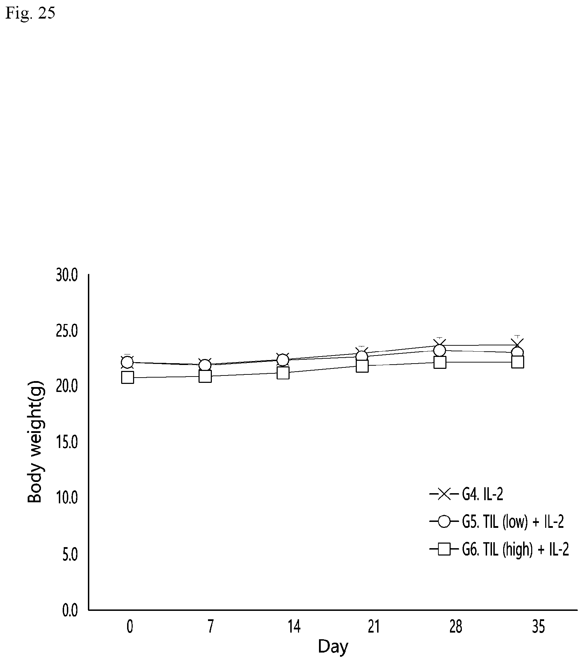

FIG. 25 shows the results of confirming the change in body weight of a mouse in each group after the co-administration of TIL and IL-2.

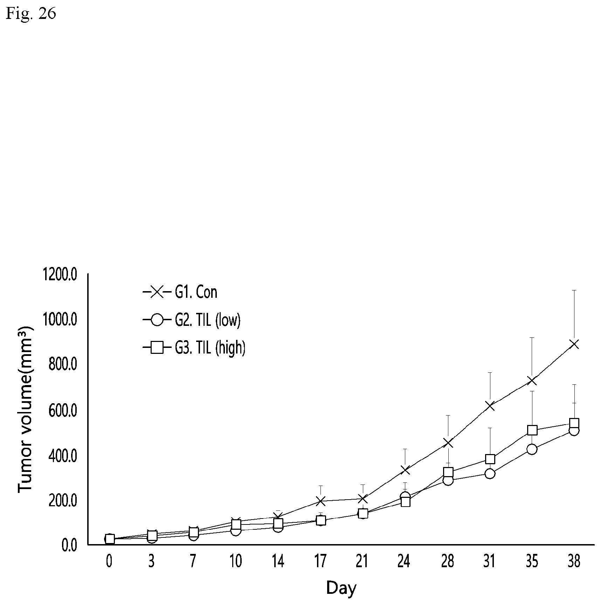

FIGS. 26, 27, 28, and 29 show the results of confirming the change in tumor volume of a mouse in each group after TIL administration.

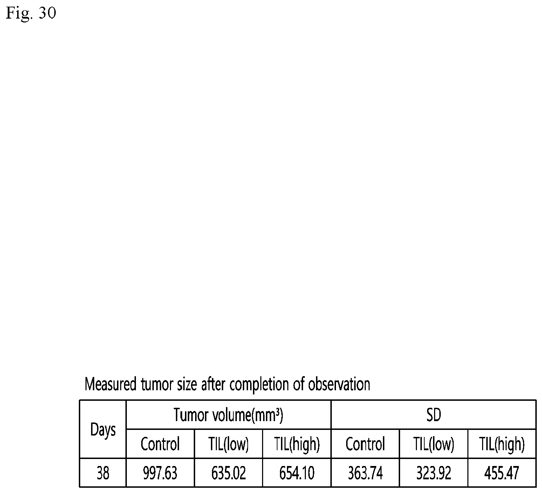

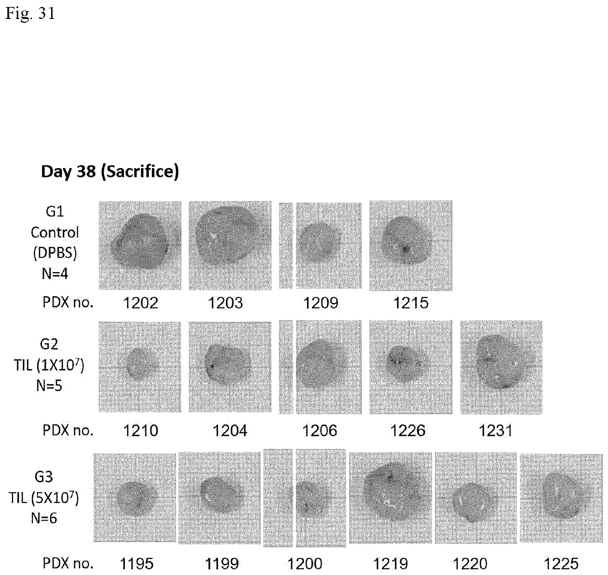

FIGS. 30 and 31 show the results of confirming a tumor size in each group, obtained by euthanizing a mouse on day 38 after TIL administration and extracting the tumor therefrom.

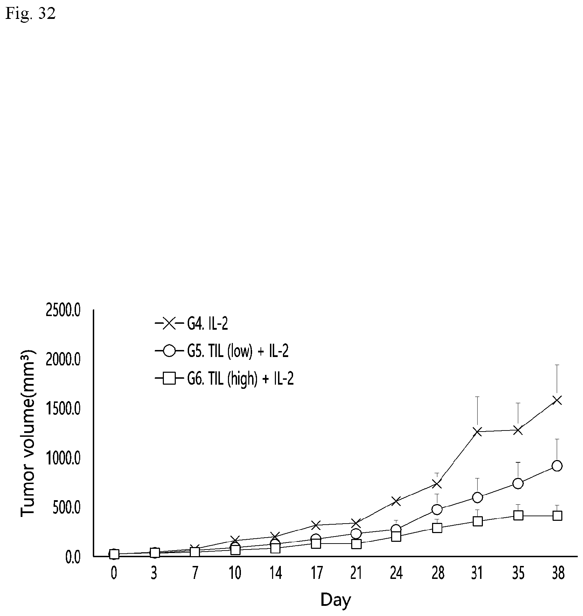

FIGS. 32, 33, 34, and 35 show the results of confirming the change in tumor size of a mouse in each group after the co-administration of TIL and IL-2.

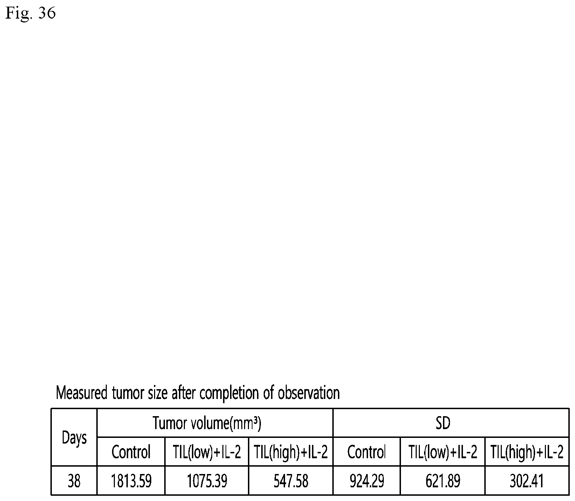

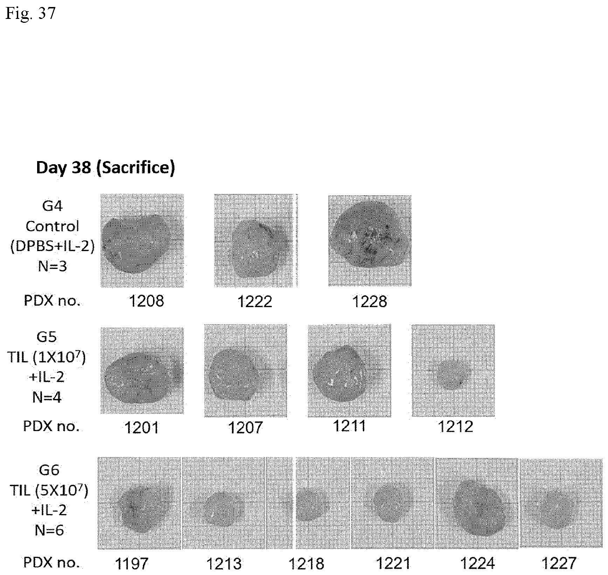

FIGS. 36 and 37 show the results of confirming a tumor size in each group, obtained by euthanizing a mouse on day 38 after TIL administration and extracting the tumor therefrom.

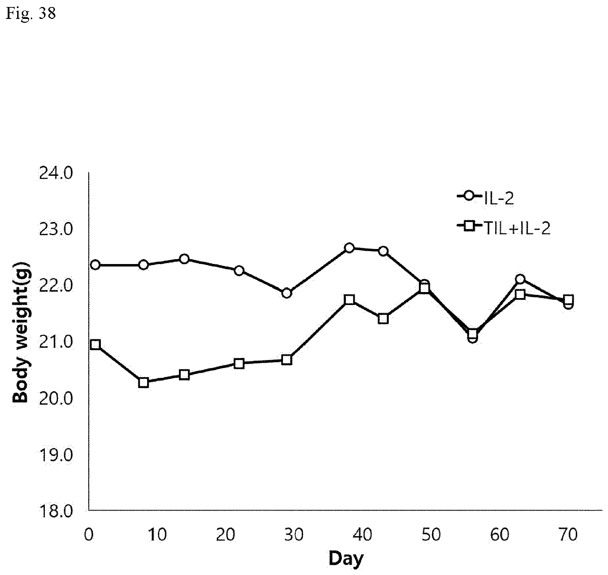

FIG. 38 shows the results of confirming the change in body weight of a mouse in each group after the co-administration of TIL and IL-2.

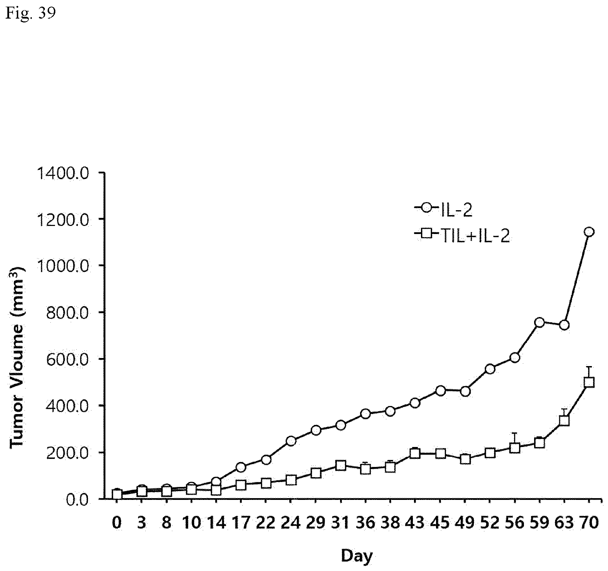

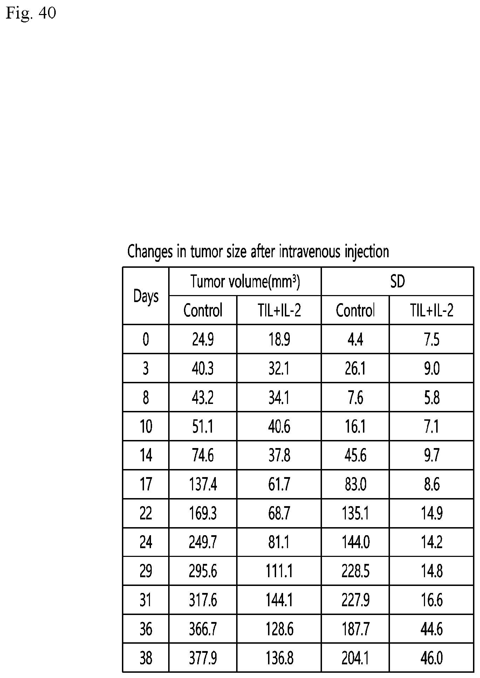

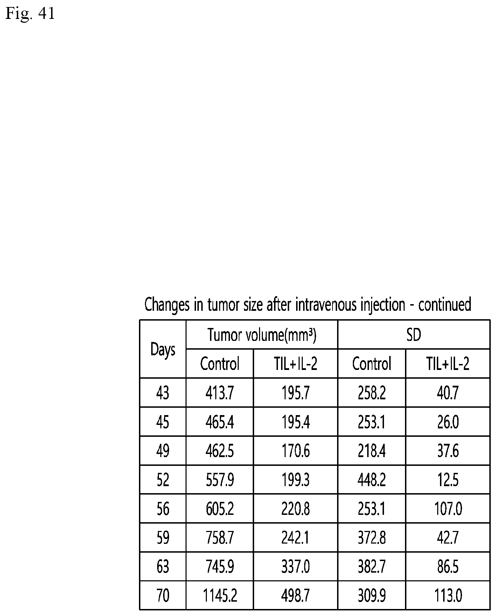

FIGS. 39, 40, 41, and 42 show the results of confirming the change in tumor volume of a mouse in each group after the co-administration of TIL and IL-2.

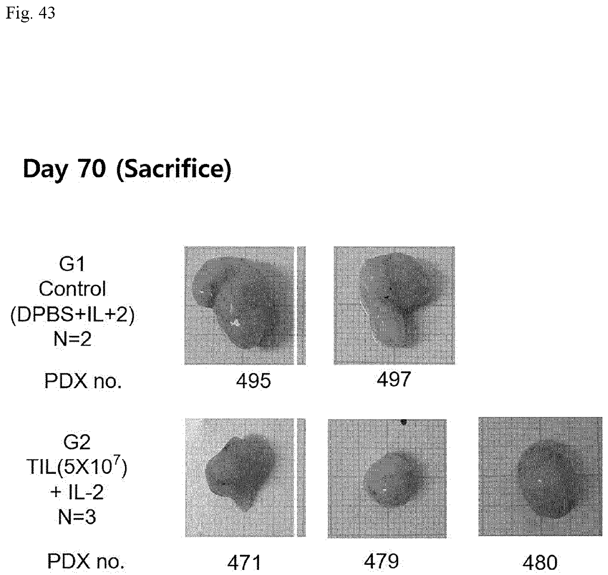

FIG. 43 shows the results of confirming a tumor volume in each group, obtained by euthanizing a mouse on day 70 after the co-administration of TIL and IL-2 and extracting a tumor therefrom.

### [Modes of the Invention]

In one embodiment of the present application, a method for producing an immune cell composition is provided.

In one embodiment of the present application, an immune cell composition produced by a method according to an embodiment of the present application is provided.

In one embodiment of the present application, a use of an immune cell composition for treating cancer in a subject is provided.

In one embodiment of the present application, a method for treating cancer in a subject using immune cell composition.

Embodiments of the present application are described detailed below.

In one embodiment of the present application, a method for producing an immune cell composition comprising the following is provided:
(a) culturing a tumor sample in a first container,
   wherein the tumor sample is cultured in a first composition in the first container, wherein the first composition comprises medium, serum, and 500 to 1500 IU/ml of IL-2,
   wherein the first container is a gas-permeable container or a container comprising gas supply system,
   wherein the culturing is performed for 13 to 15 days,
   wherein, during the culturing, a first additive composition is added to the first container once or twice or more, and the first additive composition comprises medium, serum, and IL-2;
(b) obtaining a first immune cell population from the cultured product obtained from (a);
(c) seeding a second composition and a second immune cell population in a second container,
   wherein the second immune cell population is derived from the first immune cell population,
   wherein the second composition comprises medium, serum, 1000 to 3000 IU/ml of IL-2, anti-CD3 antibody, and a feeder cell,
   wherein the second container is a gas-permeable container or a container comprising gas supply system;
(d) expanding the second immune cell population to produce a third immune cell population,
   wherein during the expanding, a second additive composition is added to the second container once or twice or more, and the second additive composition comprises medium, serum, and IL-2,
   wherein the expanding is performed for 13 to 15 days; and
(e) obtaining the third immune cell population from the expanded product expanded in (d) and producing the immune cell composition,
   wherein the third immune cell population has at least 2500 times more cells compared to the second immune cell population.

In one embodiment, the method may further comprise preparing the tumor sample, wherein the preparing the tumor sample may be performed prior to (a).

In one embodiment, in (a), the tumor sample may be obtained from a tumor fragment obtained from a subject, wherein the tumor sample may have cross-sectional area of approximately 1mm² to 4mm².

In one embodiment, in (a), 12 to 24 of the tumor samples may be cultured.

In one embodiment, a concentration of serum of the first composition may be 5 volume% or less.

In one embodiment, a concentration of serum of the first composition may be about 3 volume% or less.

In one embodiment, the medium of the first composition may be CTS-AIM-V medium.

In one embodiment, a concentration of IL-2 of the first composition may be about 1000 IU/ml.

In one embodiment, a volume of the first composition may be 30% or less of a maximum allowable capacity of the first container.

In one embodiment, the first container may be T75 flask, and a volume of the first composition may be 6ml.

In one embodiment, the first additive composition may have the same composition substance and compositional ratio as the first composition.

In one embodiment, the IL-2 concentration of the first additive composition may be about 1000 IU/ml, the serum concentration of the first additive composition may be about 3 volume%, and the medium of the first additive composition may be CTS-AIM-V medium.

In one embodiment, during the culturing, the first additive composition may be added to the first container two or more times.

Wherein, in one embodiment, during the culturing, the first additive composition may be added to the first container on the 7th, 10th, and 12th day from the start day of the culturing, wherein amount of the first additive composition added at each add point may be the same.

In one embodiment, the second immune cell population may be obtained from the first immune cell population.

In one embodiment, the second immune cell population may be obtained by a method comprising washing the first immune cell population.

In one embodiment, the medium of the second composition may be CTS-AIM-V.

In one embodiment, the serum concentration of the second composition may be 5 volume% or less.

In one embodiment, concentration of serum of the second composition may be about 3 volume%.

In one embodiment, concentration of IL-2 of the second composition may be about 2000 IU/ml.

In one embodiment, anti-CD3 antibody may be OKT3.

In one embodiment, anti-CD3 antibody concentration of the second composition may be 10 ng/ml to 100 ng/ml.

In one embodiment, anti-CD3 antibody concentration of the second composition may be about 30 ng/ml.

In one embodiment, feeder cell may be inactivated allogeneic peripheral blood mononuclear cell.

In one embodiment, feeder cell may be included in the second composition in a ratio of approximately 1:200 (number of cells of the second immune cell population:number of feeder cells) based on the number of cells in the second immune cell population.

In one embodiment, in the seeding step, the number of cells of the second immune cell population seeded in the second container may be 1E5 to 10E5.

In one embodiment, in the step of seeding, the number of cells of the second immune cell population seeded in the second container may be about 4E5.

In one embodiment, volume of the second composition may be 30% or less of the maximum allowable capacity of the second container.

In one embodiment, the second container may be GREX100M, and volume of the second composition may be about 20ml.

In one embodiment, during the expanding, immune cells may not be removed from the second container or may not be added from outside.

In one embodiment, the second additive composition may be a composition that has the same composition substances and compositional ratio as the second composition in which anti-CD3 antibody and feeder cell are excluded.

In one embodiment, IL-2 concentration of the second additive composition may be about 2000 IU/mL, serum concentration of the second additive composition may be about 3 volume%, and medium of the second additive composition may be CTS-AIM-V.

In one embodiment, during the expanding, the second additive composition may be added to the second container two or more times.

Wherein, in one embodiment, amounts of the second additive composition added may be different at each adding point.

Wherein, in one embodiment, during the expanding, the second additive composition may be added to the second container on the 4th, 6th, 8th, and 10th days from the start day of the expanding, and the amounts of the second additive composition added may be gradually increased at each add point.

Wherein, in one embodiment, the second additive composition may be added to the second container as 20ml on the 4th day from start date of the expanding, 60ml on the 6th day from start date of the expanding, 180ml on the 8th day from start date of the expanding, and 540ml on the 10th day from start date of the expanding.

In one embodiment, the number of cells of the third immune cell population may be at least 4000 times greater than the number of cells of the second immune cell population.

In one embodiment, the immune cell composition may comprise the third immune cell population.

In one embodiment, the immune cell composition may comprise a fourth immune cell population obtained from the third immune cell population.

In one embodiment, the tumor sample may be derived from solid tumor of a subject having breast cancer, colon cancer, lung cancer, stomach cancer, kidney cancer, ovarian cancer, or skin cancer including melanoma.

In one embodiment, the tumor sample may be derived from malignant pleural effusion of a subject having lung cancer.

In one embodiment, the method may further comprise: cryopreserving the first immune cell population; and thawing the cryopreserved first immune cell population, wherein cryopreserving the first immune cell population and the thawing the cryopreserved first immune cell population may be performed after (b).

In one embodiment of the present application, an immune cell composition comprising tumor infiltrating lymphocyte is provided.

In one embodiment, the immune cell composition may be produced by method for producing the immune cell composition according to an embodiment of the present application.

In one embodiment of the present application, a method for treating cancer of subject comprising administering immune cell composition comprising tumor infiltrating lymphocyte is provided. Wherein the immune cell composition may be produced by method for producing immune cell composition according to an embodiment of the present application.

In one embodiment, the subject may be a subject from whom tumor infiltrating lymphocyte is derived.

In one embodiment of the present application, a use of immune cell composition comprising a tumor infiltrating lymphocyte for treating cancer of subject is provided. Wherein the immune cell composition may be produced by a method for producing immune cell composition according to an embodiment of the present application.

In one embodiment, the subject may be a subject from whom tumor infiltrating lymphocyte is derived.

### [Disclosure of the Invention]

Hereinafter, the invention will be described in further detail through specific embodiments and examples with reference to the accompanying drawings. It should be noted that the accompanying drawings include some, but not all, embodiments. The content of the invention disclosed in the present application may be implemented in various ways and is not limited to the specific embodiments described in the present application. Various modifications and other embodiments for the content of the invention disclosed in the present application can be inferred by those of ordinary skill in the art to which the present invention belongs.

### [Definition of terms]

### Immune (cell) composition

The term "immune cell composition" or "immune composition" used in the present application refers to a composition including an immune cell population. In the present application, the immune cells encompass tumor-infiltrating lymphocytes (TILs). The tumor infiltrating lymphocyte (TIL) refers an immune cell that migrate to a tumor and live around the tumor. TILs may recognize cancer cells and kill them. TILs may include various immune cells including T cells, B cells, and natural killer cells. It is known that T cells account for the majority of TILs.

### Semi-product (half-product)

A method for producing an immune cell composition disclosed in the present application may include producing a semi-product. Here, the semi-product refers to a first immune cell population produced and obtained through isolating an immune cell population from a sample and culturing. According to conventional terminology used in the field of immune cell therapy technology, a semi-product refers to an immune cell population manufactured by a pre-Rapid Expansion Protocol (pre-REP). That is, according to conventional terminology in the field of immune cell therapy technology, a semi-product may be referred to as a pre-REP product.

### Final product

The method for producing an immune cell composition disclosed in the present application may include producing a final product (finished product). Here, the final product refers to an immune cell composition or immune composition produced by obtaining a third immune cell population expanded from a second immune cell population. According to conventional terminology used in the field of immune cell therapy technology, the final product refers to an immune cell population manufactured by a Rapid Expansion Protocol (REP). That is, according to conventional terminology used in the field of immune cell therapy technology, the final product may be referred to as a REP product. It may also be referred to as a drug product.

### Subject

As used in the present application, a subject refers to an individual having a tumor, and includes a human. The tumor may be a solid tumor and a liquid tumor. The solid tumor includes tumor in the lung tumor, breast tumor, bladder tumor, ovarian tumor, etc., and the liquid tumor includes hematological tumor such as leukemia, myeloma, lymphoma, etc., but the present application is not limited thereto.

### Container

As used in the present application, a container refers to a container used to culture tumor tissue or a tumor sample, or expand an immune cell population. In the present application, the container is used to culture or expand tumor tissue or an immune cell population, along with a composition including a medium, serum, plasma, IL-2, OKT3 and/or feeder cells. The container may be a gas-permeable container through which gas passes or a container including a gas supply system to provide a gas. That is, the container may be a container manufactured so that a gas can pass through the container, or a container that is not manufactured to allow a gas to pass through but includes a separate gas supply system to allow gas permeation. The container may be, for example, a 24-well plate, a T25, T75, or T125 flask, a flask such as a GREX 10, GREX 10M, GREX 10M-CS GREX 100, GREX 100M, GREX 100M-CS, GREX 500, GREX 500M, or GREX 500M-CS flask, or a culture bag, but the present application is not limited thereto.

### Medium

As used in the present application, a medium refers to a nutritional source for culturing cells and/or tissue. The medium may vary according to the type of object to be cultured. The medium may be AlyS505N, an AIM-V medium, or an RPMI medium, but the present application is not limited thereto. According to one embodiment the present application, the medium is supplemented with serum, plasma, IL-2, OKT3 and/or feeder cells to be used for culturing and/or expanding tissue and/or immune cells.

### Serum

As used in the present application, the term "serum" or "blood serum" refers to an additive added to a basal culture medium, the serum may contain high molecular weight proteins, low molecular weight nutrients, and components required for cell growth. The serum is human serum such as human AB serum which is commercially available, but the present application is not limited thereto. Here, the concentration of serum in the medium may be approximately 10 vol% or less, approximately 9 vol% or less, approximately 8 vol% or less, approximately 7 vol% or less, approximately 6 vol% or less, approximately 5 vol% or less, approximately 4 vol% or less, or 3 vol% or less.

### Plasma

As used in the present application, the term "plasma" or "blood plasma" refers to an additive added to a basal culture medium, and the plasma indicates fibrinogenfree serum. The plasma contains components required for cell growth. The plasma used in the present application includes plasma isolated from a patient's blood, but the present application is not limited thereto. In the present application, instead of serum, plasma may be used while being included in a medium. Here, the concentration of the plasma in the medium may be approximately 10 vol% or less, approximately 9 vol% or less, approximately 8 vol% or less, approximately 7 vol% or less, approximately 6 vol% or less, approximately 5 vol% or less, approximately 4 vol% or less, or 3 vol% or less.

### IL-2

The term "IL-2" used in the present application is an abbreviation of interleukin-2, referring to a factor that promotes T cell proliferation. The IL-2 includes various types of IL-2, such as human recombinant IL-2. In the present application, IL-2 may be included in a first composition, and the concentration of IL-2 may be approximately 10,000 IU/mL, approximately 9,000 IU/mL, approximately 8,000 IU/mL, approximately 7,000 IU/mL, approximately 6,000 IU/mL, approximately 5,000 IU/mL, approximately 4,000 IU/mL, approximately 3,000 IU/mL, approximately 2,000 IU/mL, approximately 1,000 IU/mL, or approximately 500 IU/mL. In addition, in the present application, IL-2 may be included in a second composition, together with OKT3 and feeder cells, and here, the concentration of IL-2 may be approximately 10,000 IU/mL, approximately 9,000 IU/mL, approximately 8,000 IU/mL, approximately 7,000 IU/mL, approximately 6,000 IU/mL, approximately 5,000 IU/mL, approximately 4,000 IU/mL, approximately 3,000 IU/mL, approximately 2,000 IU/mL, approximately 1,000 IU/mL, or approximately 500 IU/mL.

### Immune cell population

As used in the present application, the term "immune cell population" refers to a group or population consisting essentially of a plurality of immune cells, and the immune cells include TILs. Here, a first immune cell population means an immune cell population obtained by first culturing a subject's tumor tissue, a second immune cell population means an immune cell population, which is derived from the first immune cell population, and as needed, used by dividing the first immune cell population, and a third immune cell population means an immune cell population obtained by expanding the second immune cell population.

### Cryopreservation

As used in the present application, the term "cryopreservation" refers to storage of an immune cell population at an ultra-low temperature to inhibit damage to immune cells and preserve the immune cell population for a long period of time. For example, in the present application, the cryopreservation may be used to represent a process of storing cells or an immune cell population in a liquid nitrogen tank. In one embodiment, the cryopreservation period may be at most approximately 1 year, approximately 2 years, approximately 3 years, approximately 4 years, approximately 5 years, approximately 6 years, approximately 7 years, approximately 8 years, approximately 9 years, approximately 10 years, approximately 11 years, approximately 12 years, approximately 13 years, approximately 14 years, approximately 15 years, approximately 16 years, approximately 17 years, approximately 18 years, approximately 19 years, or approximately 20 years. To cryopreserve the immune cell population, a medium for cryopreservation is used. The medium for cryopreservation may include approximately 10% DMSO, and the medium includes a Cryostor CS10 medium, but the present application is not limited thereto. The cryopreservation may be selectively included between each step of the method for producing an immune cell composition.

### Seeding

As used in the present application, the term "seeding" refers to dispensing cells into a container, and the seeding includes dispensing a second immune cell population into a second container in the present application. For example, the seeding of the second immune cell population may be performed by suspending approximately 2E4 cells/mL in the second composition and dispensing the same in different amounts according to the type of container. In one example, 20 mL of the cell suspension may be dispensed in GREX100M, and the second immune cell population may be seeded with a cell number of approximately 4E5 cells.

### Anti-CD3 antibody

As used in the present application, the term "anti-CD3 antibody" refers to an antibody targeting a membrane protein on the T cell surface, that is, a CD3 receptor. The anti-CD3 antibody includes OKT3, and also includes all types of monoclonal and polyclonal anti-CD3 antibodies, including human, humanized and murine antibodies. The OKT3 used in the present application includes all monoclonal antibodies such as human, humanized, chimeric or murine antibodies, biosimilars, and variants thereof. Examples of other anti-CD3 antibodies include otelixizumab, teplizumab, and visilizumab.

### Feeder cells

As used in the present application, the term "feeder cells" refer to cells that are included in a composition for culturing or expanding immune cells to activate and expand immune cells. For example, in the present application, the feeder cells may be included in a second composition to activate and expand immune cells of the second composition. The feeder cells include peripheral blood mononuclear cells (PBMCs) which refer to peripheral blood cells with round nuclei. The PBMCs used in the present application include irradiated allogeneic peripheral blood mononuclear cells.

### Expansion

As used in the present application, the term "expansion" means that a cell population or immune cell population is increased by a predetermined fold or more over a predetermined period of time.

### Approximately

As used in the present application, the term "approximately" or "about" means being in a statistically significant range. Unless expressed otherwise, the term "approximately" or "about" used in the present application means having a value in a range within 20% or 10% of a given value.

### Including/comprising

The term "including" used in the present application means not to exclude optionally added materials or steps.

### [Immune cell therapy]

### Summary

Cancer is a group of diseases in which cell division continues due to an uncontrollable cell cycle, including lung cancer, breast cancer, and colorectal cancer. Recently, as one of the methods of treating cancer, cancer immunotherapy is being studied, and cancer immunotherapy refers to a method of treating cancer by activating the immune system of the human body. As a cancer immunotherapeutic used for cancer immunotherapy, immune cell therapy is a cell therapeutic method that collects immune cell from the body, reinforces them or genetically modifies them, and put again into the body.

This method is called adoptive cell transfer (ACT), classified into a treatment using TILs, and a treatment using T cell receptor (TCR)-expressing T cells or chimeric antigen receptor (CAR)-expressing T cells, which uses T cells modified through genetic engineering. In one embodiment of the present application, a therapeutic method using TILs is disclosed.

### Tumor-infiltrating lymphocytes (TILs) and therapy using the same

Tumor-infiltrating lymphocytes (TILs) refer to lymphocytes that gather around cancer cells. Treatments using TILs involves harvesting naturally occurring T cells that have infiltrated the patient's tumor, activating and amplifying these T cells, and then reintroducing a large number of the activated T cells into the patient to suppress or destroy the tumor.

### Conventional method for producing immune cell composition

### Summary

A process for producing an immune cell composition, that is, an immune cell composition including TILs, generally includes the following steps.

### 1. Sample processing

Generally, to obtain an immune cell composition including TILs, a step of processing a sample is first performed. Here, the sample means a tumor sample obtained from a patient, and the tumor sample is cut into small fragments in the sample processing step.

### 2. Culture

Generally, after the processing of a tumor sample, the acquired small tumor fragment is cultured in a medium for culturing the tumor. Generally, the culture is performed in a medium containing a high concentration of IL-2. Through the culturing step, a first immune cell population is obtained.

### 3. Expansion

The first immune cell population that has undergone the culturing step goes through an expansion step. Generally, the expansion step may be referred to as a rapid expansion protocol (REP). The expansion step is generally performed in a medium containing a high concentration of IL-2, anti-CD3 antibody, and feeder cells.

### 4. Acquisition and formulation

The immune cell population that has undergone the expansion step is acquired in a non-contaminated manner and is formulated by transferring it to a container suitable for administration to a patient.

### [Limitations of prior arts]

To treat cancer using TILs, first, TILs must be isolated, cultured, and expanded from cancer tissue. A series of processes for isolating TILs from cancer tissue, culturing, and then expanding are considerably complicated, and such a series of processes may affect the yield of TILs. Accordingly, conventional methods have problems with low yields because each process is not optimized or standardized, or has low efficiency. To overcome this, it is necessary to optimize and standardize each process to create a method with higher efficiency and a higher yield than the conventional methods. Therefore, a method for producing an immune cell composition provided in the present application have been developed to obtain a high yield of TILs by optimizing and standardizing a series of processes for TIL culture and expansion.

### [Method for producing immune cell composition disclosed in the present application and use of immune cell composition]

The present application discloses a method for producing an immune cell composition (immune composition).

In one embodiment, the method for producing an immune cell composition of the present application may include any one or more of the following processes:
production of a semi-finished product; cryopreservation; thawing of a cryopreserved first immune cell population; and production of a final product.

In one embodiment, the method for producing an immune cell composition may include: culturing a tumor sample; obtaining a first immune cell population; seeding a second immune cell population; expanding the second immune cell population; and producing an immune cell composition.

In one embodiment, the method for producing an immune cell composition may include: culturing a tumor sample; obtaining a first immune cell population; cryopreserving the first immune cell population; thawing the cryopreserved first immune cell population; seeding a second immune cell population; expanding the second immune cell population; and producing an immune cell composition.

Each process that can be included in the method for producing an immune cell composition of the present application will be disclosed in detail below.

### I. Producing semi-finished product

The method for producing an immune cell composition disclosed in the present application may include a process of producing a semi-product and/or a process of producing a final product. Here, the semi-product refers to a first immune cell population obtained from a cultured composition after culturing a tumor sample. The process of producing a semi-product may include contents described below. This step may correspond to a pre-rapid expansion protocol (pre-REP) in accordance with conventional terminology in the field of immune cell therapy.

In one embodiment, the production of a semi-product may include:
culturing a tumor sample; and
obtaining a first immune cell population.

In one embodiment, the production of a semi-product may further include preparing a tumor sample. Here, the preparing a tumor sample is performed prior to the culturing a tumor sample.

Each process included in the producing of a semi-product will be described in detail below.

### 1. Preparing tumor sample

### Overview

To acquire a first immune cell population, first, a tumor sample must be prepared. Here, the tumor sample is derived from the tumor tissue of a subject, and includes tumor infiltrating lymphocytes (TILs) with the characteristics of existing around cancer cells.

In one embodiment, the preparing of a tumor sample may include processing the tumor tissue obtained from the subject. In another embodiment, the tumor tissue obtained from the subject may be used as a tumor sample without further processing.

The process of preparing a tumor sample will be described in detail below.

### Obtaining tumor tissue

Tumor tissue used in the preparing of a tumor sample is acquired from a subject with a tumor. Here, the subject with a tumor may include a human with a tumor. The obtaining tumor tissue may be directly performed by a practitioner of the present invention, or the obtaining tumor tissue may be performed by obtaining the tumor tissue from a hospital.

In one embodiment, the tumor tissue may be obtained from a human with a solid tumor or a liquid tumor. The human having a solid tumor may be, for example, a patient with breast cancer, colorectal cancer, lung cancer, stomach cancer, kidney cancer, ovarian cancer, or skin cancer including melanoma, but the present application is not limited thereto. The human with a liquid tumor may be, for example, a patient with leukemia or blood cancer including lymphoma, but the present application is not limited thereto.

Wherein, the tumor tissue includes Tumor infiltrating lymphocytes (TILs).

The tumor tissue is collected from a subject via various routes. For example, the tumor tissue may be obtained by collecting a tumor of the subject (e.g., a cancer patient). As another example, the tumor tissue may be obtained by collecting a malignant pleural effusion, observed in cancer patients. As a specific example, in case of a lung cancer patient, the tumor tissue acquired from a subject may be obtained by collecting the pleural effusion of the patient.

The tumor tissue may be obtained by methods known in the art. In one embodiment, the tumor tissue may be obtained by a biopsy. The biopsy includes an excisional biopsy for removing part of a tumor, and an aspiration biopsy using the needle of a syringe, but the present application is not limited thereto. The tumor tissue may be obtained by a biopsy as well as other methods known in the art. In one embodiment, the tumor tissue may be obtained via an excisional biopsy for removing part of the tumor of patient with a breast cancer and/or lung cancer.

In one embodiment, the tumor tissue may be breast cancer tissue and/or lung cancer tissue obtained from a breast cancer and/or lung cancer patient.

### Processing tumor tissue

In one embodiment, the tumor tissue obtained from a subject may be prepared as a tumor sample after has undergoing further processing, or the tumor tissue obtained from a subject may be prepared as a tumor sample without any processing.

The processing of the tumor tissue will be described in detail below.

In one embodiment, the processing of the tumor tissue may include washing, removing adipose tissue, and/or forming small fragments, but the present application is not limited thereto.

### 1) Washing

The processing of the tumor tissue may include washing the tumor tissue. In one embodiment, the washing may include washing the tumor tissue with a medium. In one embodiment, the washing may include removing an antibiotic from the tumor tissue that has been transferred to a transport medium containing the antibiotic.

In one embodiment, the tumor tissue may be washed with a medium once, twice, three, four, five times or more. In a specific embodiment, the tumor sample may be washed two or three times using a medium.

In one embodiment, the tumor tissue may be washed using a medium that does not comprise antibiotic. In a specific embodiment, the tumor tissue may be washed with an antibiotic-free RPMI1640 medium. In a specific embodiment, the tumor tissue may be washed with an antibiotic-free RPMI1640 medium three times.

### 2) Removing adipose tissue

The processing of the tumor tissue may include removing adipose tissue. Here, the removing of adipose tissue may be performed or omitted depending on whether the tumor sample contains a lot of fat. The removing of adipose tissue includes detaching adipose tissue from the tumor tissue. In one embodiment, the removing of adipose tissue may be performed using tweezers and scissors.

### 3) Forming tumor tissue into small fragments

The processing of the tumor tissue may include forming the tumor tissue into small fragments. The tumor tissue may be formed into small fragments using a cutter (e.g., scissors or knife), or a solution containing a digestive enzyme, but the present application is not limited thereto.

In one embodiment, the tumor tissue may be cut such that the width of one side is approximately 0.1 to 10 mm, approximately 0.4 to 5 mm, approximately 0.6 to 4 mm, approximately 0.8 to 3 mm, or approximately 1 to 2 mm. In a specific embodiment, the tumor tissue may be cut such that the width of one side is approximately 1 to 2 mm.

In one embodiment, the tumor tissue may be cut such that a cross-sectional area is approximately 0.01 to 100 mm², approximately 0.1 to 50 mm², approximately 0.2 to 10 mm², approximately 0.5 to 5 mm², approximately 0.7 to 4 mm², or approximately 1 to 2 mm². In a specific embodiment, the tumor tissue may be cut to have a cross-sectional area of approximately 1 to 2 mm².

In another embodiment, the tumor tissue may be formed into small fragments by a digestive solution including a digestive enzyme, and here, the tumor tissue may be cut into small fragments by a digestive solution including an enzyme such as collagenase.

### 2. Culturing tumor sample

### Overview

In this step, the tumor sample is cultured.

In one embodiment, a first immune cell population may be produced by culturing the tumor sample. The process of producing a first immune cell population by culturing the tumor sample will be described in detail below.

### Culturing tumor sample

The tumor sample is cultured in a first composition in a first container. The first immune cell population may be produced by culturing the tumor sample.

Here, the tumor sample is cultured in an environment suitable for culture. In one embodiment, the tumor sample may be cultured in a 37 °C, 5% CO₂ environment.

### 1) Number of tumor sample fragments contained in first container

A predetermined number of tumor sample fragments, which have been cut are placed and cultured in the first composition of the first container. Here, the number of tumor sample fragments placed in the first container may vary according to the cell culture area or volume of the first container.

In one embodiment, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 32, 34, 36, 38, 40, 42, 44, 46, 48, or 50 the tumor sample fragments may be placed and cultured in the first container. In a specific embodiment, 12, 16, 20, 24, 28, or 32 tumor sample fragments may be placed and cultured in the first container.

In one embodiment, when the first container is a T75 flask, 12, 16, 20, 24, 28, or 32 tumor sample fragments may be placed and cultured in the first container. In a specific embodiment, when the first container is a T75 flask, 24 tumor sample fragments may be placed and cultured in the first container.

### 2) First container

The tumor sample is cultured in the first composition in the first container, and here, the first container means a container used in culturing of the tumor sample. The first container in the present application is used to culture the tumor sample, together with a composition that includes a medium, serum, and IL-2.

The container may be a gas-permeable container through which a gas passes, or a container that includes a gas supply system to provide a gas. That is, the container may be a container manufactured so that a gas can pass through the container, or a container that is not manufactured to allow a gas to pass through but includes a separate gas supply system to allow gas permeation.

In one embodiment, the first container may be a container having a maximum allowable capacity of approximately 1, 2, 3, 4, 5, 6, 7, 8, 9, 10, 12, 14, 16, 18, 20, 22, 24, 26, 28, 30, 35, 40, 45, 50, 55, 60, 70, 80, 90, 100, 120, 140, 160, 180, 200, 220, 240, 260, 280, 300, 350, 400, 450, 500, 550, 600, 700, 800, 900, 1000, 1100, 1200, 1300, 1400, 1600, 1800, 2000, 2500, 3000, 3500, 4000, 4500, 5000, 6000, 7000, 8000, 9000, or 10000 mL, or a container having a maximum allowable capacity within a range set by two values selected from aforesaid values. In a specific embodiment, the first container may be a container having a maximum allowable capacity of approximately 1, 2, 3, 4, 5, 6, 7, 8, 9, 10, 20, 22, 24, 26, 28, 30, 35, 40, 45, 50, 55, 60, 70, 80, 90, or 100 mL, or a container having a maximum allowable capacity within a range set by two values selected from aforesaid values.

In one embodiment, the container may be, for example, a 24-well plate, a flask such as a T25, T75, or T125 flask, a GREX 10, GREX 10M, GREX 10M-CS, GREX 100, GREX 100M, GREX 100M-CS, GREX 500, GREX 500M, or GREX 500M-CS flask, or a culture bag, but the present application is not limited thereto.

In one embodiment, the tumor sample may be cultured in a T75 flask. In a specific embodiment, 24 of tumor sample fragments may be cultured in a T75 flask.

### 3) First composition

In the first container, the tumor sample is cultured in the first composition. Here, the first composition includes any one or more of a culture medium, serum, plasma, an antibiotic, IL-2, and an antibody.

In one embodiment, the first composition may include a medium, serum, and IL-2. In another embodiment, the first composition may include a medium, plasma, and IL-2.

In one embodiment, the first composition may include a medium; any one of serum and plasma; and IL-2, and may further include any one or more of an antibody and an antibiotic.

In a specific embodiment, the first composition includes a medium, serum, and IL-2. In another specific embodiment, the first composition includes a medium, serum, IL-2, and an antibiotic. In still another specific embodiment, the first composition includes a medium, serum, IL-2, and an antibody. In yet another specific embodiment, the first composition includes a medium, serum, IL-2, an antibiotic, and an antibody.

Generally, in culturing tissue or cells, the volume of the composition used in a container (e.g., flask) may vary according to the type of container. For example, for a 24-well plate, generally, 2 mL of the composition is used, and for a T75 flask, generally, 20 mL of the composition is used.

However, to culture the tumor sample of the present application, the amount of the first composition initially added to the first container may be different from the above-described amount, which is generally used.

In one embodiment, the amount of the first composition used to culture the tumor sample (that is, added to the first container) may be approximately 80%, 70%, 60%, 50%, 45%, 40%, 35%, 30%, 25%, 20%, 15%, 10%, 9%, 8%, 7%, 6%, or 5% or less of the maximum allowable capacity of the first container. In a specific embodiment, the amount of the first composition added to the first container for culturing the tumor sample may be approximately 30% or less of the maximum allowable capacity of the first container. Here, the maximum allowable capacity means the maximum capacity that can be added to the first container.

In one embodiment, when the first container is a 24-well plate, the amount of the initially added first composition may be 1.5 mL or less or 1 mL or less. In a specific embodiment, when the first container is a 24-well plate, the amount of the initially added first composition may be approximately 1 mL.

In another embodiment, when the first container is a T75 flask, the amount of the used first composition may be 15 mL, 12 mL, 10 mL, 9 mL, 8 mL, 7 mL, 6 mL, 5 mL, 4 mL, or 3 mL or less. In a specific embodiment, when the first container is a T75 flask, the amount of the used first composition may be approximately 6 mL.

The amount of the initial first composition of the present application may affect the culturing environment of the tumor sample, thereby helping to optimize the production of the first immune cell population. Specifically, when the amount of the initial composition is less than that of the maximum allowable amount of the composition, a material required for culture may be provided by simply adding without replacing the composition during the culture, thereby reducing the stress that the cultured cells receive. In addition, the control of the amount of the composition may have a positive effect on the proliferation of the cultured cells by affecting the interaction between the cells.

The medium, serum, plasma, antibiotic and/or IL-2 that may be included in the first composition will be described below.

### i) Medium

### a) Type of basal medium

A basal medium used in the present application refers to the minimum nutritional source for culturing cells and/or tissue. As the basal medium, any medium suitable for culturing immune cells including TILs may be used.

In one embodiment, the medium may be an AlyS505N medium, an AIM-V medium, or an RPMI1640 medium, but the present application is not limited thereto. In a specific embodiment, the tumor sample may be cultured using a CTS-AIM-V medium.

In one embodiment, the medium included in the first composition may be an AlyS505N medium, an AIM-V medium, or an RPMI1640 medium. In a specific embodiment, the medium included in the first composition may be a CTS-AIM-V medium.

### ii) Additives

### a) Serum

In one embodiment, serum may be added to the basal culture medium (e.g., the above-described AlyS505N, AIM-V, or RPMI1640 medium). In one embodiment, the first composition of the present application may include serum. The serum may contain high molecular weight proteins and low molecular weight nutrients, and contains components required for cell growth. The serum includes human serum, but the present application is not limited thereto. In one embodiment, the serum may be human AB serum.

In one embodiment, approximately 10 vol% or less of human serum may be added to the medium. In another embodiment, approximately 9 vol% or less, approximately 8 vol% or less, approximately 7 vol% or less, approximately 6 vol% or less, approximately 5 vol% or less, approximately 4vol% or less, or 3 vol% or less of human serum may be added to the medium. In a specific embodiment, approximately 3 vol% of human serum may be added to a CTS-AIM-V medium and used to culture the tumor fragment.

In one embodiment, when the serum is included in the first composition, the concentration of the serum included in the first composition may be approximately 20, 19, 18, 17, 16, 15, 14, 13, 12, 11, 10, 9, 8, 7, 6, 5, 4, 3, 2, or 1 vol% or less. In a specific embodiment, the concentration of the serum included in the first composition may be approximately 10, 9, 8, 7, 6, 5, 4, 3, 2, or 1 vol% or less. In a specific embodiment, the concentration of the serum included in the first composition may be approximately 5, 4, 3, 2, or 1 vol% or less.

In one embodiment, the concentration of human serum included in the first composition may be approximately 5, 4, 3, 2, or 1 vol%. In a specific embodiment, the concentration of human serum included in the first composition may be approximately 3 vol%.

### b) Plasma

In one embodiment, plasma may be added to the basal culture medium. In one embodiment, the first composition of the present application may include plasma. The plasma refers to that fibrinogen is excluded from the serum. The plasma may contain components required for cell growth.

The plasma used in the present application may be human plasma or plasma isolated from a patient's blood, but the present application is not limited thereto.

In one embodiment, plasma, instead of serum, may be added to the medium and used.

In one embodiment, approximately 10 vol% or less of plasma may be added to the medium and used. In one embodiment, approximately 9 vol% or less, approximately 8 vol% or less, approximately 7 vol% or less, approximately 6 vol% or less, approximately 5 vol% or less, approximately 4 vol% or less, or 3 vol% or less of plasma may be added to the medium and used. In a specific embodiment, approximately 3 vol% of plasma may be added to a CTS-AIM-V medium and used to culture the tumor fragment.

In one embodiment, when the plasma is included in the first composition, the concentration of the plasma included in the first composition may be approximately 20, 19, 18, 17, 16, 15, 14, 13, 12, 11, 10, 9, 8, 7, 6, 5, 4, 3, 2, or 1 vol% or less. In a specific embodiment, the concentration of the plasma included in the first composition may be approximately 10, 9, 8, 7, 6, 5, 4, 3, 2, or 1 vol% or less. In a specific embodiment, the concentration of the plasma included in the first composition may be approximately 5, 4, 3, 2, or 1 vol% or less.

In one embodiment, the concentration of plasma included in the first composition may be approximately 5, 4, 3, 2, or 1 vol%. In a specific embodiment, the concentration of plasma included in the first composition may be approximately 3 vol%.

### c) IL-2

IL-2 (interleukin-2) used in the present application refers to a factor that promotes T-cell proliferation. The IL-2 includes various types of IL-2, including the human recombinant IL-2 form.

In one embodiment, the first composition may include IL-2.

In one embodiment, the concentration of IL-2 added to the medium may be approximately 10,000 IU/mL or less. In one embodiment, approximately 9,000 IU/mL, approximately 8,000 IU/mL, approximately 7,000 IU/mL, approximately 6,000 IU/mL, approximately 5,000 IU/mL, approximately 4,000 IU/mL, approximately 3,000 IU/mL, approximately 2,000 IU/mL, approximately 1,000 IU/mL, or approximately 500 IU/mL of IL-2 may be added to the medium. In a specific embodiment, approximately 1,000 IU/mL of IL-2 may be added to a CTS-AIM-V medium and used to culture the tumor fragment.

In one embodiment, the first composition may include approximately 10,000 IU/mL, approximately 9,500 IU/mL, approximately 9,000 IU/mL, approximately 8,500 IU/mL, approximately 8,000 IU/mL, approximately 7,500 IU/mL, approximately 7,000 IU/mL, approximately 6,500 IU/mL, approximately 6,000 IU/mL, approximately 5,500 IU/mL, approximately 5,000 IU/mL, approximately 4,500 IU/mL, approximately 4,000 IU/mL, approximately 3,500 IU/mL, approximately 3,000 IU/mL, approximately 2,500 IU/mL, approximately 2,000 IU/mL, approximately 1,500 IU/mL, approximately 1,000 IU/mL, approximately 900 IU/mL, approximately 800 IU/mL, approximately 700 IU/mL, approximately 600 IU/mL, approximately 500 IU/mL, approximately 400 IU/mL, approximately 300 IU/mL, approximately 200 IU/mL, or approximately 100 IU/mL of IL-2, or IL-2 at a concentration in the range determined by two values selected from the aforesaid values. In a specific embodiment, the first composition may include approximately 2,000 IU/mL, approximately 1,500 IU/mL, approximately 1,000 IU/mL, approximately 900 IU/mL, approximately 800 IU/mL, approximately 700 IU/mL, approximately 600 IU/mL, or approximately 500 IU/mL of IL-2, or IL-2 at a concentration within a range set by two values selected from aforesaid values. In a specific embodiment, the first composition may include approximately 1,000 IU/mL of IL-2.

### d) Antibiotic

In one embodiment, the first composition may further include an antibiotic.

The antibiotic may be used to prevent contamination by bacteria or fungi during cell culture and/or expansion.

Whether or not to use an antibiotic in the production of a semi-product may be determined by appropriately considering the type of tumor of tumor tissue acquired from a subject, the condition (state) of the cultured tumor sample, and the condition of the cultured and/or expanded cells. For example, when the tumor sample is derived from breast cancer of a subject, in the initially added first composition, an antibiotic may not be included. In another example, although the tumor sample is derived from the breast cancer of a subject, a first composition comprising an antibiotic may be used depending on the condition of the tumor sample. In still another example, when the tumor sample is derived from head-and-neck cancer of a subject, the first composition comprising antibiotic may be used.

When the first composition includes the antibiotic, the antibiotic may be included at a concentration that does not affect cell culture and/or expansion, and here, the concentration of the antibiotic exhibiting cytotoxicity may be considered. An appropriate concentration of the antibiotic included in the first composition may vary according to the type of antibiotic.

Examples of the antibiotics included in the first composition include ampicillin, gentamycin, kanamycin, neomycin, penicillin, and streptomycin, but the present application is not limited thereto. That is, as the antibiotic that is added to the medium for cell culture and/or expansion, any antibiotic known in the art may be used, and may be used after being appropriately selected by those of ordinary skill in the art as needed. In a specific embodiment, the first composition may include gentamycin.

### e) Antibody

As described above, in one embodiment of the present application, an antibody may be further included in the first composition. When an antibody is included in the first composition, the antibody included in the first composition may be any one or more selected from an antibody targeting CD137(4-1BB), and an antibody blocking the binding of PD-1 and PD-L1. In one embodiment, the antibody included in the first composition may be any one or more selected from an anti-CD137 antibody, anti-PD-1 antibody, and anti-PD-L1 antibody.

The anti-CD137 antibody is an antibody targeting CD137 (4-1BB). In one embodiment, the anti-CD137 antibody includes any type of monoclonal or polyclonal anti-CD137 antibody, such as a human, humanized or murine antibody. In one embodiment, when the anti-CD137 antibody is included in the first composition, the anti-CD137 antibody may be any one or more selected from urelumab, utomilumab, and a variant thereof, but the present application is not limited thereto.

As an example of antibody hindering the binding of PD-1 and PD-L1 may include anti-PD-1 antibody or anti-PD-L1 antibody. In one embodiment, the anti-PD-1 antibody or anti-PD-L1 antibody includes any type of monoclonal or polyclonal anti-PD-1 antibody or PD-L1 antibody, including a human, humanized or murine antibody. In one embodiment, when the anti-PD-1 antibody is included in the first composition, the anti-PD-1 antibody may be any one or more selected from nivolumab, pembrolizumab, cemiplimab, dostarlimab, and a variant thereof, but the present application is not limited thereto. In one embodiment, when the anti-PD-L1 antibody is included in the first composition, the anti-PD-L1 antibody may be any one or more selected from atezolizumab, avelumab, durvalumab, and a variant thereof, but the present application is not limited thereto.

In one embodiment, when the antibody is included in the first composition, the concentration of the antibody included in the first composition may be approximately 1 ng/mL, approximately 10 ng/mL, approximately 100 ng/mL, approximately 1 µg/mL, approximately 2 µg/mL, approximately 3 µg/mL, approximately 4 µg/mL, approximately 5 µg/mL, approximately 10 µg/mL, approximately 15 µg/mL, approximately 20 µg/mL, approximately 25 µg/mL, approximately 30 µg/mL, approximately 35 µg/mL, approximately 40 µg/mL, approximately 45 µg/mL, approximately 50 µg/mL, approximately 60 µg/mL, approximately 70 µg/mL, approximately 80 µg/mL, approximately 90 µg/mL, or approximately 100 µg/mL, or a value included in a range set by two values selected from aforesaid values. In a specific embodiment, the concentration of the antibody included in the first composition may be approximately 10 µg/mL.

As described above, the first composition of the present application includes a medium and additives.

In one embodiment, the first composition may include a medium, serum, and IL-2. In another embodiment, the first composition may include a medium, plasma, and IL-2. In still another embodiment, the first composition may include a medium, serum, IL-2, and an antibiotic. In yet another embodiment, the first composition may include a medium, plasma, IL-2, and an antibiotic. However, the present application is not limited thereto.

In a specific embodiment, the first composition may include a medium, serum, and IL-2, wherein the medium may be a CTS-AIM-V medium, approximately 3 vol% of human AB serum as the serum may be included in the first composition, and approximately 1,000 IU/mL of IL-2 may be included in the first composition. In a specific embodiment, the first composition may include a medium, serum, IL-2, and an antibiotic. Here, in the first composition, the medium may be a CTS-AIM-V medium, the antibiotic may be gentamycin, the serum may be approximately 3 vol% of human AB serum, and IL-2 may be included at approximately 1,000 IU/mL.

### 4) Culture period

The tumor sample is cultured for a predetermined period in a first container. In one embodiment, the tumor sample is cultured for approximately 1 day or more. In one embodiment, the tumor sample may be cultured for approximately 2 days or more, 3 days or more, 4 days or more, 5 days or more, 6 days or more, 7 days or more, 8 days or more, 9 days or more, 10 days or more, 11 days or more, 12 days or more, 13 days or more, 14 days or more, 15 days or more, 16 days or more, 17 days or more, 18 days or more, 19 days or more, 20 days or more, 21 days or more, 22 days or more, 23 days or more, or 24 days or more. In a specific embodiment, the tumor sample may be cultured in the first container for approximately 7, 8, 9, 10, 11, 12, 13, 14, 15, 16, 17, 18, 19, 20, or 21 days. In a specific embodiment, the tumor sample may be cultured in the first container for approximately 14 days.

### 5) Adding first additive composition

While the tumor sample is cultured in the first container, a first additive composition may be added to the first container. Here, the first additive composition may include any one or more of a medium, serum, plasma, an antibiotic, and IL-2. The configuration of the first additive composition is disclosed in detail in related paragraphs.

In one embodiment, the first additive composition may be added 1, 2, 3, 4, 5, 6, 7, 8, 9, or 10 times, or a number of times greater than these number of times. In a specific embodiment, the first additive composition may be added three times.

Here, in one embodiment, the first additive composition may be added on any one or more days selected from day 1, 2, 3, 4, 5, 6, 7, 8, 9, 10, 11, 12, 13, 14, 15, 16, 17, 18, 19, 20, and 21 after the start date of culture, and added 1, 2, 3, 4, 5, 6, 7, 8, 9, or 10 times during the culture period. In a specific embodiment, the first additive composition may be added three times on day 7, 10, and 12.

In the process of adding the first additive composition, the first additive composition may be added by a method for minimizing the stress of the environment in which the tumor sample is cultured.

In the process of adding the first additive composition, the amount of the added first additive composition may vary according to the type of container and/or an addition time.

In one embodiment, the first additive composition may be added in the same amount at each addition time. In another embodiment, the first additive composition may be added in a different amount at each addition time. In another embodiment, the first additive composition may be added by increasing its addition amount at each addition time.

In one embodiment, the first additive composition may be added in the same amount or different amount of approximately 1 mL, 2 mL, 3 mL, 4 mL, 5 mL, 6 mL, 7 mL, 8 mL, 9 mL, 10 mL, 12 mL, 14 mL, 16 mL, 18 mL, 20 mL, 24 mL, 28 mL, 32 mL, 36 mL, 40 mL, 45 mL, or 50 mL, or may be added in an amount greater than or equal to aforesaid values at each addition time, but the present application is not limited thereto. The amount of the added first additive composition may be determined as an appropriate value in consideration of various conditions (e.g., the capacity of a container, the condition of the cultured cells, and/or addition time). In a specific embodiment, the first additive composition may be added in the same amount of 3 mL at each addition time. In a specific embodiment, when the first container is a T75 flask, the first additive composition may be added in the same amount of 3 mL at each addition time.

In a specific embodiment, the first additive composition may be added to the first container on day 7, 10, and 12 from the start of culture, wherein 3 mL of the first additive composition may be added at each addition time.

In one embodiment, the first additive composition added to the first container may include any one or more selected from a medium, serum, plasma, an antibiotic, IL-2, and an antibody.

In one embodiment, the first additive composition may include a medium, serum, and IL-2. In another embodiment, the first additive composition may include a medium, plasma, and IL-2.

In one embodiment, the first additive composition may include a medium; any one of serum and plasma; and IL-2, and further include any one or more of an antibody and an antibiotic.

In a specific embodiment, the added first additive composition includes a medium, serum, and IL-2. In a specific embodiment, the first additive composition includes a medium, serum, IL-2, and an antibiotic. In another specific embodiment, the first additive composition includes a medium, serum, IL-2, and an antibody. In a specific embodiment, the first additive composition includes a medium, serum, IL-2, an antibiotic, and an antibody.

Embodiments for the components included in the first additive composition are the same as in the description of the first composition.

### a) Medium

Embodiments related to the medium that can be included in the first additive composition are as described above in the first composition. For example, in a specific embodiment, the medium included in the first additive composition may be an AIM-V medium, but the present application is not limited thereto. In a specific embodiment, the medium included in the first additive composition may be a CTS-AIM-V medium.

### b) Serum

When serum is included in the first additive composition, embodiments related to the serum are as described above in the first composition. For example, in a specific embodiment, the serum may be human serum, but the present application is not limited thereto. For example, in a specific embodiment, the concentration of the serum included in the first additive composition may be approximately 3 vol%, but the present application is not limited thereto.

### c) Plasma

When plasma is included in the first additive composition, embodiments related to the plasma are as described above in the first composition. For example, in a specific embodiment, the plasma may be human plasma, but the present application is not limited thereto. For example, in a specific embodiment, the concentration of the plasma included in the first additive composition may be approximately 3 vol%, but the present application is not limited thereto.

### d) Antibiotic

When an antibody is included in the first additive composition, embodiments related to the antibiotic are as described above in the first composition. As described above, whether or not to use an antibiotic may be determined by appropriately considering the type of tumor acquired from a subject, the state of the cultured tumor sample, culture and/or the state of the expanded cells. For example, in a specific embodiment, the antibiotic included in the first additive composition may be gentamycin.

### e) IL-2

When IL-2 is included in the first additive composition, embodiments related to IL-2 are as described above in the first composition. For example, in a specific embodiment, the concentration of IL-2 included in the first additive composition may be 1,000 IU/mL.

### f) Antibody

When an antibody is included in the first additive composition, embodiments related to the antibody are as described above in the first additive composition. For example, the antibody included in the first additive composition may be any one or more selected from anti-CD137 antibody, anti-PD-1 antibody, and anti-PD-L1 antibody.

In one embodiment, the configuration(constituent) of the first additive composition added to the first container may be different for each addition time. In another embodiment, the configuration of the first additive composition added to the first container may be the same at every addition time.

In one embodiment, the configuration of the first additive composition added to the first container may be the same as that of the first composition. In another embodiment, the configuration of the first additive composition added to the first container may be different from that of the first composition.

In a specific embodiment, the first additive composition added to the first container may include a medium, serum, and IL-2. In a specific embodiment, the first additive composition added to the first container may include a CTS-AIM-V medium, approximately 3 vol% human serum, and approximately 1,000 IU/mL of IL-2.

In one embodiment, during culture, immune cells are not removed from the first container, or are not added from the outside. This means that, during culture, immune cells are not transferred to a container, other than the first container, or immune cells are not newly added to the first container from the outside.

A specific example for production of a first immune cell population may be described as below, but the present application is not limited thereto:
- 24 fragments of the tumor sample with a cross-sectional area of 1 to 4 mm² are cultured in a T75 flask, and 6 mL of the first composition is used at the beginning of the culture. Here, the first composition includes a CTS-AIM-V medium, 3 vol% human serum, and 1,000 IU/mL of IL-2. The tumor sample is cultured in a 37 °C, 5% CO₂ environment in the first container for 14 days.
- On day 7, 10, and 12 from the start date of the culture, the first additive composition having the same composition as the first composition is added to the first container, and here, the added first additive composition is added at 3 mL each on day 7, 10, and 12.

### 3. Acquiring first immune cell population

### Overview

The first immune cell population is obtained from the culture product obtained through the culturing of the above-described tumor sample.

### Acquiring first immune cell population

An immune cell population refers to a group comprising substantially a plurality of immune cells, and the immune cells include TILs. Here, the first immune cell population refers to an immune cell population obtained by culturing the tumor sample.

The method of acquiring a first immune cell population includes various known methods for acquiring cells produced in the process of culturing cells using tissue, but the present application is not limited thereto. In one embodiment, the first immune cell population may be obtained through filtering of tumor tissue, washing cells, processing, isolating, and/or cell sorting from the composition cultured by the process of culturing the tumor sample, but the present application is not limited thereto. The first immune cell population may be acquired by a method known in the art.

Here, exemplary processes that can be included in the obtaining the first immune cell population will be disclosed.

### 1) Filtering (straining) tumor tissue

The acquiring of the first immune cell population may include filtering tumor tissue fragments. In one embodiment, to acquire the first immune cell population from the cultured composition, the filtering of tumor tissue fragments may be performed using a cell strainer. Here, the size of a mesh pore of the cell strainer may vary according to the type of acquired immune cells. In a specific embodiment, a fragment of tumor tissue may be strained using the cell strainer with a mesh pore of 40 µm.

### 2) Cell collection

Obtaining the first immune cell population may include acquiring or collecting cells. As a method for acquiring or collecting cells from the cultured composition or the strained cell solution, a method known in the art may be used. In one embodiment, to acquire or collect the first immune cell population, centrifugation may be used. In one example, the centrifugation may be performed under the condition of approximately 100 g, 150 g, 200 g, 250 g, 300 g, 400 g, 450 g, 500 g, 600 g, 700 g, 800 g, 900 g, or 1,000 g, or a g value greater than or equal to aforesaid g values, but the preset application is not limited thereto. In another example, the centrifugation may be performed under a condition of approximately 100 RPM, 200 RPM, 300 RPM, 400 RPM, 500 RPM, 600 RPM, 700 RPM, 800 RPM, 900 RPM, 1000 RPM, 1100 RPM, 1200 RPM, 1300 RPM, 1400 RPM, 1500 RPM, 1600 RPM, 1700 RPM, 1800 RPM, 1900 RPM, 2000 RPM, 2200 RPM, 2500 RPM, 3000 RPM, 3500 RPM, 4000 RPM, or 5000 RPM, or an RPM value greater than or equal to aforesaid RPM values, but the present application is not limited thereto.

### 3) Cell sorting

Obtaining the first immune cell population may include sorting cells included in the cultured composition.

Cell sorting means a method used to "select desired cells" from the above-described isolated cell population. For cell sorting, a method conventionally used in the art may be used. For example, a method such as fluorescence-activated cell sorting (FACS), centrifugation, or isolation according to cell adhesion to a container may be used for cell sorting.

### 4) Cell washing

The acquiring of the first immune cell population may include washing an immune cell population included in the cultured composition. The process of washing cells that is described in this paragraph may be performed any number of times in the step of obtaining the first immune cell population. For example, cell washing may be performed between the processes included in the obtaining the first immune cell population. In another example, cell washing may be performed on the cell population that has been strained, collected and/or sorted as described above. In still another example, cell washing may be performed concurrently with the cell collection.

In one embodiment, the cultured immune cell population may be washed with a buffer solution including PBS or DPBS. The cultured immune cell population may be washed 1, 2, 3, 4 or 5 times or more. Here, for example, centrifugation may be used. In a specific embodiment, the cultured immune cell population may be washed with DPBS three times to obtain the first immune cell population.

In one embodiment, the obtaining the first immune cell population may include cell collection. Here, the obtaining the first immune cell population may further include cell washing, and the cell washing may be performed any appropriate number of times at any appropriate time.

In one embodiment, the obtaining the first immune cell population may include: filtering tumor tissue; and cell collection. Here, the obtaining the first immune cell population may further include cell washing, and the cell washing may be performed any appropriate number of times at any appropriate time.

In another embodiment, the obtaining the first immune cell population may include: filtering tumor tissue; cell collection; and cell sorting. Here, the acquiring of the first immune cell population may further include cell washing, and the cell washing may be performed any appropriate number of times at any appropriate time.

### Number of cells in obtained first immune cell population

Through obtaining the first immune cell population, a predetermined number of cells or more is acquired. In one embodiment, for each tumor sample cultured to produce the first immune cell population, approximately 1E4, approximately 2E4, approximately 3E4, approximately 4E4, approximately 5E4, approximately 6E4, approximately 7E4, approximately 8E4, approximately 9E4, approximately 1E5, approximately 2E5, approximately 3E5, approximately 4E5, approximately 5E5, approximately 6E5, approximately 7E5, approximately 8E5, approximately 9E5, approximately 1E6, approximately 2E6, approximately 3E6, approximately 4E6, or approximately 5E6 or more cells may be obtained. In a specific embodiment, for one tumor sample cultured to produce the first immune cell population, approximately 1E5, approximately 2E5, approximately 3E5, approximately 4E5, approximately 5E5, approximately 6E5, approximately 7E5, approximately 8E5, approximately 9E5, or approximately 1E6 cells may be acquired. In one example, when the tumor sample cultured to produce the first immune cell population is a tumor sample having a cross-sectional area of approximately 1 to 4 mm², approximately 8E5 or more cells for each tumor sample may be acquired. The number of cells obtained through the culture for producing the first immune cell population may vary according to the type of tumor and the size of the tumor sample and is not limited to the above-described embodiment.

### II. Cryopreservation

### Overview

In the method for producing an immune cell composition disclosed in the present application, in between of each process included in the method, cryopreservation of the result of each process may optionally be included.

In one example, after obtaining the first immune cell population, cryopreserving the first immune cell population or semi-product may optionally be included. In the cryopreservation of the first immune cell population or semi-product, the first immune cell population or semi-product is included in a cryopreservation composition and cryopreserved. The cryopreservation of the first immune cell population will be described in detail below.

### Medium for cryopreservation

As described above, the first immune cell population is included in the cryopreservation composition and cryopreserved. The cryopreservation composition has a composition suitable for cryopreservation. Here, a medium suitable for cryopreservation may be included. The medium suitable for cryopreservation includes all types of media for cryopreservation, known in the art.

In one embodiment, the cryopreservation composition may include DMSO at a concentration of approximately 0.1%, approximately 0.5%, approximately 1%, approximately 3%, approximately 5%, approximately 8%, approximately 10%, approximately 13%, or approximately 15%, or a concentration greater than aforesaid percentage values, but the present application is not limited thereto. In a specific embodiment, the cryopreservation composition may include DMSO at a concentration of 10%. For example, CryoStor^{®} CS10 including 10% DMSO may be used.

### Cell concentration and number of cryopreserved cells

The first immune cell population may be included in the cryopreservation composition at a specific concentration and cryopreserved.

In one embodiment, the first immune cell population may be included in the cryopreservation composition at a concentration of approximately 1E5 cells/mL, approximately 1E6 cells/mL, approximately 2E6 cells/mL, approximately 3E6 cells/mL, approximately 4E6 cells/mL, approximately 5E6 cells/mL, approximately 6E6 cells/mL, approximately 7E6 cells/mL, approximately 8E6 cells/mL, approximately 9E6 cells/mL, or approximately 1E7 cells/mL, but the present application is not limited. The first immune cell population may be included at a concentration suitable for cryopreservation in the cryopreservation composition. In a specific embodiment, the first immune cell population may be included at a concentration of 2E6 cells/mL in the cryopreservation composition.

In one embodiment, approximately 0.1 mL, approximately 0.2 mL, approximately 0.3 mL, approximately 0.4 mL, approximately 0.5 mL, approximately 0.7 mL, approximately 1 mL, approximately 2 mL, approximately 3 mL, approximately 4 mL, approximately 5 mL, approximately 7 mL, or approximately 10 mL, or an amount greater than or equal to aforesaid mL amounts of the cryopreservation composition may be placed in a cryovial and cryopreserved. In a specific embodiment, approximately 1 mL of the cryopreservation composition may be placed in the cryovial and cryopreserved.

For example, the first immune cell population may be suspended in the cryopreservation composition at a concentration of approximately 2E6 cells/mL, and approximately 1 mL of the cryopreservation composition may be placed in the cryovial and cryopreserved.

### Cryopreservation period

The first immune cell population may be cryopreserved for a predetermined period, after then may be used through expansion to produce an immune cell composition. The cryopreservation period is not limited. In one embodiment, the period of cryopreserving the first immune cell population may be at most approximately 1 year, approximately 2 years, approximately 3 years, approximately 4 years, approximately 5 years, approximately 6 years, approximately 7 years, approximately 8 years, approximately 9 years, approximately 10 years, approximately 11 years, approximately 12 years, approximately 13 years, approximately 14 years, approximately 15 years, approximately 16 years, approximately 17 years, approximately 18 years, approximately 19 years, or approximately 20 years, or a period longer than or equal to aforesaid periods. For example, the first immune cell population may be used to produce an immune cell composition after cryopreserved for 1 hour and thawed. In another example, after being cryopreserved for one day, the first immune cell population may be thawed and used to produce an immune cell composition. In still another example, after being cryopreserved for 10 years, the first immune cell population may be thawed and used to produce an immune cell composition.

### Cryopreservation temperature

The first immune cell population is cryopreserved at a temperature suitable for long-term preservation. In one embodiment, the first immune cell population may be cryopreserved at approximately -40 °C or less, -60 °C or less, -80 °C or less, approximately -100 °C or less, approximately -120 °C or less, approximately -140 °C or less, approximately -160 °C or less, approximately -180 °C or less, or approximately -190 °C or less. In a specific embodiment, the first immune cell population may be cryopreserved at approximately -190 °C or less. For example, the first immune cell population may be cryopreserved at approximately -190 °C or less in liquid nitrogen (LN₂) or gaseous liquid nitrogen (LN₂).

### III. Thawing cryopreserved first immune cell population

### Overview

In the method for producing an immune cell composition disclosed in the present application, between each process included in the method, cryopreserving the result of each process may optionally be included, and when such a cryopreservation process is performed, after the cryopreservation step, a process of thawing the cryopreserved result of each process may be further included.

In one example, the cryopreserved first immune cell population may be thawed and used to produce an immune cell composition.

### Thawing method

The thawing may be performed through methods for thawing cryopreserved cells, known in the art. In one embodiment, the cryopreserved first immune cell population may be thawed using a thermostat (water bath). In another embodiment, the cryopreserved first immune cell population may be thawed using a thawing device. Here, the thawing device may be a device for thawing cells by a dry-thawing method. The dry-thawing method may prevent the contamination of cells. Here, the dry-thawing method includes both a water-free method and a method in which water is present in a form of being blocked from a thawed subject (e.g., in the form of a pack containing water), but the present application is not limited thereto. For example, the cryopreserved first immune cell population may be thawed in a thermostat at approximately 37 °C for approximately 2 minutes. In another example, the cryopreserved first immune cell population may be thawed using a thawing device including a water-containing pack at approximately 37 °C for approximately 7 minutes.

As described above, the method for producing an immune (cell) composition disclosed in the present application includes producing a final product by using a semi-product including a first immune cell population immediately after producing the semi-product including the first immune cell population; or producing a final product by optionally cryopreserving the semi-product for a predetermined period and thawing it.

### IV. Production of final product

The method for producing an immune cell composition disclosed in the present application may include producing a semi-product and/or producing a final product. Here, the final product refers to an immune cell composition produced by acquiring a third immune cell population expanded from a second immune cell population, wherein the second immune cell population is derived from the first immune cell population, or a thawed first immune cell population after cryopreservation.

In one embodiment, the producing of an immune cell composition or the producing of a final product may include:
seeding a second immune cell population;
expanding the second immune cell population; and
producing an immune cell composition.

Hereinafter, each step included in the producing of a final product will be disclosed in detail.

### 1. Seeding second immune cell population

### Overview

A second immune cell population derived from a first immune cell population or a thawed first immune cell population after cryopreservation is suspended in a second composition, and seeded in a second container.

The seeding refers to dispensing cells into a container, and means dispensing the second immune cell population into the second container. Here, an appropriate number of cells for expansion may be dispersed according to a cell culture area or volume of the container. In one embodiment, the second immune cell population is suspended in the second composition, and seeded at an appropriate amount to the second container.

In one embodiment, the second immune cell population may be suspended in the second composition, and the cells may be seeded in the second container at a cell number of approximately 1E3 to 1E4, approximately 1E4 to 1E5, approximately 1E5 to 1E6, or approximately 1E6 to 1E7. In a specific embodiment, the second immune cell population may be suspended in the second composition, and the cells may be seeded in the second container at a cell number of approximately 1E5, 2E5, 3E5, 4E5, 5E5, 6E5, 7E5, 8E5, 9E5, 1E6, 1.1E6, 1.2E6, 1.3E6, 1.4E6, or 1.5E6, or a cell number within a range set by two values selected from aforesaid values. In a specific embodiment, the second immune cell population may be suspended in the second composition, and the cells may be seeded in the second container at a cell number of 4E5.

### Second immune cell population

In this step, the second immune cell population seeded in the second container is derived from the above-described first immune cell population or first immune cell population thawed after cryopreservation. That is, the second immune cell population may be obtained from the first immune cell population or the first immune cell population thawed after cryopreservation.

In one embodiment, a method of obtaining the second immune cell population from the first immune cell population or first immune cell population thawed after cryopreservation may include various known methods for acquiring cells in the process of culturing cells, but the present application is not limited thereto.

For example, the method of obtaining the second immune cell population from the first immune cell population or the thawed first immune cell population after cryopreservation may include any one or more of the following steps. The obtaining of the second immune cell population may further include confirming cell viability when needed, but the present application is not limited thereto.

### 1) Cell washing

The acquiring of a second immune cell population may include washing the cell population. In one embodiment, a second immune cell population may be acquired by washing the first immune cell population or the thawed first immune cell population. For example, for cell washing, centrifugation may be used. In one embodiment, the first immune cell population or thawed first immune cell population may be washed with a buffer such as DPBS or PBS, or washed with a medium.

### 2) Centrifugation

In one embodiment, to acquire a second immune cell population, a method of centrifuging the first immune cell population or the thawed first immune cell population may be used. In one embodiment, centrifugation may be performed on the washed immune cell population. An embodiment of the centrifugation method is the same as described in the related paragraphs.

### 3) Cell counting, division, or combining

In one embodiment, to acquire the second immune cell population, cell counting may be performed on the first immune cell population. For example, after counting the cell number of the first immune cell population that has undergone cell washing, the second immune cell population may be acquired by dividing an immune cell population to have an appropriate cell number for seeding to be described below. In addition, the second immune cell population may be acquired by combining immune cell populations cultured in different first containers.

In one embodiment, obtaining the second immune cell population may include cell washing.

In one embodiment, obtaining the second immune cell population may include cell washing and cell counting. Here, the immune cell population that has undergone cell counting may be divided to have an appropriate cell number for seeding.

### Second container

The second immune cell population may be seeded in a second container, wherein the second container refers to a container used to expand the second immune cell population. The second container is used to expand the second immune cell population, along with a second composition including a medium, serum, plasma, IL-2, anti-CD-3 antibody, feeder cells and/or an antibiotic. The second container is a gas-permeable container through which a gas passes, or a container including a gas supply system to provide a gas. That is, the container may be a container manufactured so that a gas can pass through the container, or a container that is not manufactured to allow a gas to pass through but includes a separate gas supply system to allow gas permeation.

In one embodiment, the second container may be a container having a maximum allowable capacity of approximately 1, 2, 3, 4, 5, 6, 7, 8, 9, 10, 12, 14, 16, 18, 20, 22, 24, 26, 28, 30, 35, 40, 45, 50, 55, 60, 70, 80, 90, 100, 120, 140, 160, 180, 200, 220, 240, 260, 280, 300, 350, 400, 450, 500, 550, 600, 700, 800, 900, 1000, 1100, 1200, 1300, 1400, 1600, 1800, 2000, 2500, 3000, 3500, 4000, 4500, 5000, 6000, 7000, 8000, 9000, or 10000 mL, or a container having a maximum allowable capacity within a range set by two values selected from aforesaid values. In a specific embodiment, the second container may be a container having a maximum allowable capacity of approximately 500, 550, 600, 700, 800, 900, 1000, 1100, 1200, 1300, 1400, 1600, 1800, or 2000 mL, or a container having a maximum allowable capacity within a range set by two values selected from aforesaid values.

In one embodiment, the second container may be, for example, a 24-well plate, a flask such as a T25, T75, or T125 flask, a GREX 10, GREX 10M, GREX 10M-CS GREX 100, GREX 100M, GREX 100M-CS, GREX 500, GREX 500M, or GREX 500M-CS flask, or a culture bag, but the present application is not limited thereto. In a specific embodiment, the second container may be GREX 100M.

### Second composition

The second immune cell population is suspended in the second composition and seeded in the second container, and here, the second composition includes any one or more selected from a medium, serum, plasma, IL-2, anti-CD3 antibody, feeder cells, and an antibiotic.

In one embodiment, the second composition may include a medium; serum; and IL-2. In another embodiment, the second composition may include a medium; plasma; and IL-2. In one embodiment, the second composition may further include antibodies (e.g., any one or more selected from anti-CD137 antibody, anti-PD-1 antibody, and anti-PD-L1 antibody) described in the table of contents for the first composition of the present application, in addition to the above-described materials, and the embodiments of the antibodies are the same as described in the related paragraphs.

In a specific embodiment, the second composition includes a medium; serum; IL-2; anti-CD3 antibody; and feeder cells. In another specific embodiment, the second composition includes a medium; plasma; IL-2; anti-CD3 antibody; and feeder cells. In still another specific embodiment; the second composition includes a medium; any one of serum and plasma; IL-2; anti-CD3 antibody; an antibiotic; and feeder cells.

Generally, in the culture and/or expansion of tissue or cells, the volume of the composition used in the container (e.g., flask) may vary according to the type of container. For example, generally, for GREX10, 40 mL of the composition is used, for GREX100, 400 mL of the composition is used, and for GREX100M, 1,000 mL of the composition is used.

However, to expand the second immune cell population of the present application, the amount of the composition initially added to the second container may be different from the generally used amount described above.

In one embodiment, the amount of the second composition initially added to the second container may be approximately 80%, 70%, 60%, 50%, 45%, 40%, 35%, 30%, 25%, 20%, 15%, 10%, 9%, 8%, 7%, 6%, or 5% or less of the maximum allowable capacity of the second container. In a specific embodiment, the amount of the second composition initially added to the second container may be approximately 30% or less of the maximum allowable capacity of the second container. In a specific embodiment, the amount of the second composition initially added to the second container may be approximately 10% or less of the maximum allowable capacity of the second container. Here, the maximum allowable capacity means the maximum amount that can be added to the second container.

In one embodiment, when the second container is GREX100M, the amount of the initially added second composition may be 200 mL, 150 mL, 100 mL, 80 mL, 60 mL, 40 mL, 20 mL, or 10 mL or less. In a specific embodiment, when the second container is GREX100M, the amount of the initially added second composition may be approximately 20 mL.

### 1) Medium

A medium used in the present application means a nutritional source for culturing cells and/or tissue. As the medium, all media suitable for culturing immune cells containing TILs may be used.

In one embodiment, the medium may be an AlyS505N medium, an AIM-V medium, or an RPMI medium, but the present application is not limited thereto. In a specific embodiment, a CTS-AIM-V medium may be used.

In one embodiment, the medium included in the second composition may be an AlyS505N medium, an AIM-V medium, or an RPMI medium. In a specific embodiment, the medium included in the second composition may be a CTS-AIM-V medium.

Serum, plasma, IL-2, anti-CD-3 antibody, feeder cells and/or an antibiotic may be added to the medium and used in the expansion of immune cells. That is, the second composition may further include serum, plasma, IL-2, anti-CD-3 antibody, feeder cells, and/or an antibiotic, in addition to the medium.

### 2) Additives

### a) Serum

In one embodiment, serum may be added to a basal culture medium (e.g., the above-described AlyS505N medium, AIM-V medium, or RPMI1640 medium). In one embodiment, the second composition of the present application may include serum. The serum may contain high molecular weight proteins and low molecular weight nutrients, and contains components required for cell growth. The serum may include human serum, but the present application is not limited thereto.

In one embodiment, the human serum may be used while being added to the medium at a concentration of approximately 10 vol% or less. In another embodiment, the human serum may be used while being added to the medium at a concentration of approximately 9 vol% or less, approximately 8 vol% or less, approximately 7 vol% or less, approximately 6 vol% or less, approximately 5 vol% or less, approximately 4 vol% or less, or 3 vol% or less. In a specific embodiment, the human serum may be used while being added to a CTS-AIM-V medium at approximately 3 vol%.

In one embodiment, when the serum is included in the second composition, the concentration of the serum included in the second composition may be approximately 20, 19, 18, 17, 16, 15, 14, 13, 12, 11, 10, 9, 8, 7, 6, 5, 4, 3, 2, or 1 vol%, or a concentration less than or equal to aforesaid vol%. In a specific embodiment, the concentration of the serum included in the second composition may be approximately 10, 9, 8, 7, 6, 5, 4, 3, 2, or 1 vol%, or a concentration less than or equal to aforesaid vol%. In a specific embodiment, the concentration of the serum included in the second composition may be approximately 5, 4, 3, 2, or 1 vol%, or a concentration less than or equal to aforesaid vol%.

In one embodiment, the concentration of human serum included in the second composition may be approximately 5, 4, 3, 2, or 1 vol%. In a specific embodiment, the concentration of human serum included in the second composition may be approximately 3 vol%.

### b) Plasma

In one embodiment, plasma may be added to a basal medium. In one embodiment, the second composition of the present application may include plasma. The plasma refers to fibrinogen-free serum. The plasma contains components required for cell growth.

The plasma used in the present application may be human plasma, or plasma isolated from a patient's blood, but the present application is not limited thereto.

In one embodiment, plasma, instead of serum, may be added to the medium and used.

In one embodiment, plasma may be used while being added to the medium at approximately 10 vol% concentration or less. In another embodiment, plasma may be used while being added to the medium at a concentration of approximately 9 vol% or less, approximately 8 vol% or less, approximately 7 vol% or less, approximately 6 vol% or less, approximately 5 vol% or less, approximately 4 vol% or less, or 3 vol% or less. In a specific embodiment, plasma may be added to the CTS-AIM-V medium at approximately 3 vol% and used.

In one embodiment, when the plasma is included in the second composition, the concentration of the plasma included in the second composition may be approximately 20, 19, 18, 17, 16, 15, 14, 13, 12, 11, 10, 9, 8, 7, 6, 5, 4, 3, 2, or 1 vol%, or a concentration less than or equal to aforesaid vol%. In a specific embodiment, the concentration of the plasma included in the second composition may be approximately 10, 9, 8, 7, 6, 5, 4, 3, 2, or 1 vol%, or a concentration less than or equal to aforesaid vol%. In a specific embodiment, the concentration of the plasma included in the second composition may be approximately 5, 4, 3, 2, or 1 vol%, or a concentration less than or equal to aforesaid vol%.

In one embodiment, the concentration of plasma included in the second composition may be approximately 5, 4, 3, 2, or 1 vol%. In a specific embodiment, the concentration of plasma included in the second composition may be approximately 3 vol%.

### c) IL-2

The IL-2 used in the present application is an abbreviation of interleukin-2, referring to a factor promoting T-cell proliferation. IL-2 includes various types of IL-2 including human recombinant IL-2 type.

In one embodiment, the second composition may include IL-2.

In one embodiment, IL-2 may be added to the medium at a concentration of approximately 10,000 IU/mL. In one embodiment, the concentration of IL-2 added to the medium may be approximately 9000 IU/mL, approximately 8,000 IU/mL, approximately 7,000 IU/mL, approximately 6,000 IU/mL, approximately 5,000 IU/mL, approximately 4,000 IU/mL, approximately 3,000 IU/mL, approximately 2,000 IU/mL, approximately 1,000 IU/mL, or approximately 500 IU/mL. In a specific embodiment, IL-2 may be added to a CTS-AIM-V medium at a concentration of approximately 2,000 IU/mL.

In one embodiment, the second composition may include IL-2 at a concentration of approximately 10,000 IU/mL, approximately 9,500 IU/mL, approximately 9,000 IU/mL, approximately 8,500 IU/mL, approximately 8,000 IU/mL, approximately 7,500 IU/mL, approximately 7,000 IU/mL, approximately 6,500 IU/mL, approximately 6,000 IU/mL, approximately 5,500 IU/mL, approximately 5,000 IU/mL, approximately 4,500 IU/mL, approximately 4,000 IU/mL, approximately 3,500 IU/mL, approximately 3,000 IU/mL, approximately 2,500 IU/mL, approximately 2,000 IU/mL, approximately 1,500 IU/mL, approximately 1,000 IU/mL, approximately 900 IU/mL, approximately 800 IU/mL, approximately 700 IU/mL, approximately 600 IU/mL, approximately 500 IU/mL, approximately 400 IU/mL, approximately 300 IU/mL, approximately 200 IU/mL, or approximately 100 IU/mL, or a concentration within a range set by two values selected from aforesaid values. In a specific embodiment, the second composition may include IL-2 at a concentration of approximately 3,000 IU/mL, approximately 2,500 IU/mL, approximately 2,000 IU/mL, approximately 1,500 IU/mL, or approximately 1,000 IU/mL, or a concentration within a range set by two values selected from aforesaid values. In a specific embodiment, the second composition may include IL-2 at a concentration of approximately 2,000 IU/mL.

### d) Anti-CD3 antibody

An anti-CD3 antibody refers to an antibody targeting a membrane protein on the T cell surface, that is, a CD3 receptor.

In one embodiment, the second composition may include an anti-CD3 antibody. In one embodiment, the anti-CD3 antibody may be any one selected from OKT3, Otelixizumab, teplizumab, and visilizumab, or a combination of two or more thereof. In a specific embodiment, the anti-CD3 antibody included in the second composition may be OKT3.

In one embodiment, the anti-CD-3 antibody may be used while being added to the medium at a concentration of approximately 1 ng/mL, approximately 2 ng/mL, approximately 3 ng/mL, approximately 4 ng/mL, approximately 5 ng/mL, approximately 10 ng/mL, approximately 15 ng/mL, approximately 20 ng/mL, approximately 25 ng/mL, approximately 30 ng/mL, approximately 35 ng/mL, approximately 40 ng/mL, approximately 45 ng/mL, approximately 50 ng/mL, approximately 60 ng/mL, approximately 70 ng/mL, approximately 80 ng/mL, approximately 90 ng/mL, approximately 100 ng/mL, approximately 200 ng/mL, approximately 300 ng/mL, approximately 400 ng/mL, approximately 500 ng/mL, or approximately 1,000 ng/mL, or a concentration within a range set by two values selected from aforesaid values.

**In** one embodiment, when the anti-CD3 antibody is included in the second composition, the concentration of the anti-CD3 antibody included in the second composition may be approximately 1 ng/mL, approximately 2 ng/mL, approximately 3 ng/mL, approximately 4 ng/mL, approximately 5 ng/mL, approximately 10 ng/mL, approximately 15 ng/mL, approximately 20 ng/mL, approximately 25 ng/mL, approximately 30 ng/mL, approximately 35 ng/mL, approximately 40 ng/mL, approximately 45 ng/mL, approximately 50 ng/mL, approximately 60 ng/mL, approximately 70 ng/mL, approximately 80 ng/mL, approximately 90 ng/mL, approximately 100 ng/mL, approximately 200 ng/mL, approximately 300 ng/mL, approximately 400 ng/mL, approximately 500 ng/mL, or approximately 1000 ng/mL, or a concentration within a range set by two values selected from aforesaid values. **In** a specific embodiment, the concentration of the anti-CD3 antibody included in the second composition may be approximately 30 ng/mL.

### e) Feeder cells

### i) Feeder cell percentage

Feeder cells used in the present application are cells that are included in the second composition and serve to activate and expand TILs when the second immune cell population is expanded. **In** one embodiment, the feeder cells include peripheral blood mononuclear cells (PBMCs), and here, the PBMCs refer to peripheral blood cells with round nuclei.

**In** one embodiment, the feeder cells may be peripheral blood mononuclear cells (PBMCs). **In** one embodiment, the feeder cells may be inactivated PBMCs. **In** one embodiment, the feeder cells may be allogeneic PBMCs. **In** one embodiment, the feeder cells may be inactivated allogeneic PBMCs. Here, the PBMCs may be inactivated by irradiation, and a method of inactivating PBMCs is not limited thereto. For example, the PBMCs may be inactivated through irradiation of approximately 60Gy.

The feeder cells may be added to the second composition in a predetermined ratio with respect to the second immune cell population (TIL). **In** one embodiment, after the second immune cell population is suspended in the second composition, the feeder cells may be included in the second composition to have the following ratio with cells in the second immune cell population (cells included in the second immune cell population : feeder cells): approximately 1:1000, approximately 1:900, approximately 1:800, approximately 1:700, approximately 1:600, approximately 1:500, approximately 1:450, approximately 1:400, approximately 1:350, approximately 1:300, approximately 1:280, approximately 1:260, approximately 1:240, approximately 1:220, approximately 1:200, approximately 1:180, approximately 1:160, approximately 1:140, approximately 1:120, approximately 1:100, approximately 1:90, approximately 1:80, approximately 1:70, approximately 1:60, or approximately 1:50.

**In** a specific embodiment, the feeder cells may be included in the second composition such that a ratio with the immune cell population (cells included in the second immune cell population : feeder cells) is 1:200 (±100).

**In** a specific embodiment, the feeder cells may be included in the second composition such that the ratio with the immune cell population (cells included in the second immune cell population : feeder cells) is approximately 1:200.

### ii) Isolation of PBMCs

**In** one embodiment, the feeder cells may be PBMCs. Here, the PBMCs may be isolated from the blood of a healthy person. When the feeder cells are inactivated allogeneic PBMCs, the inactivated allogeneic PBMCs are prepared by inactivating (e.g., inactivation by irradiation) peripheral blood cells isolated from the blood of a healthy patient.

The isolation of PBMCs from a healthy person may be performed through a known method. For example, the method of isolating PBMCs may include a general isolation method using a Ficoll solution.

### f) Antibiotic

**In** one embodiment, the second composition may further include an antibiotic.

The antibiotic may be used to prevent contamination by bacteria or fungi during cell culture and/or expansion.

Whether or not to use an antibiotic in the production of a final product may be determined by appropriately considering the condition of the used first immune cell population or second immune cell population, and the condition of the cultured and/or expanded cells.

When the second composition includes an antibiotic, the antibiotic may be included in a concentration that does not affect cell culture and/or expansion, and here, the concentration of the antibiotic exhibiting cell toxicity may be considered. The concentration of the antibiotic included in the second composition may vary according to the type of antibiotic.

Examples of the antibiotics included in the second composition include ampicillin, gentamycin, kanamycin, neomycin, penicillin, and streptomycin, but the present application is not limited thereto. That is, as the antibiotic added to the medium for cell culture and/or expansion, any antibiotic known in the art may be used. In a specific embodiment, the second composition may include gentamycin.

As described above, the second composition in the present application includes a culture medium and additives. In one embodiment, the second composition may include a medium, serum, IL-2, anti-CD3 antibody, and feeder cells. In another embodiment, the second composition may include a medium, plasma, IL-2, anti-CD3 antibody, and feeder cells. In still another embodiment, the second composition may include a medium, serum, IL-2, anti-CD3 antibody, feeder cells, and an antibiotic. In yet another embodiment, the second composition may include a medium, plasma, IL-2, anti-CD3 antibody, feeder cells, and an antibiotic. However, the present application is not limited thereto.

In a specific embodiment, the second composition includes a medium, serum, IL-2, OKT3, and feeder cells, wherein the medium is a CTS-AIM-V medium, the serum is human AB serum and the human AB serum is included in the second composition at a concentration of approximately 3 vol%, IL-2 is included in the second composition at a concentration of approximately 2,000 IU/mL, OKT3 is included in the second composition at a concentration of approximately 30 ng/mL, and the feeder cells are inactivated allogeneic PBMCs. Here, the inactivated allogeneic PBMCs may be included in the second composition at a ratio (cells included in the second immune cell population : feeder cells) of 1:200 with respect to cells included in the immune cell population.

In a specific embodiment, the second composition includes a medium, serum, IL-2, OKT3, an antibiotic, and feeder cells, wherein the medium is a CTS-AIM-V medium, the serum is human AB serum and the human AB serum is included in the second composition at a concentration of approximately 3 vol%, IL-2 is included in the second composition at a concentration of approximately 2,000 IU/mL, OKT3 is included in the second composition at a concentration of 30 ng/mL, and the feeder cells are inactivated allogeneic PBMCs. Here, the inactivated allogeneic PBMCs may be included in the second composition at a ratio (cells included in the second immune cell population : feeder cells) of 1:200 with respect to cells included in the immune cell population.

A specific example of the seeding the second immune cell population will be described below, but the present application is not limited thereto:
From the first immune cell population or thawed first immune cell population after cryopreservation, a second immune cell population is acquired. The second immune cell population is suspended in the second composition and seeded in a second container GREX 100M, and here, the cells are seeded at a cell number of approximately 4E5 per container. Here, the second composition includes a CTS-AIM-V medium, 3 vol% of human serum, 2,000 IU/mL of IL-2, 30 ng/mL of OKT3, and inactivated allogeneic PBMCs. Here, based on the time point after the second immune cell population is suspended in the second composition, the inactivated allogeneic PBMCs are included in the second composition at a ratio (cells included in the second immune cell population : inactivated allogeneic PBMCs) of approximately 1:200 with respect to the cells included in the second immune cell population. That is, the second immune cell population is suspended in the second composition, and the second composition including the second immune cell population is added at 20 mL per container.

### 2. Expanding second immune cell population

### Overview

The method for producing an immune cell composition of the present application includes expanding the second immune cell population. In one embodiment, the method for producing an immune cell composition of the present application includes expanding the second immune cell population to produce a third immune cell population.

The seeded second immune cell population is expanded in the second composition in the second container during an expansion period, and through this, a third immune cell population is produced. The third immune cell population refers to an immune cell population obtained by expanding the second immune cell population.

The expanding the second immune cell population may include adding a second additive composition.

Hereinafter, the expanding of the second immune cell population will be described in detail.

### Adding second additive composition into second container

During the expansion of the second immune cell population, a second additive composition may be added to the second container. Here, the second additive composition may include any one or more selected from a medium, serum, plasma, an antibiotic, IL-2, anti-CD3 antibody, and feeder cells. In a specific embodiment, the second additive composition may include a medium, serum, and IL-2.

The process of addition of the second additive composition will be described in detail below.

### 1) Number of additions

While the second immune cell population is cultured in the second composition in the second container, the second additive composition is added (supplemented) into the second container.

In one embodiment, during expansion, the second additive composition may be added to the second container 1, 2, 3, 4, 5, 6, 7, 8, 9, or 10 times, or a number of times greater than aforesaid number of times. In a specific embodiment, during the expansion period, the second additive composition may be added 4 times.

During expansion, when the second additive composition is added twice or more, the second additive composition may be added in the same amount at every addition time, or may be added in a different amount at every addition time. The amount of the added second additive composition will be described in detail in the related paragraphs.

### 2) Addition time

Here, in one embodiment, the second additive composition may be added on any one or more days selected from day 2, day 3, day 4, day 5, day 6, day 7, day 8, day 9, day 10, day 11, day 12, day 13, day 14, day 15, day 16, day 17, day 18, day 19, day 20, and day 21 from the start date of expansion, and may be added 1, 2, 3, 4, 5, 6, 7, 8, 9, or 10 times during the expansion period. In a specific embodiment, the second additive composition is added 4 times on days 4, 6, 8, and 10.

### 3) Addition amount

The amount of the added second additive composition may vary according to the type of container and/or an addition time. In one embodiment, the second additive composition may be added in the same amount at each addition time. In another embodiment, the second additive composition may be added in a different amount at each addition time. In still another embodiment, the second additive composition may be added by increasing its addition amount at each addition time.

In one embodiment, the second additive composition may be added in the same or different amount, such as approximately 1 mL, 2 mL, 3 mL, 4 mL, 5 mL, 6 mL, 7 mL, 8 mL, 9mL, 10 mL, 11 mL, 12 mL, 13 mL, 14 mL, 15 mL, 16 mL, 17 mL, 18 mL, 19 mL 20 mL, 21 mL, 22 mL, 23 mL, 24 mL, 25 mL, 26 mL, 27 mL, 28 mL, 29 mL, 30 mL, 32 mL, 35 mL, 37 mL, 40 mL, 45 mL, 50 mL, 55 mL, 60 mL, 65 mL, 70 mL, 80 mL, 90 mL, 100 mL, 110 mL, 120 mL, 130 mL, 140 mL, 150 mL, 160 mL, 170 mL, 180 mL, 190 mL, 200 mL, 250 mL, 300 mL, 350 mL, 400 mL, 450 mL, 500 mL, 550 mL, 600mL, 650 mL, 700 mL, 750 mL, 800 mL, 850 mL, 900 mL, 950 mL, 1000 mL, 1500 mL, 2 L, 3 L, 4 L, or 5 L, or an amount greater than or equal to aforesaid values at each addition time. However, the present application is not limited thereto, and the amount of the added second additive composition may be determined as an appropriate value in consideration of various conditions (e.g., the capacity of the container, the condition of the cultured cells, and/or the addition time point). In a specific embodiment, the second additive composition may be added by increasing an addition amount at each addition time, and 20 mL, 60 mL, 180 mL, and 540 mL of the second additive composition may be added to the second container on days 4, 6, 8, and 10, respectively.

### 4) Configuration of second additive composition

The second additive composition added in the second container may include any one or more selected from a medium, serum, plasma, IL-2, anti-CD3 antibody, and feeder cells.

In one embodiment, the second additive composition may include a medium; serum; and IL-2. In another embodiment, the second additive composition may include a medium; plasma; and IL-2.

In a specific embodiment, the second additive composition may include a medium; serum; and IL-2. In another specific embodiment, the second additive composition may further include any one or more of anti-CD3 antibody and feeder cells, in addition to a medium, serum, and IL-2.

Embodiments for each component that can be included in the second additive composition are as described above in the second composition.

### a) Medium

Embodiments related to the medium that can be included in the second additive composition are as described above in the second composition. For example, in a specific embodiment, the medium included in the second additive composition may be a CTS-AIM-V medium, but the present application is not limited thereto.

### b) Serum

When serum is included in the second additive composition, embodiments related to the serum are the same as described above in the second composition. For example, in a specific embodiment, the serum may be human serum, but the present application is not limited thereto. For example, in a specific embodiment, the concentration of the serum included in the second additive composition may be approximately 3 vol%, but the present application is not limited thereto.

### c) Plasma

When plasma is included in the second additive composition, embodiments related to the plasma are the same as described above in the second composition. For example, in a specific embodiment, the plasma may be human plasma, but the present application is not limited thereto. For example, in a specific embodiment, the concentration of the plasma included in the second additive composition may be approximately 3 vol%, but the present application is not limited thereto.

### d) IL-2

When IL-2 is included in the second additive composition, embodiments related to the IL-2 are the same as described above in the second composition. For example, in a specific embodiment, the concentration of IL-2 included in the second additive composition may be approximately 2,000 IU/mL, but the present application is not limited thereto.

### e) Anti-CD3 antibody

When an anti-CD3 antibody is included in the second additive composition, embodiments related to the anti-CD3 antibody are the same as described above in the second composition. For example, in a specific embodiment, the anti-CD3 antibody may be OKT3, but the present application is not limited thereto. For example, in a specific embodiment, the concentration of the anti-CD3 antibody included in the second additive composition may be approximately 30 ng/mL, but the present application is not limited thereto.

### f) Feeder cells

When feeder cells are included in the second additive composition, embodiments related to the feeder cells are the same as described above in the second composition. For example, in a specific embodiment, the feeder cells may be inactivated allogeneic PBMCs, but the present application is not limited thereto. For example, in a specific embodiment, the feeder cells may be included in the second additive composition at a ratio (immune cells or cells included in the second immune cell population : feeder cells) of 1:200, with respect to the immune cells included in the second container or the cells included in the second immune cell population which have been initially seeded in the second container.

### g) Antibiotic

When an antibiotic is included in the second additive composition, embodiments related to the antibiotic are the same as described above in the second composition.

In one embodiment, the configuration of the second additive composition added in the second container may vary at each addition time. In another embodiment, the configuration of the second additive composition added in the second container may be the same at each addition time.

In one embodiment, the configuration of the second additive composition added in the second container may be the same as that of the second composition. In another embodiment, the configuration of the second additive composition added in the second container may be different from that of the second composition. In a specific embodiment, the second additive composition may be a composition that has the same compositional components and compositional ratio as the second composition except for the anti-CD3 antibody and feeder cells.

In one embodiment, the second additive composition added in the second container may include a medium, serum, and IL-2. In a specific embodiment, the second additive composition added in the second container may include a CTS-AIM-V medium, approximately 3 vol% of human serum, and approximately 2,000 IU/mL of IL-2.

### Whether or not to add new immune cells during expansion

While the second immune cell population is expanded in the second container, immune cells may not be further added to the second immune cell population from the outside.

In addition, in such an expansion process, a container change may be optionally performed, but during the expansion of the second immune cell population, since there is no need to transfer the cell population from the second container to another container, it is advantageous for providing convenience in the process.

This means that, in the method of the present application, during the expansion of the second immune cell population, there is no need to transfer to a container, other than the second container, or no need to further add new immune cells to the second container from the outside.

### Expansion period

The second immune cell population may be expanded in the second container for a predetermined period. In one embodiment, the second immune cell population is expanded for approximately 1 day or more. In one embodiment, the second immune cell population may be expanded for approximately 2 days or more, 3 days or more, 4 days or more, 5 days or more, 6 days or more, 7 days or more, 8 days or more, 9 days or more, 10 days or more, 11 days or more, 12 days or more, 13 days or more, 14 days or more, 15 days or more, 16 days or more, 17 days or more, 18 days or more, 19 days or more, 20 days or more, 21 days or more, 22 days or more, 23 days or more, or 24 days or more. In a specific embodiment, the second immune cell population may be expanded in the second container for approximately 7, 8, 9, 10, 11, 12, 13, 14, 15, 16, 17, 18, 19, 20, or 21 days. In a specific embodiment, the second immune cell population may be expanded in the second container for approximately 14 days.

### Expansion efficiency

The second immune cell population seeded in the second container may be expanded a predetermined fold or more through the expansion of the second immune cell population. That is, the seeded second immune cell population is expanded a predetermined fold or more in the second container.

In one embodiment, the seeded second immune cell population is expanded in the second container, and thus a third immune cell population may be produced. Compared to the second immune cell population, the third immune cell population has a cell number that is greater than or equal to a certain fold. In one embodiment, the third immune cell population has at least approximately 100 times more cells than the second immune cell population. In one embodiment, the third immune cell population may have at least approximately 200 times, approximately 300 times, approximately 400 times, approximately 500 times, approximately 600 times, approximately 700 times, approximately 800 times, approximately 900 times, approximately 1,000 times, approximately 1,100 times, approximately 1,200 times, approximately 1,300 times, approximately 1,400 times, approximately 1,500 times, approximately 1,600 times, approximately 1,700 times, approximately 1,800 times, approximately 1,900 times, approximately 2,000 times, approximately 2,200 times, approximately 2,300 times, approximately 2,400 times, approximately 2,500 times, approximately 2,600 times, approximately 2,700 times, approximately 2,800 times, approximately 3,000 times, approximately 3,500 times, approximately 4,000 times, approximately 4,500 times, approximately 5,000 times, approximately 5,500 times, approximately 6,000 times, approximately 6,500 times, approximately 7,000 times, approximately 7,500 times, approximately 8,000 times, approximately 8,500 times, approximately 9,000 times, approximately 9,500 times, approximately 10,000 times, or approximately 20,000 times more cells than the second immune cell population. In a specific embodiment, the third immune cell population may have at least approximately 2,500 times more cells than the second immune cell population. In a specific embodiment, the third immune cell population may have at least approximately 9,400 times more cells than the second immune cell population. In a specific embodiment, the third immune cell population may have approximately 2,500 to 9,400 times more cells than the second immune cell population.

A specific example of expanding the second immune cell population may be described as follows, but the present application is not limited thereto:
- The second immune cell population seeded after being suspended in the second composition is expanded for an expansion period of 14 days.
- The second additive composition is added to the second container on days 4, 6, 8, and 10 from the start date of expansion. Here, the added second additive composition includes a CTS-AIM-V medium, 3 vol% of human serum, and 2,000 IU/mL of IL-2. More specifically, the second additive composition may be added to the second container at 20 mL on day 4, at 60 mL on day 6, at 180 mL on day 8, and at 540 mL on day 10 from the start date of expansion.
- A third immune cell population is produced from the second immune cell population through expansion process for 14 days.

### 3. Producing immune cell composition (immune composition)

### Overview

A method for producing an immune cell composition or an immune composition of the present application may include preparing an immune (cell) composition including immune cells, as an active ingredient, acquired through the above-described process.

After completing the expansion of the second immune cell population, an immune (cell) composition is prepared by obtaining a third immune cell population from the expanded product.

In one embodiment, the immune (cell) composition may include the third immune cell population as an active ingredient. In another embodiment, the immune (cell) composition may include a fourth immune cell population derived from the third immune cell population as an active ingredient.

The immune cell composition or the immune composition refers to a composition including an "immune cell population" as an active ingredient. Here, the immune cell population may include TILs, and the immune cell composition (immune composition) of the present application may include TILs as an active ingredient.

In one embodiment, the producing of an immune cell composition may include obtaining a third immune cell population from the expanded product.

In one embodiment, the producing of an immune cell composition may further include obtaining a fourth immune cell population from the third immune cell population, in addition to obtaining the third immune cell population from the expanded product.

The producing of an immune cell composition will be described in detail below.

### Acquiring third immune cell population

A third immune cell population is obtained from the expanded product. A method of obtaining a third immune cell population may include various known methods for obtaining cells in the process of culturing or expanding cells, but the present application is not limited thereto. For example, the obtaining a third immune cell population may further include processes conventionally performed in the art, including cell counting and/or confirmation of cell viability, in addition to the processes to be described below, but the present application is not limited thereto.

In one embodiment, the obtaining of an immune cell population may include the following processes: collecting the immune cell population produced by the expansion process from the second container (cell collection). That is, a third immune cell population may be obtained by collecting from the expanded product the immune cell population.

In one embodiment, the obtaining a third immune cell population may further include the following process: washing the collected immune cell population (cell washing).

In one embodiment, the obtaining of a third immune cell population includes collecting the immune cell population produced through the expansion from the second container. Here, as a method of collecting the immune cell population, a method known in the art may be used, but the present application is not limited thereto. In one embodiment, to collect the immune cell population from the second container, centrifugation may be used. The centrifugation may be performed under the conditions of, for example, 200 g, 7 minutes, RT, and Acc9/Dcc9, but the present application is not limited thereto.

In one embodiment, the acquiring of a third immune cell population may further include washing the collected immune cell population, in addition to collecting the immune cell population produced through the expansion from the second container. In the washing of the cells, a method known in the art maybe used. For example, to wash the cells, centrifugation may be used, but the present application is not limited. Here, the washing of the immune cell population may be performed with a buffer. Here, as a buffer, for example, PBS or DPBS may be used, but the present application is not limited thereto. In one embodiment, the collected immune cell population may be washed with DPBS 1, 2, 3,4, or 5 times or more. In a specific embodiment, the immune cell population may be washed with DPBS three times.

### Third immune cell population

In one embodiment, the third immune cell population may have at least approximately 100 times, approximately 200 times, approximately 300 times, approximately 400 times, approximately 500 times, approximately 600 times, approximately 700 times, approximately 800 times, approximately 900 times, approximately 1,000 times, approximately 1,100 times, approximately 1,200 times, approximately 1,300 approximately 1,400 times, approximately 1,500 times, approximately 1,600 times, approximately 1,700 times, approximately 1,800 times, approximately 1,900 times, approximately 2,000 times, approximately 2,200 times, approximately 2,300 times, approximately 2,400 times, approximately 2,500 times, approximately 2,600 times, approximately 2,700 times, approximately 2,800 times, approximately 3,000 times, approximately 3,500 times, approximately 4,000 times, approximately 4,500 times, approximately 5,000 times, approximately 5,500 times, approximately 6,000 times, approximately 6,500 times, approximately 7,000 times, approximately 7,500 times, approximately 8,000 times, approximately 8,500 times, approximately 9,000 times, approximately 9,500 times, approximately 10,000 times, or approximately 20,000 times more cells than the second immune cell population.

**In** a specific embodiment, the third immune cell population may have at least approximately 2,500 times more cells than the second immune cell population. **In a** specific embodiment, the third immune cell population may have at least approximately 9,400 times more cells than the second immune cell population. **In a** specific embodiment, the third immune cell population may have approximately 2,500 to 9,400 times cells than the second immune cell population.

**In** one embodiment, the third immune cell population includes immune cells. The third immune cell population is obtained by the culture and/or expansion of TILs present near tumor tissue, thereby the immune cells may be TILs. Here, in the cells included in the third immune cell population, the percentage of immune cells may be approximately 70%, 75%, 80%, 85%, 86%, 87%, 88%, 89%, 90%, 91%, 92%, 93%, 94%, 95%, 96%, 97%, 98%, 99%, 99.5%, or 99.9% or more. **In** a specific embodiment, in the cells included in the third immune cell population, the percentage of TILs may be approximately 97%, 98%, 99%, 99.5%, or 99.9% or more. Here, the cells included in the third immune cell population may refer to surviving cells.

In one embodiment, the third immune cell population includes T cells. Here, the third immune cell population includes TILs, and the TILs include T cells. That is, the T cells included in the third immune cell population may be a type of TIL. In one embodiment, in the cells included in the third immune cell population, the percentage of T cells may be approximately 60%, 65%, 70%, 75%, 80%, 85%, 86%, 87%, 88%, 89%, 90%, 91%, 92%, 93%, 94%, 95%, 96%, 97%, 98%, or 99% or more. In a specific embodiment, the percentage of T cells in the cells included in the third immune cell population may be approximately 88%, 89%, 90%, 91%, 92%, 93%, 94%, 95%, 96%, 97%, 98%, or 99% or more. In a specific embodiment, the percentage of T cells in the cells included in the third immune cell population may be approximately 99% or more. In a specific embodiment, the percentage of T cells in the cells included in the third immune cell population may be approximately 99.5%, 99.6%, 99.7%, 99.8%, or 99.9% or more. Here, the cells included in the third immune cell population may refer to surviving cells.

In one embodiment, in the cells included in the third immune cell population, the percentage of natural killer cells (NK cells) may be 15, 14, 13, 12, 11, 10, 9, 8, 7, 6, 5, 4, 3, 2, or 1% or less. In a specific embodiment, in the cells included in the third immune cell population, the percentage of NK cells may be 10% or less. In a specific embodiment, the percentage of NK cells may be 1% or less. In a specific embodiment, the percentage of NK cells may be 0.2% or less. Here, the cells included in the third immune cell population may refer to surviving cells.

In one embodiment, in the cells included in the third immune cell population, the percentage of B cells may be 5, 4, 3, 2, or 1% or less. In a specific embodiment, the percentage of B cells may be 1% or less. In a specific embodiment, the percentage of B cells may be 0.2% or less. Here, the cells included in the third immune cell population may refer to surviving cells.

### Process that can be optionally included: Acquiring fourth immune cell population from third immune cell population

In one embodiment, the producing of an immune cell composition may further include obtaining a fourth immune cell population from the third immune cell population, in addition to obtaining the third immune cell population.

Here, the fourth immune cell population may be obtained from the third immune cell population through cell washing, treatment, isolation, and/or cell sorting, and may be obtained through a method known in the art without limitation. For example, the fourth immune cell population may be a processed third immune cell population which is processed to be suitable for administration to a subject.

### Cell sorting

The obtaining a fourth immune cell population from the third immune cell population may include sorting cells. Cell sorting refers to a method used to select only desired cells from a composition including a cell population. For cell sorting, a method conventionally used in the art may be used. For example, a method such as fluorescence-activated cell sorting (FACS), centrifugation, or isolation according to cell adhesion to a container may be used for cell sorting.

### Immune cell population processed into state suitable for administration to subject

The obtained third immune cell population or the third immune cell population that has undergone the cell washing may be processed into a state suitable for administration to a subject (e.g., a patient).

In one example, the obtained third immune cell population or the third immune cell population that has undergone the washing; or the immune cell population acquired by sorting the same may be treated with a pharmaceutically acceptable material. For example, the immune cell population may be suspended in a pharmaceutically acceptable material such as a diluent and/or an excipient and may be processed into a state suitable for administration to a patient. For example, the pharmaceutically acceptable material may isotonic saline, Ringer's solution, phosphate buffer, or a non-toxic pharmaceutically acceptable material known in the art.

### Immune cell composition or immune composition

The immune (cell) composition prepared by the method of the present application includes the third immune cell population or the fourth immune cell population as an active ingredient.

In one embodiment, the immune cell composition may be a composition including the third immune cell population or the fourth immune cell population, and processed into a state suitable for administration to a subject. That is, in one embodiment, the immune cell composition may be a composition in a state suitable for administration to a subject.

Hereinafter, the characteristics of the immune cell composition of the present application will be described in detail.

### Characteristics of immune cell composition

As described above, the immune cell composition may include the third immune cell population or the fourth immune cell population obtained from the third immune cell population.

**In** one embodiment, the immune cell composition includes immune cells. Here, the immune cells may be TILs. Here, in the cells included in the immune cell composition, the percentage of immune cells may be approximately 60, 65, 70%, 75%, 80%, 85%, 86%, 87%, 88%, 89%, 90%, 91%, 92%, 93%, 94%, 95%, 96%, 97%, 98%, 99%, 99.5%, or 99.9% or more. **In** a specific embodiment, in the cells included in the immune cell composition, the percentage of TILs may be approximately 97%, 98%, 99%, 99.5%, or 99.9% or more. Here, the cells included in the immune cell composition may refer to surviving cells.

**In** one embodiment, the immune cell composition includes T cells. Here, the immune cell composition includes TILs, and the TILs include T cells. That is, the T cells included in the immune cell composition may be a type of TILs. **In** one embodiment, in the cells included in the immune cell composition, the percentage of T cells may be approximately 60%, 65%, 70%, 75%, 80%, 85%, 86%, 87%, 88%, 89%, 90%, 91%, 92%, 93%, 94%, 95%, 96%, 97%, 98%, or 99% or more. **In a** specific embodiment, in the cells included in the immune cell composition, the percentage of T cells may be approximately 88%, 89%, 90%, 91%, 92%, 93%, 94%, 95%, 96%, 97%, 98%, or 99% or more. In a specific embodiment, in the cells included in the immune cell composition, the percentage of T cells may be approximately 99% or more. In a specific embodiment, in the cell included in the immune cell composition, the percentage of T cells may be approximately 99.5%, 99.6%, 99.7%, 99.8%, or 99.9% or more. Here, the cells included in the immune cell composition may refer to surviving cells.

In one embodiment, in the cells included in the immune cell composition, the percentage of NK cells may be 15, 14, 13, 12, 11, 10, 9, 8, 7, 6, 5, 4, 3, 2, or 1% or less. In a specific embodiment, in the cells included in the immune cell composition, the percentage of NK cells may be 10% or less. In a specific embodiment, the percentage of NK cells may be 1% or less. In a specific embodiment, the percentage of NK cells may be 0.2% or less. Here, the cells included in the immune cell composition may refer to surviving cells.

In one embodiment, in the cells included in the immune cell composition, the percentage of B cells may be 5, 4, 3, 2, or 1% or less. In a specific embodiment, the percentage of B cells may be 1% or less. In a specific embodiment, the percentage of B cells may be 0.2% or less. Here, the cells included in the immune cell composition may refer to surviving cells.

### Material that can be further included in immune (cell) composition

As in the combined administration section, which will be described below, an immune cell composition including the third immune cell population or the fourth immune cell population acquired from the third immune cell population may be administered together with a material that can increase a therapeutic effect.

Here, the material to be co-administered may be, for example, an interleukin such as IL-2, and/or an immune checkpoint inhibitor, but the present application is not limited thereto.

In one embodiment, the immune checkpoint inhibitor may be a material that prevents the binding of PD-L1 and PD-1, for example, an anti-PD-L1 agent or an anti-PD-1 agent. In one embodiment, the immune checkpoint inhibitor may be any one or more selected from nivolumab, pembrolizumab, cemiplimab, dostarlimab, atezolizumab, avelumab, durvalumab, and a variant thereof. In a specific embodiment, the immune checkpoint inhibitor may be nivolumab.

The above material that can be co-administered may be further included in the immune cell composition. In this case, the material that can be co-administered in the immune cell composition is administered to a subject, concomitantly with the administration of the immune cell composition. Here, when the co-administered material is included in the immune cell composition, the concentration of the co-administered material may be determined in consideration of an amount suitable for administration to a subject.

In one embodiment, the immune cell composition may further include any one or more selected from IL-2 and an immune checkpoint inhibitor. In a specific embodiment, the immune cell composition may further include IL-2. In a specific embodiment, the immune cell composition may further include nivolumab.

### Use of produced immune (cell) composition

### Overview

The produced immune cell composition may be used in administration to a subject in need thereof. In one embodiment, the produced immune cell composition may be cryopreserved for storage. The cryopreserved immune cell composition may be thawed and used for administration to a subject in need thereof. Here, the period of cryopreserving the immune cell composition is not limited thereto. Moreover, here, the method of cryopreserving the immune cell composition may be, but is not limited to, a method known in the art for cryopreserving the immune cell composition. The administration of the immune cell composition may have an effect of delaying a symptom of a particular medical condition or the progression of a disease, compared to a subject who is not administered the immune cell composition. In one embodiment, the immune cell composition may be filled in a container and administered to a subject.

In one embodiment, a method of treating a tumor in a subject, which includes administering the immune cell composition or immune composition produced by the method for producing the immune cell composition of the present application to a subject is provided.

In one embodiment, a use of the immune cell composition or immune composition produced by the method for producing the immune cell composition of the present application for treating a tumor of a subject is provided.

### Filling container with immune cell composition

The immune cell composition including the third immune cell population or the fourth immune cell population from the third immune cell population may be filled in a container. Here, the container may be an injection container for injecting tumor infiltrating lymphocytes (TILs) expanded according to the method of the present application into a subject. Here, the immune cell composition may be filled in a container in consideration of an amount suitable for administration to a subject (e.g., a patient). Here, a patient's condition, and a condition of the immune cells included in the immune cell composition may be considered.

In one embodiment, the immune cells may be filled in a container such that the concentration of the immune cells is approximately 1E8/200 mL, 2E8/200 mL, 3E8/200 mL, 4E8/200 mL, 5E8/200 mL, 6E8/200 mL, 7E8/200 mL, 8E8/200 mL, 9E8/200 mL, 1E9/200 mL, 2E9/200 mL, 3E9/200 mL, 4E9/200 mL, 5E9/200 mL, 6E9/200 mL, 7E9/200 mL, 8E9/200 mL, 9E9/200 mL, 1E10/200 mL, 2E10/200 mL, 3E10/200 mL, 4E10/200 mL, 5E10/200 mL, 6E10/200 mL, 7E10/200 mL, 8E10/200 mL, 9E10/200 mL, 1E11/200 mL, 2E11/200 mL, 3E11/200 mL, 4E11/200 mL, or 5E11/200 mL or more, but the present application is not limited thereto. In a specific embodiment, the immune cells may be filled in a container such that the concentration of the immune cells is approximately 1E9/200 mL or more. In a specific embodiment, the immune cells may be filled in a container such that the concentration of the immune cells is approximately 1E10/200 mL or more. In a specific embodiment, the immune cells may be filled in a container such that the concentration of the immune cells is 1E10/200 mL to 1.3E11/200 mL.

### Subject for administration

The immune cell composition may be administered to a subject in need thereof. The subject includes a human with a tumor. Here, the tumor includes a solid tumor or a liquid tumor, wherein the solid tumor includes, but not limited to, breast cancer, lung cancer, stomach cancer, ovarian cancer, kidney cancer, or skin cancer including melanoma. The liquid tumor includes, but not limited to, blood cancer, including leukemia or lymphoma, or a pleural effusion of a patient with lung cancer. In a specific embodiment, the immune cell composition may be administered to a human with breast cancer. In a specific embodiment, the immune cell composition may be administered to a human with lung cancer.

In one embodiment, the subject in need of the immune cell composition may be the subject providing tumor tissue in I. Producing semi-product. For example, the immune cell composition produced using this method via a tumor sample derived from a subject (obtained from the tumor tissue acquired from the subject) may be administered back to the same subject.

### Administration method

A method of administering the immune cell composition to a subject in need thereof may be one of the methods known in the art. In one embodiment, the immune cell composition may be intraarterially, intravenously, intracutaneously or intraperitoneally administered. In a specific embodiment, the immune cell composition may be intraarterially or intravenously administered.

In one embodiment, the immune cell composition may be administered with an additional material. Here, the co-administration may include administering the immune cell composition before or after the administration of an additional material, or simultaneously administration with an additional material. The additional material includes all materials known in the art as materials boosting the therapeutic effect for a subject. In one embodiment, the additional material may include IL-2, cyclophosphamide, and/or fludarabine. The co-administration (concomitant administration) with an additional material will be disclosed in detail below.

### Dose

A dose of the immune cell composition administered to a subject in need thereof may be an effective amount suitable for treatment. Here, the dose may vary according to parameters including a subject's tumor volume, body weight, and age. In one embodiment, the immune cell composition including approximately 1E6, approximately 1.5E6, approximately 2E6, approximately 2.5E6, approximately 3E6, approximately 3.5E6, approximately 4E6, approximately 4.5E6, approximately 5E6, approximately 5.5E6, approximately 6E6, approximately 6.5E6, approximately 7E6, approximately 7.5E6, approximately 8E6, approximately 8.5E6, approximately 9E6, approximately 9.5E6, approximately 1E7, approximately 1.5E7, approximately 2E7, approximately 2.5E7, approximately 3E7, approximately 3.5E7, approximately 4E7, approximately 4.5E7, approximately 5E7, approximately 5.5E7, approximately 6E7, approximately 6.5E7, approximately 7E7, approximately 7.5E7, approximately 8E7, approximately 8.5E7, approximately 9E7, approximately 9.5E7, approximately 1E8, approximately 1.5E8, approximately 2E8, approximately 2.5E8, approximately 3E8, approximately 3.5E8, approximately 4E8, approximately 4.5E8, approximately 5E8, approximately 5.5E8, approximately 6E8, approximately 6.5E8, approximately 7E8, approximately 7.5E8, approximately 8E8, approximately 8.5E8, approximately 9E8, approximately 9.5E8, approximately 1E9, approximately 1.5E9, approximately 2E9, approximately 2.5E9, approximately 3E9, approximately 3.5E9, approximately 4E9, approximately 4.5E9, approximately 5E9, approximately 5.5E9, approximately 6E9, approximately 6.5E9, approximately 7E9, approximately 7.5E9, approximately 8E9, approximately 8.5E9, approximately 9E9, approximately 9.5E9, approximately 1E10, approximately 1.5E10, approximately 2E10, approximately 2.5E10, approximately 3E10, approximately 3.5E10, approximately 4E10, approximately 4.5E10, approximately 5E10, approximately 5.5E10, approximately 6E10, approximately 6.5E10, approximately 7E10, approximately 7.5E10, approximately 8E10, approximately 8.5E10, approximately 9E10, approximately 9.5E10, approximately 1E11, approximately 1.5E11, approximately 2E11, approximately 2.5E11, approximately 3E11, approximately 3.5E11, approximately 4E11, approximately 4.5E11, approximately 5E11, approximately 5.5E11, approximately 6E11, approximately 6.5E11, approximately 7E11, approximately 7.5E11, approximately 8E11, approximately 8.5E11, approximately 9E11, approximately 9.5E11, or approximately 1E12 or more immune cells may be administered to a subject in need thereof, once or several times, but the present application is not limited thereto.

### Combined administration

As described above, the immune cell composition may be administered with an additional material. Here, the additional material to be co-administered may be administered before or after the administration of an immune cell composition, or concomitantly administered with the immune cell composition. For example, the additional material may be administered on the day of administering the immune cell composition. In another example, the additional material may be administered to a subject on a day prior to the day of administering the immune cell composition. In still another example, the additional material may be administered to a subject on a day after the day of administering the immune cell composition. In yet another example, the additional material may be administered multiple times, and here, the additional material may be administered once on the same day as that of administering the immune cell composition and administered multiple times on a day after that of administering the immune cell composition. Further, the co-administered additional material may be administered via the same route as those of the immune cell composition, or via a different route.

The additional material includes all materials known in the art as materials boosting the therapeutic effect for a subject. In one embodiment, the additional material may include IL-2, an immune checkpoint inhibitor (e..g., a PD-1 or PD-L1 blocker such as nivolumab, pembrolizumab, semiplimab, dostalimab, atezolizumab, avelumab, or duvalumab), cyclophosphamide, and/or fludarabine.

In one embodiment, the immune cell composition may be administered with an immune checkpoint inhibitor and/or IL-2.

In a specific embodiment, the immune cell composition may be administered with IL-2. Here, the amount of IL-2 administered to a subject may be an effective amount suitable for treatment. Here, the amount of IL-2 administered to a subject may vary according to parameters including a subject's tumor volume, body weight, and age, but the present application is not limited thereto.

In one embodiment, IL-2 may be administered to a subject in need thereof once or multiple times in an amount of approximately 1E3 IU, approximately 5E3 IU, approximately 1E4 IU, approximately 5E4 IU, approximately 1E5 IU, approximately 5E5 IU, approximately 1E6 IU, approximately 5E6 IU, approximately 1E7 IU, approximately 5E7 IU, approximately 1E8 IU, approximately 5E8 IU, approximately 1E9 IU, approximately 5E9 IU, approximately 1E10 IU, approximately 5E10 IU, approximately 1E11 IU, approximately 5E11 IU, approximately 1E12 IU, approximately 5E12 IU, approximately 1E13 IU, approximately 5E13 IU, approximately 1E14 IU, approximately 5E14 IU, approximately 1E15 IU, or approximately 5E15 IU or more.

In a specific embodiment, IL-2 may be administered to a subject in need thereof once or multiple times, and administered in an amount of 720,000 IU/kg or less per administration.

In a specific embodiment, IL-2 may be administered to a subject in need thereof once or multiple times, and administered in an amount of 18E6 IU/head or less per administration.

For example, when IL-2 is intravenously administered, administration according to through the following administration method may be performed once, or repeatedly performed multiple times:
- administering for five days in an amount of 18E6 IU/day
- breaking for 2 to 6 days (discontinuation of administration)
- administering for 5 days in an amount of 18E6 IU/day
- breaking for 3 weeks (discontinuation of administration).

In another example, when IL-2 is subcutaneously administered, administration through the following administration method may be performed once, or repeated multiple times:
- administering for 5 days in an amount of 18E6 IU/day
- breaking for 2 days (discontinuation of administration)
- administering for 3 weeks: In Week 1(first week), on the first and second days, administered in an amount of 18E6 IU; on the third, fourth and fifth days, administered in an amount of 9E6 IU; and on the sixth and seventh days, medication is not administered. Administration in Week 2(second week) and Week 3(third week) during the three weeks is also the same as the administration in Week 1.

In a specific embodiment, IL-2 may be administered at the following dose: administration for 14 days at an amount of 2E6 IU/head per day.

### Exemplary examples of methods disclosed in the present application

Exemplary examples of the methods disclosed herein are presented below. The following exemplary examples are illustrative methods for easy understanding of the methods disclosed in the present application, and the scope of the method disclosed in the present application is not limited by the following examples.

### 1. Exemplary example of producing semi-product (first immune cell population produced and acquired from tumor sample)

### 1) Preparation of tumor sample

Tumor tissue of a patient with breast cancer or lung cancer is obtained from a medical institution. The tumor tissue is washed three times using an RPMI1640 medium not including an antibiotic. Adipose tissue is removed from the washed tumor tissue. The adipose tissue-removed tumor tissue is cut to have a cross-sectional area of approximately 1 mm² to approximately 2 mm², thereby preparing a tumor sample.

### 2) Production of first immune cell population

Twenty-four fragments of tumor sample are placed in a T75 flask along with a CTS-AIM-V medium including approximately 3 vol% of human AB serum and approximately 1,000 IU/mL of IL-2 (first composition, a total of approximately 6 mL), and cultured for 14 days in a 37 °C, 5% CO₂ environment. Here, during the culture, a CTS-AIM-V medium including approximately 3 vol% of human AB serum and approximately 1,000 IU/mL of IL-2 (added first additive composition, a total of approximately 3 mL) is added to a T75 flask on days 7, 10, and 12 from the start date of the culture.

### 3) Obtaining the first immune cell population

The fragments of the tumor tissue are strained from the cultured composition using a cell strainer having a mesh pore of 40 µm. A first immune cell population is acquired by washing the cultured immune cell population three times using DPBS.

### 4) Cryopreservation of first immune cell population

The first immune cell population is suspended at a concentration of approximately 2E6 cells/mL using CryoStor^{®} CS10, and added to a cryovial at 1 mL, followed by storage in gaseous liquid nitrogen (LN2) at approximately -190 °C or less.

### 2. Exemplary example of producing final product (immune cell composition produced by acquiring third immune cell population) from cryopreserved semi-product

### 1) Thawing of cryopreserved first immune cell population

The cryopreserved first immune cell population is thawed in an approximately 37 °C water bath for approximately 2 minutes.

### 2) Seeding of second immune cell population

A second immune cell population divided from the first immune cell population and a CTS-AIM-V medium including approximately 3 vol% of human AB serum, approximately 2,000 IU/mL of IL-2, approximately 30 ng/mL of OKT3, and irradiated PBMCs which have a ratio with the second immune cell population (TIL) of approximately 1:200(TIL : PBMC) (second composition, a total of approximately 20 mL) are seeded in GREX-100M. Here, seeding is performed to include approximately 4E5 immune cells in the GREX-100M.

### 3) Expansion of second immune cell population

During expansion, a CTS-AIM-V medium including approximately 3 vol% of human AB serum and approximately 2,000 IU/mL of IL-2 (added second additive composition) is added to GREX-100M at 20 mL on day 4, 60 mL on day 6, 180 mL on day 8, and 540 mL on day 10 from the start date of expansion. Here, the expansion is performed for 14 days, and during this period, the second immune cell population is not transferred to a new container, or new immune cells are not added from the outside. During the expansion period, the second immune cells are expanded 2,500 times or more.

### 4) Production of immune cell composition

The composition including the immune cell population expanded through the above-described process is centrifuged to acquire a third immune cell population, thereby producing an immune cell composition.

### 3. Exemplary example of producing final product (immune cell composition produced by acquiring third immune cell population) through cryopreservation

### 1) Acquisition of sample for producing immune cell composition

Tumor tissue of a patient with breast cancer or lung cancer is obtained from a medical institution. The tumor tissue is washed three times using an RPMI1640 medium not including an antibiotic. Adipose tissue is removed from the washed tumor tissue. The adipose tissue-removed tumor tissue is cut to have a cross-sectional area of approximately 1 mm² to approximately 2 mm², thereby preparing a tumor sample.

### 2) Production of first immune cell population

Twenty-four fragments of tumor sample are placed in a T75 flask along with a CTS-AIM-V medium including approximately 3 vol% of human AB serum and approximately 1,000 IU/mL of IL-2 (first composition, a total of approximately 6 mL), and cultured for 14 days in a 37 °C, 5% CO₂ environment. Here, during the culture, a CTS-AIM-V medium including approximately 3 vol% of human AB serum and approximately 1,000 IU/mL (added first additive composition, a total of approximately 3 mL) of IL-2 is added to a T75 flask on days 7, 10, and 12 from the start date of the culture.

### 3) Acquisition of first immune cell population

The fragments of the tumor tissue are strained from the culture composition using a cell strainer having a mesh pore of 40 µm. A first immune cell population is acquired by washing the cultured immune cell population three times using DPBS.

### 4) Cryopreservation of first immune cell population

The first immune cell population is suspended at a concentration of approximately 2E6 cells/mL using CryoStor^{®} CS10, and added to a cryovial at 1 mL, followed by storage in gaseous liquid nitrogen (LN2) at approximately -190 °C or less.

### 5) Thawing of cryopreserved first immune cell population

The cryopreserved first immune cell population is thawed in an approximately 37 °C water bath for approximately 2 minutes.

### 6) Seeding of second immune cell population

A second immune cell population divided from the first immune cell population and a CTS-AIM-V medium including approximately 3 vol% of human AB serum, approximately 2,000 IU/mL of IL-2, approximately 30 ng/mL of OKT3, and irradiated PBMCs which have a ratio with the second immune cell population (TIL) of approximately 1:200 (TIL : PBMC) (second composition, a total of approximately 20 mL) are seeded in GREX-100M. Here, seeding is performed to include approximately 4E5 immune cells in GREX-100M.

### 7) Expansion of second immune cell population

During expansion, a CTS-AIM-V medium including approximately 3 vol% of human AB serum and approximately 2,000 IU/mL of IL-2 (added second additive composition) is added to GREX-100M at 20 mL on day 4, 60 mL on day 6, 180 mL on day 8, and 540 mL on day 10 from the start date of expansion. Here, the expansion is performed for 14 days, and during this period, the second immune cell population is not transferred to a new container, or new immune cells are not added from the outside. During the expansion period, the second immune cells are expanded 2,500 times or more.

### 8) Production of immune cell composition

The composition including the immune cell population expanded through the above-described process is centrifuged to acquire a third immune cell population, thereby producing an immune cell composition.

### 4. Exemplary example of producing final product (immune cell composition produced by acquiring third immune cell population) without cryopreservation

### 1) Acquisition of sample for producing immune cell composition

Tumor tissue of a patient with breast cancer or lung cancer is obtained from a medical institution. The tumor tissue is washed three times using an RPMI1640 medium not including an antibiotic. Adipose tissue is removed from the washed tumor tissue. The adipose tissue-removed tumor tissue is cut to have a cross-sectional area of approximately 1 mm² to approximately 2 mm², thereby preparing a tumor sample.

### 2) Production of first immune cell population

Twenty-four fragments of tumor sample are placed in a T75 flask along with a CTS-AIM-V medium including approximately 3 vol% of human AB serum and approximately 1,000 IU/mL of IL-2 (first composition, a total of approximately 6 mL), and cultured for 14 days in a 37 °C, 5% CO₂ environment. Here, during the culture, a CTS-AIM-V medium including approximately 3 vol% of human AB serum and approximately 1,000 IU/mL of IL-2 (added first additive composition, a total of approximately 3 mL) is added to a T75 flask on days 7, 10, and 12 from the start date of the culture.

### 3) Acquisition of first immune cell population

The fragments of the tumor tissue are filtered from the culture composition using a cell strainer having a mesh pore of 40 µm. A first immune cell population is acquired by washing the cultured immune cell population three times using DPBS.

### 4) Seeding of second immune cell population

A second immune cell population divided from the first immune cell population and a CTS-AIM-V medium including approximately 3 vol% of human AB serum, approximately 2,000 IU/mL of IL-2, approximately 30 ng/mL of OKT3, and irradiated PBMCs which have a ratio with the second immune cell population (TIL) of approximately 1:200 (TIL : PBMC) (second composition, a total of approximately 20 mL) are seeded in GREX-100M. Here, seeding is performed to include approximately 4E5 immune cells in GREX-100M.

### 5) Expansion of second immune cell population

During expansion, a CTS-AIM-V medium including approximately 3 vol% of human AB serum and approximately 2,000 IU/mL of IL-2 (added second additive composition) is added to GREX-100M at 20 mL on day 4, 60 mL on day 6, 180 mL on day 8, and 540 mL on day 10 from the start date of expansion. Here, the expansion is performed for 14 days, and during this period, the second immune cell population is not transferred to a new container, or new immune cells are not added from the outside. During the expansion period, the second immune cells are expanded 2,500 times or more.

### 6) Production of immune cell composition

The composition including the immune cell population expanded through the above-described process is centrifuged to acquire a third immune cell population, thereby producing an immune cell composition.

### Exemplary embodiments of methods provided by the present application

Hereinafter, exemplary embodiments of the methods provided by the present application are disclosed. The following exemplary embodiments are provided to easily understand the methods disclosed in the present application, and the scope of the invention disclosed in the present application is not limited to the following examples.

### PR01.

Method for producing first immune cell population, which includes:
(a) culturing a tumor sample in a first container,
   wherein the tumor sample is cultured in a first composition in the first container, wherein the first composition includes a medium, serum, and IL-2,
   the first container is a gas-permeable container, or a container including a gas supply system,
   the culturing is performed for 7 to 21 days, and
   during the culture, a first additive composition is added to the first container once or twice or more; and
(b) obtaining a first immune cell population from the cultured composition in (a).

### PR02.

The method according to PR01, wherein the method further includes preparing a tumor sample.

### PR03.

The method according to any one of PR01 and PR02, wherein the tumor sample is obtained from a tumor fragment obtained from a subject, wherein the tumor sample has a cross-sectional area of approximately 1 mm² to 4 mm².

### PR04.

The method according to any one of PR01 to PR04, wherein, 12 to 24 tumor samples are cultured in (a).

### PR05.

The method according to any one of PR01 to PR04, wherein the serum is human serum.

### PR06.

The method according to any one of PR01 to PR04, wherein the concentration of the serum in the first composition is 5 vol% or less.

### PR07.

The method according to any one of PR01 to PR04, wherein the concentration of the serum in the first composition is approximately 3 vol%.

### PR08.

The method according to any one of PR01 to PR07, wherein the medium in the first composition is an AIM-V medium.

### PR09.

The method according to any one of PR01 to PR07, wherein the medium in the first composition is a CTS-AIM-V medium.

### PR10.

The method according to any one of PR01 to PR09, wherein the concentration of IL-2 in the first composition is 500 IU/mL to 3,000 IU/mL.

### PR11.

The method according to any one of PR01 to PR09, wherein the concentration of IL-2 of the first composition is approximately 1,000 IU/mL.

### PR12.

The method according to any one of PR01 to PR11, wherein the first container is a T75 flask.

### PR13.

The method according to any one of PR01 to PR12, wherein the volume of the first composition is 30% or less of the maximum allowable capacity of the first container.

### PR14.

The method according to any one of PR01 to PR12, wherein, when the first container is a T75 flask, the volume of the first composition is 6 mL.

### PR15.

The method according to any one of PR01 to PR14, wherein the first additive composition includes a medium, serum, and IL-2.

### PR16.

The method according to any one of PR01 to PR14, wherein the first additive composition includes a CTS-AIM-V medium, 3 vol% of serum, and 1,000 IU/mL of IL-2.

### PR17.

The method according to any one of PR01 to PR14, wherein the first additive composition has the same composition substances and composition ratio as the first composition.

### PR18.

The method according to any one of PR01 to PR17, wherein, during the culture, the first additive composition is added to the first container twice or more.

### PR19.

The method according to any one of PR01 to PR17, wherein, during the culture, the first additive composition is added to the first container on days 7,10, and 12 from the start date of the culture, and the amount of the added first additive composition is the same at each addition time.

### PR20.

The method according to any one of PR01 to PR17, wherein, during the culture, the first additive composition may be added to the first container at 3 mL on day 7, 3 mL on day 10, and 3 mL on day 12, from the start date of the culture.

### PR21.

The method according to any one of PR01 to PR20, wherein the culturing is performed for 13 to 15 days.

### PR22.

The method according to any one of PR01 to PR21, wherein the culturing is performed for 14 days.

### F01.

A method of cryopreserving a first immune cell population, including
cryopreserving the first immune cell population produced by any one of PR01 to PR22.

### T01.

A method of thawing the cryopreserved first immune cell population, including thawing the cryopreserved first immune cell population according to F01.

### R01.

A method for producing an immune cell composition, including:
(a) seeding a second composition and a second immune cell population in a second container,
   wherein the second immune cell population is derived from a first immune cell population,
   the second composition includes a medium, serum, 1,000 IU/mL to 3,000 IU/mL of IL-2, anti-CD3 antibody, and feeder cells, and
   the second container is a gas-permeable container, or a container including a gas supply system;
(b) expanding the second immune cell population to produce a third immune cell population,
   wherein, during the expansion, a second additive composition is added to the second container once or twice or more, and
   the expansion is performed for 7 to 21 days; and
(c) obtaining a third immune cell population from the expanded product obtained in (b) and producing an immune cell composition,
   wherein the third immune cell population has at least 2500 times more cells than the second immune cell population.

### R02.

The method according to R01, wherein the first immune cell population is an immune cell population produced by any one of PR01 to PR22, or an immune cell population thawed by T01.

### R03.

The method according to any one of R01 and R02, wherein the medium in the second composition is an AIM-V medium.

### R04.

The method according to any one of R01 to R03, wherein the serum in the second composition is human serum.

### R05.

The method according to any one of R01 to R03, wherein the concentration of the serum in the second composition is 5 vol% or less.

### R06.

The method according to any one of R01 to R03, wherein the concentration of the serum in the second composition is approximately 3 vol%.

### R07.

The method according to any one of R01 to R06, wherein the concentration of IL-2 in the second composition is approximately 2,000 IU/mL.

### R08.

The method according to any one of R01 to R07, wherein the anti-CD3 antibody is OKT3.

### R09.

The method according to any one of R01 to R08, wherein the concentration of the anti-CD3 antibody in the second composition is 10 ng/mL to 100 ng/mL.

### R10.

The method according to any one of R01 to R08, wherein the concentration of the anti-CD3 antibody in the second composition is approximately 30 ng/mL.

### R11.

The method according to any one of R01 to R10, wherein the feeder cells are allogeneic PBMCs.

### R12.

The method according to any one of R01 to R10, wherein the feeder cells are inactivated allogeneic PBMCs.

### R13.

The method according to any one of R01 to R12, wherein the feeder cells are included the second composition such that the ratio of the number of feeder cells is approximately 1:200 (number of cells of second immune cell population : feeder cells) based on the number of cells of the second immune cell population.

### R14.

The method according to any one of R01 to R13, wherein, during the seeding, the number of cells of the second immune cell population seeded in the second container is 1E5 to 10E5.

### R15.

The method according to any one of R01 to R13, wherein, during the seeding, the number of cells of the second immune cell population seeded in the second container is approximately 4E5.

### R16.

The method according to any one of R01 to R15, wherein the second container is GREX100M.

### R17.

The method according to any one of R01 to R16, wherein the volume of the second composition is 30% or less of the maximum allowable capacity of the second container.

### R18.

The method according to any one of R01 to R16, wherein, when the second container is GREX100M, the volume of the second composition is approximately 20 mL.

### R19.

The method according to any one of R01 to R18, wherein, during the expansion, the immune cells are not removed from the second container or not added from the outside.

### R20.

The method according to any one of R01 to R19, wherein the expansion is performed for 13 to 15 days.

### R21.

The method according to any one of R01 to R19, wherein the expansion is performed for 14 days.

### R22.

The method according to any one of R01 to R21, wherein the second additive composition includes a medium, serum, and IL-2.

### R23.

The method according to any one of R01 to R21, wherein the second additive composition includes a CTS-AIM-V medium, 3 vol% of serum, and 2,000 IU/mL of IL-2.

### R24.

The method according to any one of R01 to R21, wherein the second additive composition has the same composition substances and composition ratio as the second composition except for the anti-CD3 antibody and the feeder cells.

### R25.

The method according to any one of R01 to R24, wherein the second additive composition is added to the second container twice or more.

### R26.

The method according to any one of R01 to R24, wherein the second additive composition is added to the second container twice or more, and the amount of the added second additive composition is different at each addition time.

### R27.

The method according to any one of R01 to R24, wherein the second additive composition is added to the second container on days 4, 6, 8, and 10 from the start date of the expansion, and the amount of the added second additive composition is different at each addition time.

### R28.

The method according to any one of R01 to R24, wherein the second additive composition is added to the second container at 20 mL on day 4, 60 mL on day 6, 180 mL on day 8, and 540 mL on day 10, from the start date of the expansion.

### R29.

The method according to any one of R01 to R28, wherein the third immune cell population has at least 4000 times more cells than the second immune cell population.

### R30.

The method according to any one of R01 to R29, wherein the immune cell composition includes the third immune cell population.

### R31.

The method according to any one of R01 to R29, wherein the immune cell composition includes a fourth immune cell population obtained from the third immune cell population.

### R32.

The method according to any one of R01 to R29, wherein the method further includes manipulating a gene of the immune cell.

### R33.

The method according to R32, wherein the manipulating of a gene of the immune cell is performed during or prior to the culturing of the tumor sample in the first container.

### R34.

The method according to R32, wherein the manipulating of a gene of the immune cell is performed during or prior to the seeding of the second composition and the second immune cell population in the second container.

### R35.

The method according to R32, wherein the manipulating of a gene of the immune cell includes introducing a transgene into the immune cell.

### R36.

The method according to R32, wherein the manipulating of a gene of the immune cell includes introducing mRNA into the immune cell.

### R37.

The method according to R32, wherein the manipulating of a gene of the immune cell includes manipulating a gene of the immune cell using a CRISPR-Cas system.

### R38.

The method according to R32, wherein the manipulating of a gene of the immune cell includes manipulating a gene of the immune cell by a transposon.

### R39.

The method according to R32, wherein the manipulating of a gene of the immune cell includes reducing the expression of a gene by siRNA or an antisense oligonucleotide.

### C01.

An immune cell composition including TILs, produced by R01 to R39.

### C02.

The immune cell composition according to C01, wherein the immune cell composition further includes IL-2.

### C03.

The immune cell composition according to any one of C01 and C02, wherein the immune cell composition further includes an immune checkpoint inhibitor.

### C04.

The immune cell composition according to any one of C01 and C02, wherein the immune cell composition further includes nivolumab.

### U01.

A use of the immune cell composition including TILs produced according to R01 to R39 for treating a tumor of a subject.

### U02.

The use according to U01, wherein the subject is a subject from which tumor infiltrating lymphocytes are derived.

### TM01.

A method of treating a tumor of a subject, including administering to the subject an immune cell composition including TILs, produced according to R01 to R39.

### TM02.

The method according to TM01, wherein the subject is a TIL-derived subject.

### TM03.

The method according to any one of TM01 and TM02, wherein the method further includes administering IL-2.

### TM04.

The method according to any one of TM01 to TM03, wherein the method further includes administering an immune checkpoint inhibitor.

### TM05.

The method according to any one of TM01 to TM03, wherein the method further includes administering nivolumab.

### Characteristics of method for producing immune cell composition disclosed in the present application

### IL-2 concentration

Generally, in the production of an immune cell composition including TILs, a high concentration of IL-2 is added (e.g., 6,000 IU/mL) to a medium for TIL culture. However, the method disclosed herein uses a first composition including a lower concentration of IL-2 in the culturing. The present inventors proved through experiments that the use of the composition to which a lower concentration of IL-2 is added is sufficient for the culturing of an immune cell population including TILs (see Experimental Example 3).

In addition, in addition to the composition for culturing, a high concentration of IL-2 (e.g., 3,000 IU/mL) is generally added to the composition for expansion. However, the second composition for expansion disclosed herein may include a lower concentration of IL-2. The present inventors of the present application proved through experiments that the use of the composition to which a low concentration of IL-2 is added is sufficient for the expansion of an immune cell population including TILs (see Experimental Example 3).

In conclusion, the above is significant in that an effective process that can sufficiently culture and expand an immune cell population including TILs with a low concentration of IL-2 is established.

### Culture and/or expansion composition(s)

### 1) Amount of composition for initial culturing and/or composition for expansion

Generally, in the culture and/or expansion of cells including TILs, the amount(s) of the composition for culturing and/or expansion varies according to a container in which culturing and/or expansion occurs. In addition, here, the determined amount(s) of the composition for culturing and/or expansion is/are generally determined by the maximum allowable capacity of a container. However, in the method disclosed herein, the amount of the composition for culturing (e.g., first composition) and/or expansion (e.g., second composition) is less than the maximum allowable capacity of a container at the start of cell culture and/or expansion. That is, the amount of the composition for culturing and/or expansion is set to be less than the maximum allowable capacity of a container and thus the cell concentration is adjusted to be optimal for culture and/or expansion, followed by starting culture and/or expansion. The optimized concentration of cells in the composition may have a positive effect on interactions between cells, and thus serves to increase the efficiency of cell culture and/or expansion. Referring to Experimental Example 4 and FIG. 8, when cells were cultured by varying the amount of the composition using a 24-well plate, it can be confirmed that the number of cells was higher when 1 mL was added. According to this, it means that the efficiency of cell culture and/or expansion can be further increased through the optimization of the concentration of cells in the composition.

### 2) Addition of first additive composition and/or second additive composition

Generally, in the producing of the immune cell composition including TILs, during the culture and expansion of TILs, the culture and/or expansion composition(s) is/are replaced. However, according to the method disclosed in the present application, in the process of culturing and expanding the immune cell population including TILs, the composition for culture or expansion is not replaced but only added by addition in the culture and expansion. This has two advantages.

First, materials required for TILs culture or expansion can be supplied whilie minimizing the stress that can be applied to TILs during the process of complicated TIL culture or expansion. In addition, it is also possible to prevent the loss of an immune cell population that can occur in the replacement of the culture and/or expansion composition(s).

Second, through the addition of the composition, the concentration of cells cultured and/or proliferated in the composition may be continuously maintained as an optimal concentration effective for culturing and/or proliferation. This means that the cell culture and/or proliferation environment are continuously maintained as an environment optimized for culture and/or proliferation, and through this, an immune cell composition can be produced with a higher yield.

In conclusion, the concentration of cells in the composition is optimized by adjusting the amount of the composition at the time of culture and/or expansion, and furthermore, the optimized concentration of the cells in the composition is maintained by adding the culture and/or expansion composition(s) during culture and/or expansion. In addition, during this process, since the composition is added, rather than replaced, an immune cell composition including TILs may be produced more efficiently and with a higher yield.

### Expansion efficiency

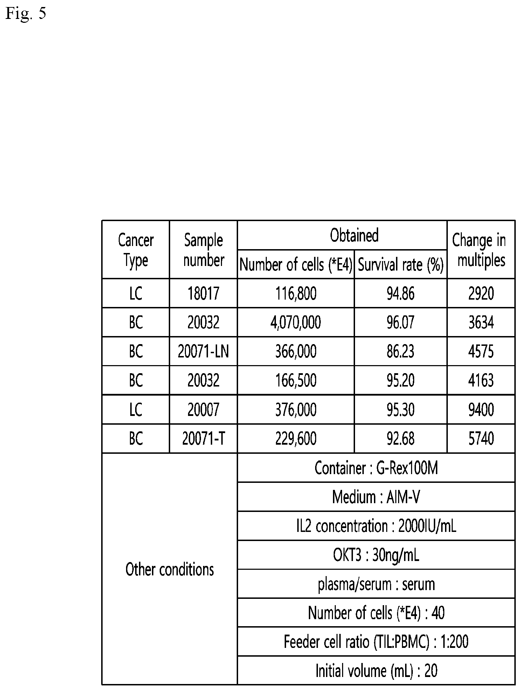

Generally, during the expansion of immune cells including TILs, TILs are expanded approximately 100 to 1,000 times. However, in the method disclosed in the present application, during expansion, TILs are expanded approximately 2,500 times or more on average. In the present application, compared to other general processes, the composition for expansion has a lower IL-2 concentration and also has a high expansion rate of approximately 2,500 times or more. This indicates that the present inventors have established an efficient immune cell production method with a higher expansion rate using a smaller amount of additive than other processes, which is shown in Experimental Example 2 and FIG. 5.

### Cryopreservation period

According to the method of producing an immune cell composition disclosed in the present application, a first immune cell population may be acquired and then cryopreserved to be stored for a certain period of time or longer, and the cryopreserved first immune cell population may be expanded after thawing in time of need, and may be used to produce an immune cell composition. Here, as an example, the first immune cell population may be cryopreserved and stored for approximately 1, 2, 3, 4, or 5 years or more, and then expanded and used to produce an immune cell composition. As another example, the first immune cell population may be cryopreserved for a short period, thawed, and then used to produce an immune cell composition. Accordingly, it is very significant that the first immune cells acquired according to the method disclosed in the present application can be used for expansion after short-term or long-term cryopreservation depending on circumstances. For example, when the production of a semi- product and the production of a final product are executed in different locations, or different companies, the first immune cell population may be transferred to another location after cryopreservation. In addition, for example, when the cancer of a patient recurs or metastasizes, the previously cryopreserved first immune cell population may be thawed, expanded, and used for patient treatment.

### [Experimental Examples]

The present invention provided in the present application will be described in further detail with reference to experimental examples and examples below. These examples are merely provided to illustrate the content disclosed in the present application, and it will be apparent to those of ordinary skill in the art that the scope of the content disclosed in the present application is not to be construed as being limited by the examples.

**Table 1. Reagent information**

| **Reagent** | **Information** |
|---|---|
| RPMI 1640 | Gibco, Cat no. 22400-089 |
| CTS-AIM-V | Gibco, Cat no. A38308 |
| IL-2 | Norvatis, aldesleukin |
| Human serum | SEXTON Biotechnologies, Cat no. PL-SP-500 |
| DPBS | Biowest, Cat no. L0615-500 |
| Cryo stor-10 | Biolife solution, Cat no. 210102 |
| OKT3 | BD bioscience, Cat no. 555336 |
| AlyS505N | Cell science & Technology, Cat no. 1020P10 |

### Experimental Example 1. TIL production

### Experimental Example 1-1. TIL isolation/culture process

### 1. TIL isolation/culture process

### - Day 0

Tumor tissue transported from the operating room of a hospital was washed three times using an RPMI1640 medium and transferred to a 100 mm petri dish. Adipose tissue in the tumor tissue was removed using sterilized surgical scissors and tweezers, and the tumor tissue was cut to a size of 1 to 2 mm using the sterilized surgical scissors and tweezers. Twenty-four fragments of the tumor tissue cut to a size of 1 to 2 mm were put into a T75 flask, and 6 mL of the prepared culture composition was added, followed by culturing at 37 °C in a 5% CO₂ incubator for 7 days.

The configuration of the culture composition is as follows: CTS-AIM-V medium, 3 vol% of human serum, and 1,000 IU/mL of IL2.

### - Day 7

3 mL of the prepared culture composition was added to each flask, and cultured at 37 °C in a 5% CO₂ incubator for 3 days.

### - Day 10

3 mL of the prepared culture composition was added to each flask, and cultured at 37 °C in a 5% CO₂ incubator for 2 days.

### - Day 12

3 mL of the prepared culture composition was added to each flask, and cultured at 37 °C in a 5% CO₂ incubator for 2 days.

### - Day 14

To collect cells, a 40-µm cell strainer was installed on top of a 50 mL tube. Tumor tissue was filtered by passing the cultured cells through the cell strainer using a 10 mL pipette, and only the cultured cells were collected in the 50 mL conical tube and then centrifuged at room temperature at 450 g for 5 minutes. 5 mL of DPBS was added to the precipitated cells, which were then suspended by pipetting. The suspended cells were counted and cell viability was determined using a hemocytometer (C-chip). After centrifugation at 450 g for 5 minutes, the cells were suspended in Cryo-stor CS10 at 2E6 cells/mL, loaded into a cryovial at 1 mL, and stored in a liquid nitrogen tank (LN₂) or a gaseous liquid nitrogen tank (LN₂).

### Experimental Example 1-2. TIL expansion process

### 1. Isolation of allo-feeder PBMCs

### - Leukapheresis for healthy volunteers and irradiation

Leukapheresis was performed on a blood bag from a healthy volunteer using leukapheresis equipment in a hospital blood collection room. The blood bag in which leukapheresis was performed was inactivated by irradiation with X-ray radiation.

### - Isolation of PBMCs from irradiated blood bag

The irradiated blood that had undergone leukapheresis was mixed with DPBS at a ratio of 1:2. After adding 15 mL of Ficoll solution to a 50 mL tube, 30 mL of the blood mixture was superimposed on the Ficoll layer without being mixed with Ficoll and centrifuged at room temperature at 800 g for 20 minutes under the condition of Acc9/Dcc0. The separated PBMC layer was collected and then transferred to a new 50 mL tube. DPBS was added to the 50 mL tube to which the PBMC layer was transferred for washing, and then centrifugation was performed at room temperature and 800 g for 5 minutes. After centrifugation, a supernatant was completely removed, and the precipitated PBMCs were suspended in 50 mL of a medium for culturing a final product. The suspended cells were counted using a hemocytometer set, and cell viability was determined. After cell counting, 3.2E9 PBMCs were prepared.

### 2. Production of final product

### - Day 0 (cell seeding)

Eleven vials of the semi-product stored in a liquid nitrogen tank (LN₂) were thawed in a water bath at 37 °C for 2 minutes. The thawed cells were washed with a CTS-AIM-V medium and centrifuged at room temperature and 1,500 rpm for 5 minutes. After discarding the supernatant, the resulting pellet was suspended in the CTS-AIM-V expansion composition, the cells were counted using a hemocytometer set, and cell viability was determined. After cell counting, 1.6E7 cells were prepared. 4E5 semi-product TILs and 8E7 irradiated PBMCs were put into each of 40 GREX100M (1L) culture containers, and a final product expansion composition was added at 20 mL. The resulting cell mixture was cultured at 37 °C in a 5% CO₂ incubator.

The configuration of the final product expansion composition is as follows: CTS-AIM-V medium, 3 % of human serum, 2,000 IU/mL of IL-2, and 30 ng/mL of OKT3

### - Day 4

IL-2 and the subdivided human serum were added to a CTS-AIM-V medium to 2,000 IU/mL and 3%, respectively, thereby preparing an additive composition in an amount needed. 20 mL of the prepared additive composition was additionally dispensed into each GREX flask and cultured at 37 °C in a 5% CO₂ incubator.

### - Day 6

IL-2 and the subdivided human serum were added to a CTS-AIM-V medium to 2,000 IU/ml and 3%, respectively, thereby preparing an additive composition in an amount needed. 60 mL of the prepared additive composition was additionally dispensed into each GREX flask and cultured at 37 °C in a 5% CO₂ incubator.

### - Day 8

IL-2 and the subdivided human serum were added to a CTS-AIM-V medium to 2,000 IU/ml and 3%, respectively, thereby preparing an additive composition in an amount needed. 180 mL of the prepared additive composition was additionally dispensed into each GREX flask and cultured at 37 °C in a 5% CO₂ incubator.

### - Day 10

IL-2 and the subdivided human serum were added to a CTS-AIM-V medium to 2,000 IU/ml and 3%, respectively, thereby preparing an additive composition in an amount needed. 540 mL of the prepared additive composition was additionally dispensed into each GREX flask and cultured at 37 °C in a 5% CO₂ incubator.

### - Day 14

The cultured cells were carefully suctioned while leaving 200 mL of a medium, and the remaining 200 mL of the medium was gently shaken and suspended and collected in a 500 mL Corning bottle. The collected cells were centrifuged at room temperature and 2000 g for 7 minutes. After centrifugation, the supernatant was completely removed, and the precipitated cells were suspended in 260 mL of an excipient. The suspended cells were counted using a hemocytometer set, and cell viability was determined. After cell counting, 200 mL of the cells were loaded into an infusion bag.

### Experimental Example 2. Experiment for TIL expansion yield

An experiment for measuring the expansion yield of TILs was performed according to the experimental protocol for TIL production (Experimental Example 1).

After acquiring the TILs produced according to the experimental protocol for TIL production (Experimental Example 1), the cells were counted using a hemocytometer set and cell viability was determined.

Referring to FIG. 5, as a result of the TIL expansion yield experiment, during the TIL expansion process, it was confirmed that TILs were expanded at least 2,500 times, and expanded at most 9,400 times.

### Experimental Example 3. Optimization Experiment for IL-2

### Experimental Example 3-1. Experiment for IL-2 optimization in TIL isolation/culture step

### 1. TIL isolation/culture process for IL-2 optimization

### - Day 0

Tumor tissue transported from the operating room of a hospital was washed three times using an RPMI1640 medium and transferred to a 100 mm petri dish. Adipose tissue in the tumor tissue was removed using sterilized surgical scissors and tweezers, and the tumor tissue was cut to a size of 1 to 2 mm using the sterilized surgical scissors and tweezers. Two fragments of the tumor tissue cut to a size of 1 to 2 mm were put into a 24-well plate, and 1 mL of the prepared culture composition according to IL-2 concentration was added, followed by culturing at 37 °C in a 5% CO₂ incubator for 7 days.

### - Day 7

0.5 mL of the prepared culture composition according to IL-2 concentration was added to each well, and cultured at 37 °C in a 5% CO₂ incubator for 3 days.

### - Day 10

0.5 mL of the prepared culture composition according to IL-2 concentration was added to each well, and cultured at 37 °C in a 5% CO₂ incubator for 2 days.

### - Day 12

0.5 mL of the prepared culture composition according to IL-2 concentration was added to each well, and cultured at 37 °C in a 5% CO₂ incubator for 2 days.

### - Day 14

To collect cells, a 40-µm cell strainer was installed on top of a 50 mL tube. Tumor tissue was filtered by passing the cultured cells through the cell strainer using a 10 mL pipette, and only the cultured cells were collected in the 50 mL conical tube and then centrifuged at room temperature at 450 g for 5 minutes. 1 mL of DPBS was added to the precipitated cells, which were then suspended by pipetting. The suspended cells were counted using a hemocytometer (C-chip), and cell viability was determined, thereby determining the cell proliferation rate for each concentration of IL-2.

Referring to FIG. 6, in the condition in which human AB serum was added, the cell proliferation rate did not show a significant difference when the concentration of IL-2 was 1,000 IU/mL or more.

### Experimental Example 3-2. Experiment for IL-2 concentration optimization in TIL expansion step

### 1. Isolation of allo-feeder PBMCs

The PBMC isolation method was performed in the same manner as in Experimental Example 1-2. TIL expansion process.

### 2. TIL expansion process for IL-2 optimization

### - Day 0 (cell seeding)

Semi-product vials stored in a liquid nitrogen tank (LN₂) were thawed in a water bath at 37 °C for 2 minutes. The thawed cells were washed with a CTS-AIM-V medium and centrifuged at room temperature and 1,500 rpm for 5 minutes. After discarding the supernatant, the resulting pellet was suspended in the CTS-AIM-V expansion composition, the cells were counted using a hemocytometer set, and cell viability was determined. 5E4 semi-product TIL cells and 1E7 irradiated PBMCs were put into each T75 flask culture container, and an expansion composition was added at 3 mL. In addition, the resulting cell mixture was cultured at 37 °C in a 5% CO₂ incubator.

### - Day 4

Each of 1000, 2000 4000, and 6,000 IU/mL of IL-2 was put into a 3 vol% human serum-containing CTS-AIM-V medium, thereby preparing an additive composition in an amount needed. 6 mL of the prepared additive composition was additionally dispensed into each T75 flask and cultured at 37 °C in a 5% CO₂ incubator.

### - Day 6

Each of 1000, 2000 4000, and 6,000 IU/mL of IL-2 was put into a 3 vol% human serum-containing CTS-AIM-V medium, thereby preparing an additive composition in an amount needed. 18 mL of the prepared additive composition was additionally dispensed into each T75 flask and cultured at 37 °C in a 5% CO₂ incubator.

### - Day 8

Each of 1000, 2000 4000, and 6,000 IU/mL of IL-2 was put into a 3 vol% human serum-containing CTS-AIM-V medium, thereby preparing an additive composition in an amount needed. 54 mL of the prepared additive composition was additionally dispensed into each T75 flask and cultured at 37 °C in a 5% CO₂ incubator.

### - Day 10

Each of 1000, 2000 4000, and 6,000 IU/mL of IL-2 was put into a 3 vol% human serum-containing CTS-AIM-V medium, thereby preparing an additive composition in an amount needed. 162 mL of the prepared additive composition was additionally dispensed into each T75 flask and cultured at 37 °C in a 5% CO₂ incubator.

### - Day 14

The cultured cells were carefully suctioned while leaving 150 mL of a medium, and the remaining 150 mL of the medium was gently shaken and suspended and collected in a 50 mL tube. The collected cells were centrifuged at room temperature and 2000 g for 7 minutes. After centrifugation, the supernatant was completely removed, and the precipitated cells were suspended in 30 mL of DPBS. The suspended cells were counted using a hemocytometer set, and cell viability was determined.

Referring to FIG. 7, it can be confirmed that even a composition to which 2,000 IU/mL of IL-2 was added was sufficient to expand TILs. In addition, considering both cell expansion yield and cell viability, it can be confirmed that the composition to which 2,000 IU/mL of IL-2 was added is the most efficient for TIL expansion.

### Experimental Example 4. Experiment for optimization of culture composition amount

### 1. TIL isolation/culture process for optimization of culture composition amount

### - Day 0

Tumor tissue transported from the operating room of a hospital was washed three times using an RPMI1640 medium and transferred to a 100 mm petri dish. Adipose tissue in the tumor tissue was removed using sterilized surgical scissors and tweezers, and the tumor tissue was cut to a size of 1 to 2 mm using the sterilized surgical scissors and tweezers. Two fragments of the tumor tissue cut to a size of 1 to 2 mm were put into a 24-well plate, and 1 mL or 2 mL of the prepared culture composition was added, followed by culturing at 37 °C in a 5% CO₂ incubator for 7 days.

### - Day 7

0.5 mL of the prepared culture composition was added to the well into which 1 mL of the composition was added on day 0, and 1 mL of the composition was carefully removed from the well into which 2 mL of the composition was added on day 0 and then 1 mL of the composition was added thereto, followed by culturing at 37 °C in a 5% CO₂ incubator for 3 days.

### - Day 10

0.5 mL of the prepared culture composition was added to the well into which 1 mL of the composition was added on day 0, and 1 mL of the composition was carefully removed from the well into which 2 mL of the composition was added on day 0 and then 1 mL of the composition was added thereto, followed by culturing at 37 °C in a 5% CO₂ incubator for 3 days.

### - Day 12

0.5 mL of the prepared culture composition was added to the well into which 1 mL of the composition was added on day 0, and 1 mL of the composition was carefully removed from the well into which 2 mL of the composition was added on day 0 and then 1 mL of the composition was added thereto, followed by culturing at 37 °C in a 5% CO₂ incubator for 3 days.

### - Day 14

To collect cells, a 40-µm cell strainer was installed on top of a 50 mL tube. Tumor tissue was filtered by passing the cultured cells through the cell strainer using a 10 mL pipette, and only the cultured cells were collected in the 50 mL conical tube and then centrifuged at room temperature and 450 g for 5 minutes. 1 mL of DPBS was added to the precipitated cells, which were then suspended by pipetting. The suspended cells were counted using a hemocytometer (C-chip), and cell viability was determined, thereby determining the cell proliferation rate according to the amount of the culture composition added on day 0.

Referring to FIG. 8, as a result of the experiment using a 24-well plate, it can be confirmed that, when 1 mL of the composition was used, the cell proliferation rate is higher than when 2 mL of the composition was used.

### Experimental Example 5. Experiment for counting cells acquired per tumor fragment

In the same manner as in Experimental Example 1-1. TIL isolation/culture process, experiments were performed using lung cancer (LC) tumor tissue, head-and-neck cancer (HNC) tumor tissue, and breast cancer (BC) tumor tissue. As a result, it was confirmed how many cells were obtained on average per tumor fragment. Referring to FIG. 9, in the case of LC tumor tissue, approximately 4E5 cells were obtained on average per tumor fragment. In the case of HNC tumor tissue, approximately 6E5 cells were obtained on average per tumor fragment. In the case of BC tumor tissue, approximately 6E5 cells were obtained on average per tumor fragment.

### Experimental Example 6. Experiment for confirming T cell percentage in cultured and expanded TILs

### 1. Preparation of antibody for confirming T cell percentage

As antibodies for confirming a T cell percentage, antibodies against CD3 and CD45 were prepared, and an isotype control antibody was also prepared.

**Table 2. Information on antibodies for confirming T cell percentage**

| **Set** | **Antibody No.** | **Marker** | **Fluorescence** | **Manufacturer** | **Cat #** |
|---|---|---|---|---|---|
| **Isotype control** | I-1 | Mouse IgG2a | APC-cy7 | BioLegend | 400230 |
| | I-2 | Mouse IgG1 | BV510(Amcyan) | BioLegend | 400172 |
| **Sample** | S-1 | CD3 | APC-cy7 | BioLegend | 300318 |
| | S-2 | CD45 | BV510(Amcyan) | BioLegend | 304036 |

### 2. Pretreatment for FACS analysis

The cultured and expanded TILs were put into a 15 mL or 50 mL conical tube, washed twice with FACS buffer (2% FBS in DPBS), and suspended in FACS buffer. 5 x 10⁵ of the suspended cells were put into a 1.7 mL microcentrifuge tube, centrifuged at 4 °C at 1,500 rpm for 5 minutes, and the supernatant was discarded except for supernatant 200 µL. The remaining supernatant was vortexed to suspend the cells, and 5 µL of each antibody was put into each tube as below.

**Table 3. Antibody added to tube**

| | | | | | |
|---|---|---|---|---|---|
| Tube | 1 | 2 | 3 | 4 | 5 |
| Antibody | No stain | I-1 & I-2 | S-1 | S-2 | S-1 & S-2 |

After adding the antibody, incubation was performed at 4 °C in a dark room for 30 minutes. After the reaction, the cells were washed three times with FACS buffer, centrifuged at 4 °C, 1,500 rpm for 5 minutes, and the supernatant was discarded except for approximately 200 µL. The remaining supernatant was vortexed to suspend the cells, and 300 µL of FACS buffer was added to perform FACS analysis.

### 3. FACS data analysis

After data was loaded into an FSC/SSC dot plot from FACS equipment, a lymphocyte region was gated. After loading the gated lymphocyte population into a dot plot, CD3-APC-cy7 was set on the x axis and CD45-Amcyan was set on the y axis, and quadrants were set. The percentages of CD3-positive and CD45-positive T cells in the quadrant were confirmed.

Referring to FIG. 10, as a result of measuring the percentage of T cells in TILs produced according to Experimental Example 1, it can be seen that 99.7%, 99.4%, or 98.4% of the produced TILs are T cells. That is, it was confirmed that T cells were present with high ratio in the TILs produced according to the production process disclosed in the present application.

### Experimental Example 7. Expansion (final product production) using semi-product cryopreserved for a long period of time and confirmation of T cell percentage in expanded TILs

### Expansion of cryopreserved semi-product

### 1. Isolation of allo-feeder PBMCs

The blood obtained from a normal donor was prepared through leukapheresis and irradiated with 60Gy X-rays. 15 mL of Ficoll solution was put into the bottom of a 50 mL conical tube without bubbles. After dividing the collected blood into 50 mL conical tubes, the amount of blood was measured. The blood divided into the 50 mL conical tubes was diluted at a ratio of 1:2 (Blood:DPBS). 30 mL of the diluted blood was carefully added to the 50 mL conical tube filled with 15 mL of Ficoll solution, and then centrifugation was performed at 800 g for 20 minutes at RT under the condition of Acc9/Dcc0. Only the PBMC layer was taken and transferred to a new 50 mL conical tube, and then centrifuged at 800 g for 5 minutes at RT under the condition of Acc9/Dcc9. After centrifugation, the supernatant was completely removed, DPBS was filled up to 50 mL, washed, and then centrifuged at 800 g for 5 minutes at RT under the condition of Acc9/Dcc9. After centrifugation, the supernatant was completely removed, and the precipitated cells were suspended in DPBS. The suspended cells were counted using a hemocytometer set and cell viability was determined. After cell counting, the required number of the cells according to a ratio of 1:200 (TILs cultured for 2 weeks: PBMCs) was prepared. The prepared cells were centrifuged at RT and 800 g for 5 minutes under the condition of Acc9/Dcc9. After centrifugation, the supernatant was completely removed, and the precipitated cells were suspended in a final product culture complete medium, and used in the manufacture of final product.

### 2. Production of final product

### - Day 0 (Thawing and cell seeding)

A vial of semi-product stored in liquid nitrogen tank (LN₂) for a long period of time of 1 year or more was thawed. The TIL semi-product was transferred to a 50 mL conical tube from the thawed vial. The TIL semi-product was washed with an AIM-V medium (volume ratio of 9:1 (medium: TIL semi-product)). The TIL semi-finished product was centrifuged at RT and 1,500 rpm for 5 minutes under the condition of Acc9/Dcc9, the supernatant was discarded, and the precipitated cells were suspended in a CTS-AIM-V medium, followed by cell counting and confirmation of cell viability. 4E5 semi-product TIL cells and 8E7 irradiated PBMCs (Allo-PBMC) were put into GREX100M(1L) culture containers, respectively, and then a final product expansion composition was added to a final volume of 20 mL. Incubation was performed at 37 °C in a 5% CO₂ incubator.

The configuration of the final product expansion composition is as follows: CTS-AIM-V medium, 3 vol% human serum, 2,000 IU/mL IL-2, and 30 ng/mL OKT3

### - Day 4

A needed amount of expansion composition was prepared by adding IL-2 and the subdivided human serum to a CTS-AIM-V medium to 2,000 IU/mL and 3%, respectively. 20 mL of the prepared expansion composition was further added to each GREX flask and incubated at 37 °C in a 5% CO₂ incubator.

### - Day 6

A needed amount of expansion composition was prepared by adding IL-2 and the subdivided human serum to a CTS-AIM-V medium to 2,000 IU/mL and 3%, respectively. 60 mL of the prepared expansion composition was further added to each GREX flask and incubated at 37 °C in a 5% CO₂ incubator.

### - Day 8

A needed amount of expansion composition was prepared by adding IL-2 and the subdivided human serum to a CTS-AIM-V medium to 2,000 IU/mL and 3%, respectively. 180 mL of the prepared expansion composition was further added to each GREX flask and incubated at 37 °C in a 5% CO₂ incubator.

### - Day 10

A needed amount of expansion composition was prepared by adding IL-2 and the subdivided human serum to a CTS-AIM-V medium to 2,000 IU/mL and 3%, respectively. 540 mL of the prepared expansion composition was further added to each GREX flask and incubated at 37 °C in a 5% CO₂ incubator.

### - Day 14

The cultured cells were carefully suctioned while leaving 200 mL of a medium, and the remaining 200 mL of the medium was gently shaken and suspended and collected in a 500 mL Corning bottle. 150 mL of DPBS was put into a flask in which the cell harvest had been done to recover the cells in the container (repeated twice). The collected cells were centrifuged at room temperature at 2,000 g for 7 minutes. After centrifugation, the supernatant was completely removed, and DPBS was added up to 500 mL for washing (2,000 g, 7 min, RT, Acc9/Dcc9, repeated three times). After centrifugation, the supernatant was completely removed, and the precipitated cells were suspended in DPBS. The suspended cells were counted using a hemocytometer set, and cell viability was determined. Afterward, some of the cells were used for the following confirmation test, and the remainder was suspended at 3 x 10⁷ cells/mL in CS10, dispensed into cryogenic vials, and frozen in a cryo container using a deep-freezer (-80 °C).

### Confirmation of cell viability upon thawing of cryopreserved semi-product

### Method

The cryopreserved semi-finished product was thawed, and cell viability was confirmed after thawing. One vial each of the TIL semi-products from three different cases (BC16208, BC17058, and BC18050), which had been stored in liquid nitrogen for different periods, was thawed. After thawing, cell viability was confirmed.

### Result

As a result of confirming cell viability after thawing, for the case of BC16208, the cell viability after thawing was 91.25%, for the case of BC17058, 95.88%, and for the case of BC18050, 69.37%.

**Table 4. Confirmation of cell viability upon semi-finished product thawing**

| **Case** | **Cell counting** | | **Cell viability (%)** |
|---|---|---|---|
| | **Live** | **Total** | |
| BC16208 (Cryopreservation period: 6 months) | 135 | 148 | 91.25 |
| | 126 | 138 | |
| BC17058 (Cryopreservation period: 50 months) | 159 | 165 | 95.88 |
| | 167 | 175 | |
| BC18050 (Cryopreservation period: 33 months) | 152 | 220 | 69.37 |
| | 156 | 224 | |

### Confirmation of yield in final product

### Method

Final products were prepared using the TIL semi-products from the thawed three cases (BC16208, BC17058, BC18050), and the number, cell viability, and cell proliferation rate of the finally harvested cells were measured.

### Result

It was confirmed that, for the case of BC16208, 1.31x10⁹ cells, for the case of BC17058, 1.71x10⁹ cells, and for the case of BC18050, 1.42x10⁹ cells were proliferated approximately 3,270, 4,275, and 3,540 times, respectively. It was confirmed that all of the cases show high cell viability of 90% or more.

**Table 5. Conditions for semi-product cell seeding to prepare final product**

| Case | Seeding Conditions | | | | |
|---|---|---|---|---|---|
| | IL-2 Cone. (IU/mL) | Supplement | | Density | Culture ware |
| | | Serum(%) | OKT-3(ng/mL) | | |
| BC16208 | 2,000 | 3 | 30 | 4 x 10⁵ cells/20mL (2 x 10⁴ cells/mL) | G-Rex |
| BC17058 | 2,000 | 3 | 30 | 4 x 10⁵ cells/20mL (2 x 10⁴ cells/mL) | G-Rex |
| BC18050 | 2,000 | 3 | 30 | 4 x 10⁵ cells/20mL (2 x 10⁴ cells/mL) | G-Rex |

**Table 6. Production yield in production of final product**

| Case | Number of harvested cells (cells) | Cell viability (%) | Cell proliferation rate (fold change) |
|---|---|---|---|
| BC16208 | 1.31 x 10⁹ | 96.79 | 3270 |
| BC17058 | 1.71 x 10⁹ | 94.97 | 4275 |
| BC18050 | 1.42 x 10⁹ | 93.64 | 3540 |

### Confirmation of cell percentage in final product

### Analysis method through FACS

A cell percentage in a final product was confirmed through FACS analysis. Cells used for the FACS analysis were suspended in FACS buffer (2% FBS in DPBS) and centrifuged (1,500 rpm, 4 °C, 5 min) (repeated twice). After centrifugation, the cells were suspended in FACS buffer. 1 to 5 x 10⁵ cells of the suspended cells were dispensed into each 1.7 mL microtube. For surface marker staining, each tube was treated with 2 µL of antibodies. Incubation was performed at 4 °C in a dark room for 30 minutes, and after incubation, 800 µL of FACS buffer was added, and then centrifugation (1,500 rpm, 4 °C, 5 min) was performed. After discarding the supernatant, the cells were suspended by vortexing, 800 µL of FACS buffer was added thereto, and then centrifugation (1,500 rpm, 4 °C, 5 min) was performed (repeated three times). After suspending using 200 µL of FACS buffer, the cells were transferred to a 5 mL FACS tube, and analysis was performed using a BD FACS Canto^{™} II flow cytometer.

### Result

The expression of CD3 and CD45, which are T cell markers, was confirmed by FACS analysis of the produced final products. To perform the analysis only on surviving cells, the cells were firstly gated with SSC-A and FSC-A (upper left graphs in FIGS. 12, 14, and 16). Here, the gated surviving cells were displayed as lymphocytes on the upper left graphs of FIGS. 12, 14, and 16. As a result of the experiment, the percentages of T lymphocytes (CD3+/CD45+) expressing both of CD3 and CD45 were 99.5% in the case of BC16208, 99.4% in the case of BC17058, and 99.1% in the case of BC18050. In addition, it was confirmed that, in the case of BC16208, the percentage of CD4+ T cells was 83.6% and the percentage of CD8+ T cells was 13.2%, and in the case of BC17058, the percentage of CD4+ T cells was 93.2%, and the percentage of CD8+ T cells was 4.71%, and in the case of BC18050, the percentage of CD4+ T cells was 78.4%, and the percentage of CD8+ T cells was 14.0% (see FIGS. 12, 14, and 16). As reference, in the table below, the percentage of each type of T cells among the CD3+/CD45+ lymphocytes was a percentage converted based on the FACS result of CD3+/CD45+ lymphocytes as 100%. The analysis results are summarized in Table 7 below.

**Table 7. Confirmation of cell percentage by case**

| Case | Live cells (lymphocytes) | CD45- | CD3- | CD3+ CD45+ | CD3+ CD45+ (T cell) | | | |
|---|---|---|---|---|---|---|---|---|
| | | | | T cell | CD4-CD8+ | CD4+ CD8+ | CD4+ CD8- | CD4-CD8- |
| BC16208 | 89.0 | 0.22 | 0.38 | 99.5 | 13.2 | 3.11 | 83.6 | 0.13 |
| BC17058 | 94.5 | 0.12 | 0.52 | 99.4 | 4.71 | 1.97 | 93.2 | 0.09 |
| BC18050 | 94.2 | 0.23 | 0.71 | 99.1 | 14.0 | 7.03 | 78.4 | 0.55 |

In addition, the percentage of memory type CD4+ T cells in the CD4+ T cells confirmed above was confirmed through CD45RA and CCR7 expression. As a result, in each case of BC16208, BC17058 or BC18050, the percentage of effector memory T cells (Tem) was confirmed to be 81.35%, 83, 84%, or 73.11% (see FIGS. 13, 15, and 17). Tscm denotes stem cell memory T cells, Tcm denotes central memory T cells, Tem denotes effector memory T cells, and Teff denotes effector T cells. The analysis results are summarized in Table 8 below.

**Table 8. Confirmation of type of CD4+ T cells by case**

| Case | CD4+ T cell | | | |
|---|---|---|---|---|
| | Tscm | Tcm | Tem | Teff |
| BC16208 | 0.15 | 0.86 | 81.35 | 0.84 |
| BC17058 | 1.10 | 1.18 | 83.84 | 6.56 |
| BC18050 | 0.72 | 2.29 | 73.11 | 1.55 |

In addition, the percentage of memory type CD8+ T cells in CD8+ T cells confirmed above was confirmed through CD45RA and CCR7 expression. As a result, in each case of BC16208, BC17058 or BC18050, the percentage of effector memory T cells (Tem) was confirmed to be 11.40%, 2,20%, or 10.90%. The analysis results are summarized in Table 9 below.

**Table 9. Confirmation of type of CD8+ T cells by case**

| Case | CD8+ T cell | | | |
|---|---|---|---|---|
| | Tscm | Tcm | Tem | Teff |
| BC16208 | 0.04 | 0.05 | 11.4 | 1.63 |
| BC17058 | 0.02 | 0.02 | 2.2 | 2.44 |
| BC18050 | 0.18 | 0.21 | 10.9 | 2.58 |

### Confirmation of purity of final product

### Analysis method through FACS

Cells to be used in FACS analysis were suspended in FACS buffer (2% FBS in DPBS) and centrifuged (1,500 rpm, 4 °C, 5 min) (repeated twice). After centrifugation, the cells were suspended in FACS buffer. 1 to 5 x 10⁵ cells of the suspended cells were dispensed into 1.7 mL microtubes. For surface marker staining, each tube was treated with 2 µL of antibodies. Incubation was performed at 4 °C in a dark room for 30 minutes. After incubation, 800 µL of FACS buffer was added, and then centrifugation (1,500 rpm, 4 °C, 5 min) was performed. After discarding the supernatant, the cells were suspended by vortexing, 800 µL of FACS buffer was added thereto, and then centrifugation (1,500 rpm, 4 °C, 5 min) was performed (repeated three times). After suspending using 200 µL of FACS buffer, the cells were transferred to a 5 mL FACS tube, and analysis was performed using a BD FACS Canto^{™} II flow cytometer.

### Result

To confirm the purity of a final product, the expression of CD19, which is a B cell marker, and CD56, which as an NK cell marker in non-T cells, was confirmed through FACS analysis. To perform the analysis only on surviving cells, the cells were primarily gated with SSC-A and FSC-A (left graphs of FIGS. 18, 20, and 22). Here, the gated surviving cells were displayed as lymphocytes on the left graphs of FIGS. 18, 20, and 22. In the cases of BC16208, BC17058, and BC18050, CD19 expression rates were confirmed to be 0.03%, 0.01%, and 0.01%, respectively. In addition, in the cases of BC16208, BC17058, and BC18050, CD56 expression rates were confirmed to be 0.02%, 0.00%, and 0.03%, respectively (see FIGS. 18 to 23). The analysis results are summarized in Table 10 below.

**Table 10. Confirmation of purity of final product**

| Case | Live cells (lymphocytes) | Non-T cell (CD45+ CD3-) | Non-T cell (CD45+ CD3-) | | |
|---|---|---|---|---|---|
| | | | CD19+ CD56- | CD19- CD56+ | CD19- CD56- |
| BC16208 | 93 | 0.27 | 0.03 | 0.02 | 0.21 |
| BC17058 | 96 | 0.28 | 0.01 | 0.00 | 0.27 |
| BC18050 | 93.7 | 0.47 | 0.01 | 0.03 | 0.42 |

### Experimental Example 8. Confirmation of effect of immune cell composition including TILs 1 - Confirmation of tumor inhibition efficacy of breast cancer patient-derived TILs

The *in vivo* tumor inhibition efficacy of TILs cultured from the tumor tissue of a breast cancer patient was confirmed in patient-derived xenografts (PDX) derived from the same patient.

### Experimental Example 8-1. Confirmation of tumor inhibition efficacy of TILs, and confirmation of tumor inhibition efficacy of co-administration of TILs and IL-2

### Method

### 1. Preparation of patient-derived xenograft (PDX)

### PDX was prepared by the following method.

Five-week-old NOD-SCID mice (NOD.CB17-*Prkdc*^{scid}, Koatech), which had been acclimated, were restrained. 20 mg/mL of tribromoethanol (1x Avertin) was administered intraperitoneally into the mice at 250 mg/kg to induce anesthesia. A subject was identified by attaching an ear tag, using an ear punch, to the ear of an anesthetized mouse. Hair was removed from the surgical site using an epilator. An approximate 1.5-cm incision was made in the abdominal subcutaneous tissue. Blunt separation was performed between the subcutaneous tissue and the peritoneum using straight forceps (iris forceps). Patient-derived tumor tissue with a size of 1 to 2 mm² was transplanted into the fourth mammary fat pad on the right side of the animal. After transplantation was completed, the incised subcutaneous tissue was sutured using an auto clip and disinfected using povidone. The animals were placed in a cage and covered with litter to maintain body temperature. After recording the animal information on a cage name tag and keeping the animals in an individual ventilation cage (IVC) rack, it was confirmed that the animals woke up from anesthesia.

### 2. Preparation of TILs

Frozen TILs (i.e., a freeze-stored final product produced according to the method of Experimental Example 1 or Experimental Example 7, BC20082-TIL, sample) freeze-stored in liquid nitrogen after expansion were thawed in a 37 °C water bath. To remove DMSO present in the freeze-storage solution, the volumes of a warm CTS-AIM-V medium and the thawed final product were mixed at a ratio of 9:1. Centrifugation was performed at room temperature and 1,500 rpm for 5 minutes. After discarding the supernatant, the precipitated cells were suspended in a CTS-AIM-V medium and counted. The supernatant was removed after centrifugation at room temperature and 1,500 rpm for 5 minutes, and the cell pellet was suspended in DPBS, and the number of TILs suitable for administration to a mouse was titrated, as shown in the table below. The dose of TILs is listed in the table below. G1 is a control. G2 is a group to which 1x10⁷ TILs are administered, and is referred to as a low-dose TIL-administered group. G3 is a group to which 5x10⁷ TILs are administered, and is referred to as a high-dose TIL-administered group. G4 is an IL-2-administered group, and on day 0 to day 5, 1.5x10⁵ IU of IL-2 was administered daily. G4 is referred to as an IL-2 administered control. G5 is a group co-administered TILs and IL-2, which was prepared by administering 1x10⁷ TILs and administering 1.5x10⁵ IU of IL-2 daily on day 0 to day 5 after TIL administration. G5 is referred to as a low-dose TIL + IL-2 co-administered group. G6 is a TIL + IL-2 co-administered group, which was prepared by administering 5x10⁷ TILs and administering 1.5x10⁵ IU of IL-2 daily on day 0 to day 5 after TIL administration. G6 is referred to as a high-dose TIL + IL-2 co-administered group.

**Table 11. Information on dose(s) of TIL and/or IL-2**

| Group | Group | TIL administration | | IL-2 administration | |
|---|---|---|---|---|---|
| | | Dosage (cells/head) | Amount of administered solution (µL/head) | Dosage (IU/head) | Amount of administered solution (µL/head) |
| G1 | Control | 0 | 200 | - | - |
| G2 | Low-dose TIL administered group | 1 x 10⁷ | 200 | - | - |
| G3 | high-dose TIL administered group | 5 x 10⁷ | 200 | - | - |
| G4 | IL-2 administered control | 0 | 200 | 1.5 x 10⁵ | 150 |
| G5 | low-dose TIL + IL-2 co-administered group | 1 x 10⁷ | 200 | 1.5 x 10⁵ | 150 |
| G6 | high-dose TIL + IL-2 co-administered group | 5 x 10⁷ | 200 | 1.5 x 10⁵ | 150 |

### 3. TIL injection and mouse observation

Mice with a tumor volume of approximately 13 to 63 mm³ were grouped. A mouse was restrained using a restrainer, and then blood vessels were relaxed by putting the tail into warm water. TILs and IL-2 were administered intravenously using a 30G insulin syringe at 1 min/head. TILs were administered once on day 0. In the co-administered group, IL-2 was administered at 1.5x10⁵ IU/head on days 0, 1, 2, 3, 4, and 5 after TIL administration. That is, at each administration time, IL-2 were administered at 1.5x10⁵ IV/head. After administration, a tumor volume was measured twice a week and a body weight was measured once a week. The tumor volume was observed until it reached 2,000 mm³, and when it exceeded 2,000 mm³, the mice were euthanized using CO₂. After euthanasia of the mice, the tumor was removed and the size of the tumor was measured.

### Result

### 1. Change in body weight of mouse

### Confirmation of change in body weight of mouse by TIL administration

In G1, G2 (low-dose TIL administered group), and G3 (high-dose TIL administered group), the changes in body weight after TILs were administered to mice were observed. The results are shown in FIG. 24. As a result of the observation, it was confirmed that there were no changes in body weight by cell administration.

### Confirmation of change in body weight according to co-administration of TIL and IL-2

In addition, in G4 (IL-2 administered control), G5 (low-dose TIL + IL-2 co-administered group), and G6 (high-dose TIL + IL-2 co-administered group), the change in body weight after TILs were administered to mice was observed. The results are shown in FIG. 25. As a result of observation, it was confirmed that there were no changes in body weight by cell administration.

### 2. Measurement of change in tumor volume

### Confirmation of change in tumor volume according to TIL administration

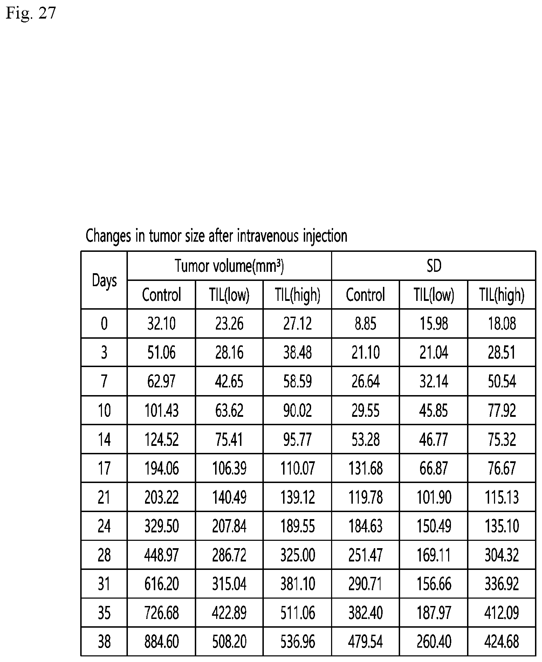

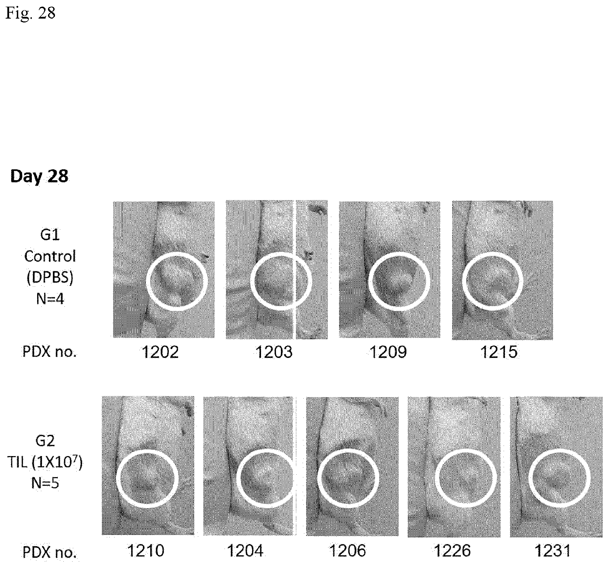

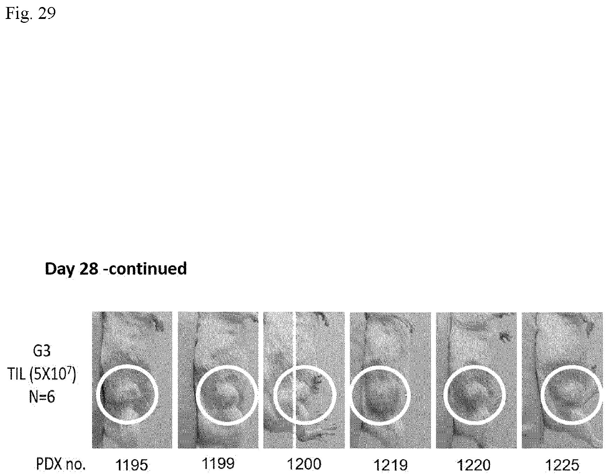

In G1, G2 (low-dose TIL administered group), and G3 (high-dose TIL administered group), the changes in tumor volume after TILs administration to mice were observed. The results are shown in FIGS. 26 and 27. FIGS. 28 and 29 are images of photographing the tumor-transplanted site of a mouse on day 28 after TIL administration. In addition, mice were euthanized on day 38 after TIL administration, tumors were extracted, and tumor volumes were measured. The results are shown in FIG. 30, and the images of the excised tumors are shown in FIG. 31. As a result of the measurement, it was confirmed that, in G2 (low-dose TIL administered group) and G3 (high-dose TIL administered group), tumor growth was inhibited and the tumor volumes are smaller than that of the control.

### Confirmation of change in tumor volume according to TIL + IL-2 co-administration

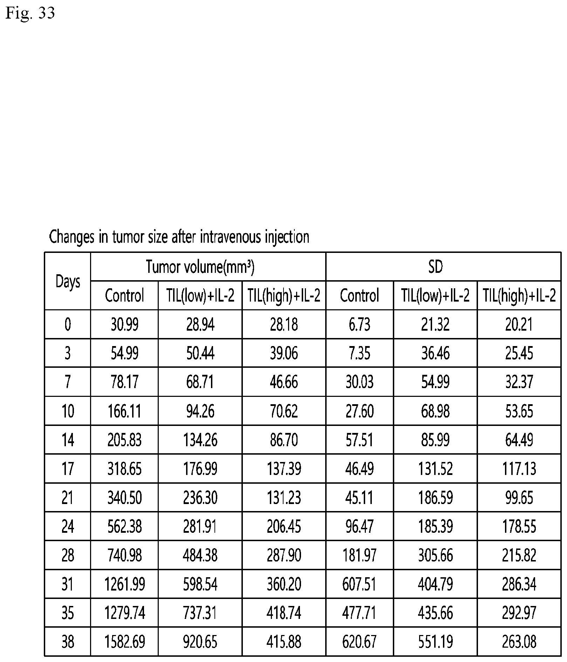

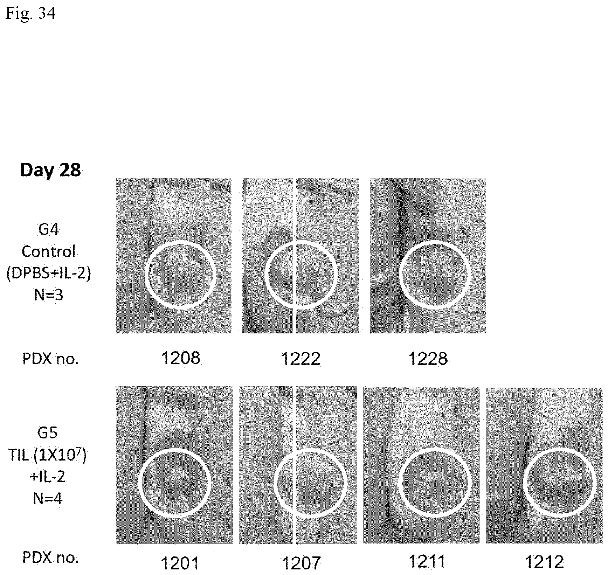

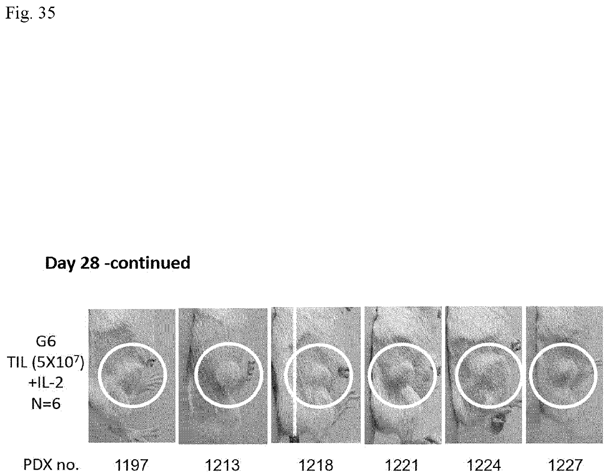

In G4 (IL-2 administered control), G5 (low-dose TIL + IL-2 co-administered group), and G6 (high-dose TIL + IL-2 co-administered group), after TILs were administered to mice, the changes in tumor volume were observed. The results are shown in FIGS. 32 and 33. FIGS. 34 and 35 are images of tumor-transplanted sites of mice on day 28 after TIL administration. In addition, on day 38 after TIL administration, the mice were euthanized, tumors were extracted, and tumor volumes were measured. The results are shown in FIG. 36, and the images of the excised tumors are shown in FIG. 37. As a result of measurement, it was confirmed that, in G5 (low-dose TIL + IL-2 co-administered group) and G6 (high-dose TIL + IL-2 co-administered group), tumor growth is inhibited and the tumor volumes are smaller than that of the control.

### Experimental Example 9. Confirmation of efficacy of immune cell composition including TILs - confirmation of tumor inhibition efficacy of lung cancer patient-derived TILs

The *in vivo* tumor inhibition efficacy of TILs cultured from tumor tissue of a lung cancer patient was confirmed in a patient-derived xenograft (PDX) derived from the same patient.

### Method

### 1. PDX preparation

### PDX was prepared by the following method.

Five-week-old NOD-SCID mice (NOD.CB17-*Prkdc*^{scid}, Koatech) which had been acclimated, were restrained. 20 mg/mL of tribromoethanol (1x Avertin) was administered intraperitoneally into the mouse at 250 mg/kg to induce anesthesia. A subject was identified by attaching an ear tag, using an ear punch, to the ear of an anesthetized mouse. Hair was removed from the surgical site using an epilator. An approximate 1-cm incision was made in the subcutaneous tissue above a thigh muscle. Blunt separation was performed between the subcutaneous tissue and the fascia using straight forceps (iris forceps). Patient-derived tumor tissue with a size of 1 to 2 mm² was transplanted under the skin of the flank at the same position on the right side of the animal. After transplantation was completed, the incised subcutaneous tissue was sutured using an auto clip and disinfected with povidone. The animals were placed in a cage and covered with litter to maintain the temperature. After recording the animal information on a cage name tag and keeping the animals in an individual ventilation cage (IVC) rack, it was confirmed that the animals woke up from anesthesia.

### 2. Preparation of TILs

Frozen TILs freeze-stored in liquid nitrogen after expansion (i.e., a freeze-stored final product produced according to the method of Experimental Example 1 or Experimental Example 7, LC20001-TIL sample) were thawed in a 37 °C water bath. To remove DMSO present in the freeze-storage solution, the volumes of a warm CTS-AIM-V medium and a final product were mixed at a ratio of 9:1. Centrifugation was performed at room temperature and 1,500 rpm for 5 minutes, and after removing the supernatant, the precipitated cells were suspended in a CTS-AIM-V medium and counted. The supernatant was removed after centrifugation at room temperature and 1,500 rpm for 5 minutes, and the cell pellet was suspended in DPBS, and the number of TILs was adjusted to 5 x 10⁷ cells per 200 µL. The dose of TILs is shown in the table below. A control is the group administered only IL-2, in which 1.5x10⁵ IU of IL-2 was administered daily on day 0 to day 5. The control is referred to as IL-2. The cell-administered group is a group co-administered TILs and IL-2, in which 5x10⁷ TILs were administered and 1.5x10⁵ IU of IL-2 was administered daily on day 0 to day 5 after TIL administration. The cell-administered group is referred to as TIL+IL-2.

**Table 12. Information on dose(s) of TIL and/or IL-2**

| Group | TIL administration | | IL-2 administration | |
|---|---|---|---|---|
| | Dosage (cells/head) | Amount of administered solution (µL/head) | Dosage (IU/head) | Amount of administered solution (µL/head) |
| Control (IL-2) | 0 | 200 | 1.5 x 10⁵ | 150 |
| Cell-administered group (TIL + IL-2) | 5 x 10⁷ | 200 | 1.5 x 10⁵ | 150 |

### 3. Co-administration of TIL and IL-2

Mice with a tumor volume of approximately 13 to 63 mm³ were grouped. A mouse was restrained using a restrainer, and then blood vessels were relaxed by putting the tail into warm water. TILs and IL-2 were administered intravenously using a 30G insulin syringe at 1 min/head. TILs were administered once on day 0. In the co-administered group, IL-2 was administered at 1.5x10⁵ IU/head on days 0, 1, 2, 3, 4, and 5 after TIL administration. That is, at each administration time, IL-2 were administered at 1.5x10⁵ IU/head. After administration, a tumor volume was measured twice a week and a body weight was measured once a week. The tumor volumes were observed until it reached 2,000 mm³, and when it exceeded 2,000 mm³, the mice were euthanized using CO₂. After euthanasia of the mice, the tumors were removed and the size of the tumors were measured.

### Result

### 1. Change in body weight of mouse

Changes in body weight were observed after intravenous administration of the control (IL-2) and the cell-administered group (TIL+IL-2). The results are shown in FIG. 38. As a result of the observation, it was confirmed that there were no changes in body weight by cell administration.

### 2. Measurement of change in tumor volume

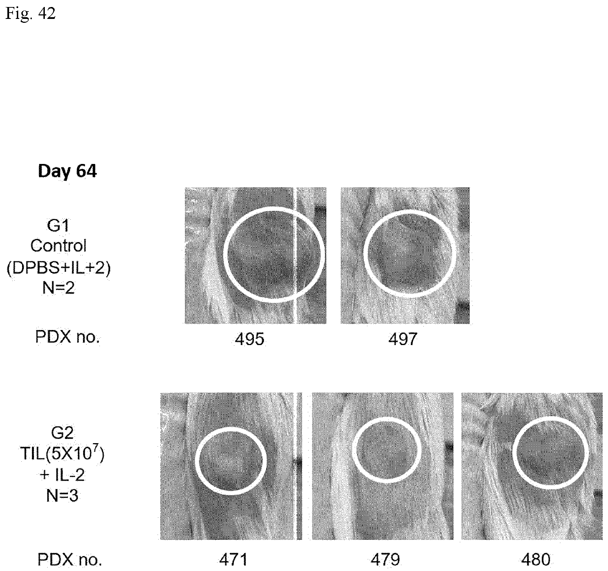

Tumor volumes were measured twice a week after intravenous administration of the control administered only IL-2 and the cell-administered group co-administered TILs and IL-2. The results are shown in FIGS. 39 to 41. FIG. 42 is an image of the tumor-transplanted site of a mouse on day 64 after TIL administration. In addition, mice were euthanized on day 70 after TIL administration, tumors were extracted, and tumor volumes were measured. The results are shown in FIG. 43. As a result of the measurement, it can be confirmed that, in the cell-administered group, tumor growth was inhibited and the tumor volume is smaller than that of the control.

## Claims

1. A method for producing an immune cell composition, comprising:
(a) culturing a tumor sample in a first container,
wherein the tumor sample is cultured in a first composition in the first container, wherein the first composition comprises medium, serum, and 500 to 1500 IU/ml of IL-2,
wherein the first container is a gas-permeable container or a container comprising gas supply system,
wherein the culturing is performed for 13 to 15 days,
wherein, during the culturing, a first additive composition is added to the first container once or twice or more, and the first additive composition comprises medium, serum, and IL-2;
(b) obtaining a first immune cell population from the cultured product obtained from (a);
(c) seeding a second composition and a second immune cell population in a second container,
wherein the second immune cell population is derived from the first immune cell population,
wherein the second composition comprises medium, serum, 1000 to 3000 IU/ml of IL-2, anti-CD3 antibody, and a feeder cell,
wherein the second container is a gas-permeable container or a container comprising gas supply system;
(d) expanding the second immune cell population to produce a third immune cell population,
wherein during the expansion, a second additive composition is added to the second container once or twice or more, and the second additive composition comprises medium, serum, and IL-2,
wherein the expansion is performed for 13 to 15 days; and
(e) obtaining the third immune cell population from the expanded product obtained from (d) and producing the immune cell composition,

2. The method of claim 1,
in (a), 12 to 24 of the tumor samples are cultured,
wherein each of the tumor sample has cross-sectional area of 1mm² to 4mm², and
wherein the tumor samples are obtained from a tumor fragment obtained from a subject.

3. The method of claim 1,
wherein a concentration of serum of the first composition is 5 vol% or less, and
wherein a concentration of serum of the second composition is 5 vol% or less.

4. The method of claim 1,
wherein a volume of the first composition is 30% or less of a maximum allowable capacity of the first container, and
wherein a volume of the second composition is 30% or less of a maximum allowable capacity of the second container.

5. The method of claim 1,
wherein the first additive composition is a composition having the same compositional substances and ratio as the first composition, and
wherein during the culturing, the first additive composition is added to the first container two or more times.

6. The method of claim 5,
during the culturing, the first additive composition is added to the first container on the 7th, 10th, and 12th day from the start date of the culturing,
wherein amount of the first additive composition added at each addition point is the same.

7. The method of claim 1,
wherein the anti-CD3 antibody is at least one selected from OKT3, Otelixizumab, teplizumab, and visilizumab, and
wherein a concentration of the anti-CD3 antibody of the second composition is 10ng/ml to 100ng/ml.

8. The method of claim 1,
wherein the feeder cell is an inactivated allogeneic peripheral blood mononuclear cell.

9. The method of claim 1,
wherein the feeder cell is included in the second composition in a ratio of approximately 1:200 (number of cells of the second immune cell population:number of feeder cells) based on the number of cells in the second immune cell population.

10. The method of claim 1,
in the step of seeding, the number of cells of the second immune cell population seeded in the second container is 1E5 to 10E5.

11. The method of claim 1,
wherein the second additive composition has the same compositional substances and ratio as the second composition excluding the anti-CD3 antibody and the feeder cell, and
wherein during the expansion, the second additive composition is added to the second container two or more times.

12. The method of claim 11,
during the expansion, the second additive composition is added to the second container on the 4th, 6th, 8th, and 10th days from the start date of expansion, and amount of the second additive composition added is gradually increased at each addition point.

13. The method of claim 1,
wherein the immune cell composition comprises the third immune cell population or a fourth immune cell population obtained from the third immune cell population.

14. The method of claim 1,
wherein the tumor sample is derived from a solid tumor of a subject having breast cancer, colon cancer, lung cancer, stomach cancer, kidney cancer, ovarian cancer, or skin cancer including melanoma, or is derived from a malignant pleural effusion of a subject having lung cancer.

15. The method of claim 1,
wherein the method further comprises:
cryopreserving the first immune cell population; and
thawing the cryopreserved first immune cell population,
wherein cryopreserving the first immune cell population and the thawing the cryopreserved first immune cell population are performed after (b) obtaining a first immune cell population from the cultured product obtained from (a).

## Patentansprüche

1. Verfahren zur Herstellung einer Immunzellzusammensetzung, umfassend:
(a) das Kultivieren einer Tumorprobe in einem ersten Behälter,
wobei die Tumorprobe in einer ersten Zusammensetzung in dem ersten Behälter kultiviert wird, wobei die erste Zusammensetzung Medium, Serum und 500 bis 1500 IE/ml IL-2 umfasst,
wobei der erste Behälter ein gasdurchlässiger Behälter oder ein Behälter mit einem Gaszufuhrsystem ist,
wobei das Kultivieren über 13 bis 15 Tage erfolgt,
wobei während des Kultivierens eine erste Additivzusammensetzung einmal oder zweimal oder mehrmals in den ersten Behälter gegeben wird und die erste Additivzusammensetzung Medium, Serum und IL-2 umfasst;
(b) das Gewinnen einer ersten Immunzellpopulation aus dem aus (a) erhaltenen kultivierten Produkt;
(c) das Aussäen einer zweiten Zusammensetzung und einer zweiten Immunzellpopulation in einem zweiten Behälter,
wobei die zweite Immunzellpopulation von der ersten Immunzellpopulation abgeleitet ist,
wobei die zweite Zusammensetzung Medium, Serum, 1000 bis 3000 IE/ml IL-2, Anti-CD3-Antikörper und eine Feeder-Zelle umfasst,
wobei der zweite Behälter ein gasdurchlässiger Behälter oder ein Behälter mit einem Gaszufuhrsystem ist;
(d) das Expandieren der zweiten Immunzellpopulation unter Bildung einer dritten Immunzellpopulation,
wobei während der Expansion eine zweite Additivzusammensetzung einmal oder zweimal oder öfter in den zweiten Behälter gegeben wird und die zweite Additivzusammensetzung Medium, Serum und IL-2 umfasst;
wobei die Expansion über 13 bis 15 Tage erfolgt; und
(e) Gewinnen der dritten Immunzellpopulation aus dem aus (d) erhaltenen expandierten Produkt und Erzeugen der Immunzellzusammensetzung.

2. Verfahren gemäß Anspruch 1,
wobei in (a) 12 bis 24 der Tumorproben kultiviert werden,
wobei jede der Tumorproben eine Querschnittsfläche von 1 mm² bis 4 mm² aufweist und
wobei die Tumorproben von einem Tumorfragment erhalten werden, das von einem Patienten erhalten wurde.

3. Verfahren gemäß Anspruch 1,
wobei die Konzentration des Serums in der ersten Zusammensetzung 5 Vol.- % oder weniger beträgt und
wobei die Konzentration des Serums in der zweiten Zusammensetzung 5 Vol.-% oder weniger beträgt.

4. Verfahren gemäß Anspruch 1,
wobei das Volumen der ersten Zusammensetzung 30% oder weniger der maximal erlaubten Kapazität des ersten Behälters beträgt und
wobei das Volumen der zweiten Zusammensetzung 30% oder weniger der maximal erlaubten Kapazität des zweiten Behälters beträgt.

5. Verfahren gemäß Anspruch 1,
wobei die erste Additivzusammensetzung eine Zusammensetzung ist, die dieselben Bestandteile in demselben Verhältnis wie die erste Zusammensetzung aufweist, und
wobei während des Kultivierens die erste Additivzusammensetzung zweimal oder öfter in den ersten Behälter gegeben wird.

6. Verfahren gemäß Anspruch 5,
wobei während des Kultivierens die erste Additivzusammensetzung am 7., 10. und 12. Tag seit dem Startdatum der Kultur in den ersten Behälter gegeben wird,
wobei die an jedem Zugabepunkt zugegebene Menge der ersten Additivzusammensetzung jeweils dieselbe ist.

7. Verfahren gemäß Anspruch 1,
wobei der Anti-CD3-Antikörper wenigstens einer ist, der aus OKT3, Otelixizumab, Teplizumab und Visilizumab ausgewählt ist, und
wobei die Konzentration des Anti-CD3-Antikörpers in der zweiten Zusammensetzung 10 ng/ml bis 100 ng/ml beträgt.

8. Verfahren gemäß Anspruch 1,
wobei die Feeder-Zelle eine inaktivierte allogene mononukleäre Zelle des peripheren Bluts ist.

9. Verfahren gemäß Anspruch 1,
wobei die Feeder-Zelle in der zweiten Zusammensetzung in einem Verhältnis von 1:200 vorhanden ist (Anzahl der Zellen der zweiten Immunzellpopulation : Anzahl der Feeder-Zellen), bezogen auf die Anzahl der Zellen in der zweiten Immunzellpopulation.

10. Verfahren gemäß Anspruch 1,
wobei in dem Schritt des Aussäens die Anzahl der Zellen der zweiten Immunzellpopulation, die in dem zweiten Behälter ausgesät werden, 1x10⁵ bis 10x10⁵ beträgt.

11. Verfahren gemäß Anspruch 1,
wobei die zweite Additivzusammensetzung dieselben Bestandteile in demselben Verhältnis wie die zweite Zusammensetzung außer dem Anti-CD3-Antikörper und der Feeder-Zelle aufweist und
wobei während der Expansion die zweite Additivzusammensetzung zweimal oder öfter in den zweiten Behälter gegeben wird.

12. Verfahren gemäß Anspruch 11,
wobei während der Expansion die zweite Additivzusammensetzung am 4., 6., 8. und 10. Tag seit dem Startdatum der Expansion in den zweiten Behälter gegeben wird und die zugesetzte Menge der zweiten Additivzusammensetzung an jedem Zugabepunkt allmählich erhöht wird.

13. Verfahren gemäß Anspruch 1,
wobei die Immunzellzusammensetzung die dritte Immunzellpopulation oder eine aus der dritten Immunzellpopulation erhaltene vierte Immunzellpopulation umfasst.

14. Verfahren gemäß Anspruch 1,
wobei die Tumorprobe aus einem festen Tumor von einem Patienten, der an Brustkrebs, Darmkrebs, Lungenkrebs, Magenkrebs, Nierenkrebs, Eierstocckrebs oder Hautkrebs einschließlich Melanom leidet, stammt oder aus einem bösartigen Pleuraerguss von einem Patienten, der an Lungenkrebs leidet, stammt.

15. Verfahren gemäß Anspruch 1,
wobei das Verfahren weiterhin umfasst:
Kryokonservieren der ersten Immunzellpopulation; und
Auftauen der kryokonservierten ersten Immunzellpopulation,
wobei das Kryokonservieren der ersten Immunzellpopulation und das Auftauen der kryokonservierten ersten Immunzellpopulation durchgeführt werden, nachdem man (b) eine erste Immunzellpopulation aus dem aus (a) erhaltenen Kulturprodukt erhalten hatte.

## Revendications

1. Procédé pour produire une composition de cellules immunitaires, comprenant les étapes consistant à :
(a) cultiver un échantillon tumoral dans un premier récipient,
dans lequel l'échantillon tumoral est cultivé dans une première composition dans le premier récipient, la première composition comprenant du milieu, du sérum et 500 à 1500 UI/ml d'IL-2,
dans lequel le premier récipient est un récipient perméable aux gaz ou un récipient comprenant un système d'alimentation en gaz,
dans lequel la culture est réalisée pendant 13 à 15 jours,
dans lequel, au cours de la culture, une première composition additive est ajoutée au premier récipient une ou deux fois ou plusieurs fois, et la première composition additive comprend du milieu, du sérum et de l'IL-2 ;
(b) obtenir une première population de cellules immunitaires à partir du produit cultivé obtenu à partir de (a) ;
(c) ensemencer une deuxième composition et une deuxième population de cellules immunitaires dans un deuxième récipient,
dans lequel la deuxième population de cellules immunitaires est dérivée de la première population de cellules immunitaires,
dans lequel la deuxième composition comprend un milieu, du sérum, 1000 à 3000 UI/ml d'IL-2, un anticorps anti-CD3 et une cellule nourricière,
dans lequel le deuxième récipient est un récipient perméable aux gaz ou un récipient comprenant un système d'alimentation en gaz ;
d) faire expanser la deuxième population de cellules immunitaires pour produire une troisième population de cellules immunitaires,
dans lequel, au cours de l'expansion, une deuxième composition additive est ajoutée au deuxième récipient une ou deux fois ou plus, et la deuxième composition additive comprend du milieu, du sérum et de l'IL-2,
dans lequel l'expansion est réalisée pendant 13 à 15 jours ; et
(e) obtenir la troisième population de cellules immunitaires à partir du produit expansé obtenu à partir de (d) et obtenir la composition de cellules immunitaires.

2. Procédé selon la revendication 1,
dans lequel dans (a) 12 à 24 échantillons tumoraux sont cultivés,
dans lequel chaque échantillon tumoral présente une surface de section transversale de 1 mm² à 4 mm², et
dans lequel les échantillons tumoraux sont obtenus à partir d'un fragment de tumeur provenant d'un sujet.

3. Procédé selon la revendication 1,
dans lequel la concentration en sérum dans la première composition est de 5 % en volume ou moins, et
dans lequel la concentration en sérum dans la deuxième composition est de 5 % en volume ou moins.

4. Procédé selon la revendication 1,
dans lequel le volume de la première composition représente 30 % ou moins de la capacité maximale admissible du premier récipient, et
dans lequel le volume de la deuxième composition représente 30 % ou moins de la capacité maximale admissible du deuxième récipient.

5. Procédé selon la revendication 1,
dans lequel la première composition additive est une composition ayant les mêmes composants et dans les mêmes proportions que la première composition, et
dans lequel, au cours de la culture, la première composition additive est ajoutée au premier récipient deux fois ou plus.

6. Procédé selon la revendication 5,
dans lequel, au cours de la culture, la première composition additive est ajoutée au premier récipient les 7e, 10e et 12e jours à compter de la date de début de la culture,
dans lequel la quantité de la première composition additive ajoutée à chaque point d'ajout est la même.

7. Procédé selon la revendication 1,
dans lequel l'anticorps anti-CD3 est au moins l'un choisi parmi OKT3, otélixizumab, téplizumab et visilizumab, et
dans lequel la concentration de l'anticorps anti-CD3 de la deuxième composition est de 10 ng/ml à 100 ng/ml.

8. Procédé selon la revendication 1,
dans lequel la cellule nourricière est une cellule mononucléaire du sang périphérique allogénique inactivée.

9. Procédé selon la revendication 1,
dans lequel la cellule nourricière est incluse dans la deuxième composition dans un rapport d'environ 1 : 200 (nombre de cellules de la deuxième population de cellules immunitaires : nombre de cellules nourricières) sur la base du nombre de cellules dans la deuxième population de cellules immunitaires.

10. Procédé selon la revendication 1,
dans lequel, lors de l'étape d'ensemencement, le nombre de cellules de la deuxième population de cellules immunitaires ensemencées dans le deuxième récipient est de 1 x 10⁵ à 10 x 10⁵.

11. Procédé selon la revendication 1,
dans lequel la deuxième composition additive présente les mêmes substances de composition et les mêmes proportions que la deuxième composition, à l'exception de l'anticorps anti-CD3 et des cellules nourricières, et
dans lequel, au cours de l'expansion, la deuxième composition additive est ajoutée au deuxième récipient deux fois ou plus.

12. Procédé selon la revendication 11,
dans lequel, au cours de l'expansion, la deuxième composition additive est ajoutée au deuxième récipient les 4e, 6e, 8e et 10e jours à compter de la date de début de l'expansion, et la quantité de la deuxième composition additive ajoutée est progressivement augmentée à chaque point d'ajout.

13. Procédé selon la revendication 1,
dans lequel la composition de cellules immunitaires comprend la troisième population de cellules immunitaires ou une quatrième population de cellules immunitaires obtenue à partir de la troisième population de cellules immunitaires.

14. Procédé selon la revendication 1,
dans lequel l'échantillon tumoral provient d'une tumeur solide d'un sujet atteint d'un cancer du sein, d'un cancer du côlon, d'un cancer du poumon, d'un cancer de l'estomac, d'un cancer du rein, d'un cancer de l'ovaire ou d'un cancer de la peau, y compris un mélanome, ou provient d'un épanchement pleural malin d'un sujet atteint d'un cancer du poumon.

15. Procédé selon la revendication 1,
dans lequel le procédé comprend en outre les étapes consistant à :
cryoconserver la première population de cellules immunitaires ; et
décongeler la première population de cellules immunitaires cryoconservées,
dans lequel la cryoconservation de la première population de cellules immunitaires et la décongélation de la première population de cellules immunitaires cryoconservées sont effectuées après l'étape (b) l'obtention d'une première population de cellules immunitaires à partir du produit cultivé obtenu à partir de (a).
